# EUROPEAN PATENT SPECIFICATION

(11) **EP 2 303 407 B1**
(45) Date of publication and mention of the grant of the patent: **21.06.2017**
(21) Application number: 09745293.2
(22) Date of filing: 12.05.2009
(51) Int. Cl.: C07K 14/47, A61P 37/02, A61K 31/711, A61P 25/28, A61K 31/713, A61K 38/00, A61P 35/00, A61K 39/395, C07K 16/28, G01N 33/53

(54) **METHODS OF DETECTING CELLS WITH A DISRUPTED CELL MEMBRANE, CELLS INFECTED WITH A PATHOGEN, DYING CELLS OR DEAD CELLS**
VERFAHREN ZUM NACHWEIS VON ZELLEN MIT EINER GESTÖRTEN ZELLMEMBRAN, MIT EINEM ERREGER INFIZIERTER ZELLEN, ABSTERBENDER ZELLEN ODER TOTER ZELLEN
PROCÉDÉ PERMETTANT DE DÉTECTER LES CELLULES DONT LA MEMBRANE CELLULAIRE EST DÉTÉRIORÉE, LES CELLULES INFECTÉES PAR UN PATHOGÈNE, LES CELLULES MOURANTES ET LES CELLULES MORTES

(30) Priority: 13.05.2008 US 52983 P; 08.12.2008 US 120801 P
(43) Date of publication of application: 06.04.2011
(73) Proprietor: The Walter and Eliza Hall Institute of Medical Research, Parkville, VIC 3050 (AU)
(72) Inventor: LAHOUD, Mireille Hanna, East Melbourne, Victoria 3002 (AU); CAMINSCHI, Irina, Ascot Vale, Victoria 3032 (AU); SHORTMAN, Ken, Princess Hill, Victoria 3054 (AU); ZHANG, Jian-Guo, Parkville, Victoria 3052 (AU); VAN DELFT, Mark Francis, Camlachie, Ontario N0N 1E0 (CA); HUANG, David Ching Siang, Fitzroy North, Victoria 3068 (AU); WRIGHT, Mark Dexter, Northcote, Victoria 3070 (AU)
(74) Representative: Sweetinburgh, Mark Roger
(86) International application number: PCT/AU2009/000591
(87) International publication number: WO 2009/137871

(56) References cited:
- WO-A1-2008/043153
- WO-A1-2009/013484
- WO-A1-2009/013484
- WO-A1-2009/026660
- HUYSAMEN, C. ET AL.: 'The fungal pattern recognition receptor, Dectin-1, and the associated cluster of C-type lectin-like receptors.' FEMS MICROBIOL LETT. vol. 290, no. 2, January 2009, pages 121 - 8, XP008140851
- HUYSAMEN, C. ET AL.: 'CLEC9A is a novel activation C-type lectin-like receptor expressed on BDCA3+ dendritic cells and a subset of monocytes.' J BIOL CHEM. vol. 283, no. 24, 13 June 2008, pages 16693 - 701, XP002503157
- MEZGER, M. ET AL.: 'Proinflammatory response of immature human dendritic cells is mediated by dectin-1 after exposure to Aspergillus fumigatus germ tubes.' J INFECT DIS. vol. 197, no. 6, 15 March 2008, pages 924 - 31, XP008140859
- DATABASE MEDLINE [Online] US NATIONAL LIBRARY OF MEDICINE (NLM), BETHESDA, MD, US February 2012 CAMINSCHI IRINA ET AL: 'Boosting antibody responses by targeting antigens to dendritic cells.' Database accession no. NLM22153931 & TRENDS IN IMMUNOLOGY FEB 2012 LNKD- PUBMED:22153931, vol. 33, no. 2, February 2012 (2012-02), pages 71-77, ISSN: 1471-4981
- DATABASE BIOSIS [Online] BIOSCIENCES INFORMATION SERVICE, PHILADELPHIA, PA, US 20 May 2002 IYODA TOMONORI ET AL: 'The CD8+ dendritic cell subset selectively endocytoses dying cells in culture and in vivo' Database accession no. PREV200200364170 & JOURNAL OF EXPERIMENTAL MEDICINE, vol. 195, no. 10, 20 May 2002 (2002-05-20) , pages 1289-1302, ISSN: 0022-1007
- SAUTER B ET AL: "Consequences of cell death: Exposure to necrotic tumor cells, but not primary tissue cells or apoptotic cells, induces the maturation of immunostimulatory dendritic cells", THE JOURNAL OF EXPERIMENTAL MEDICINE, ROCKEFELLER UNIVERSITY PRESS, US, vol. 191, no. 3, 7 February 2000 (2000-02-07), pages 423-433, XP002267741, ISSN: 0022-1007, DOI: 10.1084/JEM.191.3.423

## Description

### FIELD OF THE INVENTION

The present invention relates to the use of a compound for detecting cells with a disrupted cell membrane, cells infected with a pathogen, dying cells or dead cells, or distinguishing between an early stage apoptotic cell and a late stage apoptotic cell, necrotic cell or dead cell.

### BACKGROUND OF THE INVENTION

### Dendritic cell biology

Dendritic cells (DC) are found in most tissues, where they continuously sample the antigenic environment and use several types of receptors to monitor for invading pathogens. In steady state, and at an increased rate upon detection of pathogens, sentinel DC in non-lymphoid tissues migrate to the lymphoid organs where they present to T cells the Ag they have collected and processed. The phenotype acquired by the T cell depends on the context in which the DC presents its Ag. If the Ag is an innocuous self-component, DC may induce various forms of T cell unresponsiveness (tolerance) (Kenna et al., 2008). However, if the Ag is derived from a pathogen, or damaged self, DC receive danger signals, become activated and the T cells are then stimulated to become effectors, necessary to provide protective immunity (Heath and Villadangos, 2005). Moreover, DC can instruct effector T cells to acquire distinct abilities (migratory properties, cytokine secretion) tailored to fighting particular pathogens. The information required to tailor immunity is determined by the DC-pathogen interaction and is communicated by the DC to T cells via secreted cytokines or membrane proteins.

### D C heterogeneity

The DC network is composed of distinct DC subtypes (Shortman and Naik, 2007). DC can be broadly classified into plasmacytoid pre-DC (pDC) and conventional DC (cDC). pDC secrete high levels of IFNα upon stimulation, and only develop DC form after activation (Hochrein et al., 2001; O'Keeffe et al., 2002). cDC have immediate DC form and Ag presentation function, and may be divided into "lymphoid tissue resident" DC and classical "migratory" DC which arrive in lymph nodes (LN) via the lymph. Lymphoid tissue resident DC in mice may in turn be divided into the CD8⁺ and CD8⁻ subsets (Belz et al., 2004; Lahoud et al., 2006; Henri et al., 2001). In addition, inflammatory DC develop from monocytes as a consequence of infection or inflammation (Shortman and Naik, 2007). Importantly, DC subtypes share many functions (uptake, processing and presentation of Ag to activate naive T cells), but also exhibit subset-specific roles. These include differential expression of Toll-like receptors, production of particular chemokines, IL-12 secretion, restricted primarily to CD8⁺DC which directs T cells to a Th1 cytokine profile and cross-presentation of exogenous Ag via MHC I, mainly restricted to CD8⁺DC, which allows these DC to be major presenters of viral Ag to the CD8⁺ T cells, allowing them to develop cytotoxic function.

Humans most likely contain equivalents of these DC subsets, as mouse and human DC extracted from the same tissue (blood or thymus) are similar (Vandenabeele et al., 2001; O'Keeffe et al., 2003). However, human equivalents of most murine lymphoid organ resident DC subsets remain unknown, due to differences in markers between species (CD8α is not on human DC) and the difficulty of obtaining human lymphoid organs for detailed analyses. Definition of DC subset-specific markers conserved between mice and humans is therefore a major challenge.

### Cell death and the immune response

Apoptosis occurs continuously during development and within the immune system. It is characterised by convoluting of cell membranes, condensing of nuclei and fragmenting of cells into apoptotic bodies that still retain their cellular contents; these are usually engulfed by "phagocytes" without release of cellular contents. In contrast, necrosis, which may occur during injury or infection, is characterised by cell membrane rupturing and cellular content release. However, cellular contents may also be released from apoptotic cells if they are not rapidly engulfed (secondary necrosis). Rapid clearance of apoptotic cells generally promotes an immunosuppressive environment that avoids inflammatory responses to self-Ag, whereas necrosis or failure of apoptotic cell clearance promotes immune responses to self-Ag (Hume, 2008; Nagata, 2007; Peng et al., 2007). Thus, early recognition of apoptotic cells is essential for homeostatic maintenance and prevention of autoimmunity.

### Recognition of dead/ dying cells

Phagocytes, including DC, sense many molecular changes in dying cells. Molecules normally within the cell may be secreted or presented on the cell surface of apoptotic cells, or finally exposed when the cell membrane is disrupted. Existing molecules may be modified or new molecules produced in response to stress. An example of such a molecule is phosphotidylserine (PS) which is a lipid that is translocated from the inside to the outside of the cell membrane early in the apoptotic process, and serves as an "eat me" signal. Many molecules on phagocytes mediate the recognition of PS including CD36, MFG-E8 and Tim4.

Another example of molecules which are produced in response to stress are heat shock or stress proteins (Hsp), which serve as molecular chaperones in intact cells (Binder et al., 2004), and may provide signals to DC from stressed/ dying cells (Delneste, 2004). Proposed Hsp receptors on phagocytes and DC include CD91, Lox1/Clec8, CD40, TLR2, TLR4, CD36. Furthermore, immunisation with Hsp-chaperoned peptides is extremely effective, requiring only pg amounts of protein.

### Recognition and uptake of dead cells by DC

Both macrophages and DC can take up dead or dying cells. Many of the scavenger receptors on macrophages are also found on DC. However, only DC have the capacity to process cell components and then effectively present them to, and activate, naive T cells. While the uptake of apoptotic cells by DC in the absence of additional pathogen signals generally induces tolerance (Steinman et al., 2000), uptake of necrotic cells induces DC maturation and stimulation of immune response (Sauter et al., 2000). Thus differential recognition of these states by receptors on DC is crucial to the immune system. Murine CD8⁺ cDC are more efficient than CD8⁻ DC at both uptake of apoptotic/ dead cells and subsequent presentation of cell bound and viral Ag to T cells, particularly the "cross-presentation" on MHC I to CD8 T cells (Belz et al., 2004; Schnorrer et al., 2006; Belz et al., 2005; Iyoda et al., 2002). Human pDC have been claimed to be effective at uptake of dying cells (Hoeffel et al., 2007). Thus, the selective expression of dead cell uptake receptors by DC subtypes is an important issue. Iyoda et al (2002) J. Exp. Med 195(10)1289-1302 describes that the CD8⁺ DC subset selectively endocytoses dying cells in culture and *in vivo.*

WO2008/043153 describes a method of screening for a neoplastic cell in a subject.

### SUMMARY OF THE INVENTION

The present inventors have identified molecules that bind to a ligand(s) on cells with a disrupted cell membrane, cells infected with a pathogen, dying cells and dead cells.

In one aspect of the present invention, there is provided in vitro use of a compound for detecting a cell with a disrupted cell membrane, a cell infected with a pathogen, a dying cell or a dead cell or distinguishing between an early stage apoptotic cell and a late stage apoptotic cell, necrotic cell or dead cell,
wherein
(1) the compound is a polypeptide which comprises:
   i) an amino acid sequence as provided in any one of SEQ ID NO's 1 to 8;
   ii) an amino acid sequence which is at least 50% identical to any one or more of SEQ ID NO's 1 to 8 and which is able to detect a cell with a disrupted cell membrane, a cell infected with a pathogen, a dying cell or a dead cell or distinguish between an early stage apoptotic cell and a late stage apoptotic cell, necrotic cell or dead cell; and/or
   iii) a biologically active, soluble and/or antigenic fragment of i) or ii) and which is able to detect a cell with a disrupted cell membrane, a cell infected with a pathogen, a dying cell or a dead cell or distinguish between an early stage apoptotic cell and a late stage apoptotic cell, necrotic cell or dead cell, or
(2) the compound is an antibody or antigenic binding fragment thereof,
   (A) wherein the antibody is a monoclonal antibody, humanized antibody, single chain antibody, diabody, triabody, or tetrabody, and/or
   (B) wherein the antibody is 24/04-10B4, 42/04-42D2, 20/05-3A4 or 23/05-4C6, wherein antibodies 24/04-10B4, 42/04-42D2, 20/05-3A4 and 23/05-4C6 are produced by hydridoma cell lines deposited with the European Collection of Cell Cultures (ECACC) 24/04-10B4-24-8, 42/04-42D2-66-4-1, 20/05/3A4-26-16, 23/05-4C6-29-3 on 11 December 2007 under Deposit Reference Numbers 07121101, 07121102, 07121103, and 07121104 respectively, or by higher antibody secreting subclones of the hybridomas (24/04-10B4-24-8-FACS 9-5, 42/04-42D2-66-4-1-Clone 4, 20/05-3A4-26-16-Clone 5, 23/05-4C6-29-3-Clone 5) deposited with the ECACC on 29 April 2008, and designated Accession numbers 08042901, 08042902, 08042903, and 08042904, respectively.

In another aspect of the present invenetion, there is provided in vitro use of a compound for diagnosing, prognosing and/or monitoring the status of a disease associated with cells with a disrupted cell membrane, cells infected with a pathogen, dying cells or dead cells, wherein
(1) the compound is a polypeptide which comprises:
   i) an amino acid sequence as provided in any one of SEQ ID NO's 1 to 8;
   ii) an amino acid sequence which is at least 50% identical to any one or more of SEQ ID NO's 1 to 8 and which is able to detect a cell with a disrupted cell membrane, cells infected with a pathogen, dying cells or dead cells; and/or
   iii) a biologically active, soluble and/or antigenic fragment of i) or ii) and which is able to detect a cell with a disrupted cell membrane, cells infected with a pathogen, dying cells or dead cells, or
(2) the compound is an antibody or antigenic binding fragment thereof,
   (A) wherein the antibody is a monoclonal antibody, humanized antibody, single chain antibody, diabody, triabody, or tetrabody, and/or
   (B) wherein the antibody is 24/04-10B4, 42/04-42D2, 20/05-3A4 or 23/05-4C6, wherein antibodies 24/04-10B4, 42/04-42D2, 20/05-3A4 and 23/05-4C6 are produced by hydridoma cell lines deposited with the European Collection of Cell Cultures (ECACC) 24/04-10B4-24-8, 42/04-42D2-66-4-1, 20/05/3A4-26-16, 23/05-4C6-29-3 on 11 December 2007 under Deposit Reference Numbers 07121101, 07121102, 07121103, and 07121104 respectively, or by higher antibody secreting subclones of the hybridomas (24/04-10B4-24-8-FACS 9-5, 42/04-42D2-66-4-1-Clone 4, 20/05-3A4-26-16-Clone 5, 23/05-4C6-29-3-Clone 5) deposited with the ECACC on 29 April 2008, and designated Accession numbers 08042901, 08042902, 08042903, and 08042904, respectively.

Preferably, the compound is detectably labelled.

Preferably, the disease is selected from: graft versus host disease (GVHD), an autoimmune disease, an infection, a neurodegenerative disease, systemic inflammatory reaction syndrome (SIRS), cancer and injury.

Described herein is a compound for use in modulating the uptake and/or clearance of cells with a disrupted cell membrane, cells infected with a pathogen, dying cells or dead cells, or a portion thereof, or surrounding cells, in a subject, which modulates the binding of a polypeptide to a ligand of said polypeptide, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Also described is a compound for use in modulating the antigen recognition, processing and/or presentation of material derived from cells with a disrupted cell membrane, cells infected with a pathogen, dying cells or dead cells, or a portion thereof, or surrounding cells, in a subject, which modulates the binding of a polypeptide to a ligand of said polypeptide, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Further described is a compound for use in modulating an immune response to material derived from cells with a disrupted cell membrane, cells infected with a pathogen, dying cells or dead cells, or a portion thereof, or surrounding cells, in a subject, which modulates the binding of a polypeptide to a ligand of said polypeptide, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

In one example, the compound is a polypeptide.

In one example, the compound is an antibody or antigenic binding fragment thereof. Examples include, but are not limited to, a monoclonal antibody, humanized antibody, single chain antibody, diabody, triabody, or tetrabody.

In an example, the antibody is 24/04-10B4, 42/04-42D2, 20/05-3A4 or 23/05-4C6, or an antibody which comprises at least one complementarity determining region of 24/04-10B4, 42/04-42D2, 20/05-3A4 or 23/05-4C6.

In another example, the compound comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active, soluble and/or antigenic fragment of i) or ii).

In an example, the biologically active, soluble and/or antigenic fragment binds the ligand.

In a further example, the compound is conjugated to a therapeutic agent. In an example, the therapeutic agent is a cytotoxic agent. In a further example, the therapeutic agent is a drug and/or pharmacological agent.

Also described herein is a compound for use in modulating the uptake and/or clearance of cells with a disrupted cell membrane, cells infected with a pathogen, dying cells or dead cells, or a portion thereof, or surrounding cells, in a subject, which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Further described is a compound for use in modulating the antigen recognition, processing and/or presentation of material derived from cells with a disrupted cell membrane, cells infected with a pathogen, dying cells or dead cells, or a portion thereof, or surrounding cells, in a subject, which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Also described is a compound for use in modulating an immune response to material derived from cells with a disrupted cell membrane, cells infected with a pathogen, dying cells or dead cells, or a portion thereof, or surrounding cells, in a subject, which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

In one example, the compound is a polynucleotide. In one example, the polynucleotide is operably linked to a promoter capable of directing expression of the polynucleotide in a cell of an animal.

In an example, the polynucleotide down-regulates mRNA levels from a gene encoding the polypeptide. Examples include, but are not limited to, an antisense polynucleotide, a sense polynucleotide, a catalytic polynucleotide, a microRNA, and a double stranded RNA.

In one example, the polynucleotide is an antisense polynucleotide which hybridises under physiological conditions to a polynucleotide comprising any one or more of the sequence of nucleotides provided as SEQ ID NOs 9 to 16.

In another example, the polynucleotide is a catalytic polynucleotide capable of cleaving a polynucleotide comprising any one or more of the sequence of nucleotides provided as SEQ ID NOs 9 to 16.

In a further example, the polynucleotide is a double stranded RNA (dsRNA) molecule comprising an oligonucleotide which comprises at least 19 contiguous nucleotides of any one or more of the sequence of nucleotides provided as SEQ ID NOs 9 to 16, wherein the portion of the molecule that is double stranded is at least 19 basepairs in length and comprises said oligonucleotide. In an example, the polynucleotide is expressed from a single promoter, wherein the strands of the double stranded portion are linked by a single stranded portion.

In an alternate example, the polynucleotide up-regulates mRNA levels from a gene encoding the polypeptide.

In one example, the uptake and/or clearance of cells with a disrupted cell membrane, cells infected with a pathogen, dying cells or dead cells, or a portion thereof, or surrounding cells, is increased. In an alternate example, the uptake and/or clearance of cells with a disrupted cell membrane, cells infected with a pathogen, dying cells or dead cells, or a portion thereof, or surrounding cells, is decreased.

In another example, antigen recognition, processing and/or presentation of material derived from cells with a disrupted cell membrane, cells infected with a pathogen, dying cells, or dead cells, or a portion thereof, or surrounding cells, is increased. In an alternate example, antigen recognition, processing and/or presentation of material derived from cells with a disrupted cell membrane, cells infected with a pathogen, dying cells, or dead cells, or a portion thereof, or surrounding cells, is decreased.

In another example, the immune response to material derived from cells with a disrupted cell membrane, cells infected with a pathogen, dying cells or dead cells, or a portion thereof, or surrounding cells, is increased. In an alternate example, the immune responses to material derived from cells with a disrupted cell membrane, cells infected with a pathogen, dying cells or dead cells, or a portion thereof, or surrounding cells, is decreased.

The subject is suffering from a disease associated with cells with a disrupted cell membrane, cells infected with a pathogen, dying cells or dead cells, or a portion thereof. Examples of such diseases include, but are not limited to, graft versus host disease (GVHD), an autoimmune disease, an infection, a neurodegenerative disease, systemic inflammatory reaction syndrome (SIRS), cancer and injury.

Also described is an in vitro method of detecting a cell with a disrupted cell membrane, a cell infected with a pathogen, a dying cell or a dead cell, the method comprising
i) contacting a cell with a compound that binds a ligand of a polypeptide which comprises:
   a) an amino acid sequence as provided in any one of SEQ ID NO's 1 to 8;
   b) an amino acid sequence which is at least 50% identical to any one or more of SEQ ID NO's 1 to 8; and/or
   c) a biologically active, soluble and/or antigenic fragment of a) or b), and
ii) determining whether binding of the compound to the ligand is present or absent,
wherein the compound binding to the ligand indicates that a cell has a disrupted cell membrane, is infected with a pathogen, is dying or is dead.

Further described is an in vitro method of diagnosing, prognosing and/or monitoring the status of a disease associated with cells with a disrupted cell membrane, cells infected with a pathogen, dying cells or dead cells, the method comprising
i) contacting a cell with a compound that binds a ligand of a polypeptide which comprises:
   a) an amino acid sequence as provided in any one of SEQ ID NO's 1 to 8;
   b) an amino acid sequence which is at least 50% identical to any one or more of SEQ ID NO's 1 to 8; and/or
   c) a biologically active, soluble and/or antigenic fragment of a) or b), and
ii) determining whether the ligand is present or absent,
wherein the presence of the ligand provides a diagnosis, prognosis and/or status of the disease.

In an example, the compound is a polypeptide.

In a further example, the compound is a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active, soluble and/or antigenic fragment of i) or ii).

In another example, the compound is an antibody or antigenic binding fragment thereof. Examples include, but are not limited to, a monoclonal antibody, humanized antibody, single chain antibody, diabody, triabody, or tetrabody.

In an example, the antibody is 24/04-10B4, 42/04-42D2, 20/05-3A4 or 23/05-4C6, or an antibody which comprises at least one complementarity determining region of 24/04-10B4, 42/04-42D2, 20/05-3A4 or 23/05-4C6.

In an example, the compound is detectably labelled.

The method is performed *in vitro* on a sample obtained from a subject.

As noted above, examples of diseases associated with cells with a disrupted cell membrane, cells infected with a pathogen, dying cells or dead cells include, but are not limited to, graft versus host disease (GVHD), an autoimmune disease, an infection, a neurodegenerative disease, systemic inflammatory reaction syndrome (SIRS), cancer and injury.

Also described is an in vitro method of monitoring a therapy, the method comprising;
i) exposing a cell to the therapy, and
ii) detecting a cell with a disrupted cell membrane, a dying cell or a dead cell, or a portion thereof, using a method of the invention.

In one example, the therapy is intended to kill a cell and the presence of a cell with a disrupted cell membrane, a dying cell or a dead cell indicates that the therapy is effective. In an alternate example, the therapy is not intended to kill a cell and the presence of a cell with a disrupted cell membrane, a dying cell or a dead cell indicates that the therapy has an unwanted side effect.

The cell in step i) is *in vitro.*

The subject is suffering from a disease associated with cells with a disrupted cell membrane, cells infected with a pathogen, dying cells or dead cells. In one example, the subject has cancer or an infection.

In a further example, step ii) is performed on a sample obtained from a subject.

The therapy can be any type of procedure. Examples include, but are not limited to, drug therapy or radiotherapy.

Further described is an in vitro method of identifying a ligand of a polypeptide, the method comprising:
a) obtaining a cell with a disrupted cell membrane, a cell infected with a pathogen, a dying cell or a dead cell, or a portion thereof,
b) contacting the polypeptide with a candidate compound obtained from the cell or portion thereof,
c) determining whether the compound binds the polypeptide,
wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active, soluble and/or antigenic fragment of i) or ii).

Further described is an in vitro method of identifying a ligand of a polypeptide, the method comprising:
a) obtaining a cell with a disrupted cell membrane, a cell infected with a pathogen, a dying cell or a dead cell, or a portion thereof,
b) exposing the polypeptide to a binding partner which binds the polypeptide, and a candidate compound obtained from the cell or portion thereof, and
c) assessing the ability of the candidate compound to compete with the binding partner for binding to the polypeptide,
wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active soluble, and/or antigenic fragment of i) or ii).

In an example, the candidate compound is a protein, or protein-associated molecule such as a carbohydrate.

In a further example, the candidate compound is located intracellularly in viable cells and exposed after membrane disruption.

In another example, the candidate compound may be obtained from a combination of one or more of a cell with a disrupted cell membrane, a cell infected with a pathogen, a dying cell, a dead cell or a portion thereof.

In an example, the binding partner is an antibody.

In a further example, the binding partner is detectably labelled.

Also described is an in vitro method of distinguishing between an early stage apoptotic cell and a late stage apoptotic cell, necrotic cell or a dead cell, the method comprising
i) contacting a cell with a compound that binds a ligand of a polypeptide which comprises:
   a) an amino acid sequence as provided in any one of SEQ ID NO's 1 to 8;
   b) an amino acid sequence which is at least 50% identical to any one or more of SEQ ID NO's 1 to 8; and/or
   c) a biologically active, soluble and/or antigenic fragment of a) or b), and
ii) determining whether binding of the compound to the ligand is present or absent,
wherein the compound binding to the ligand indicates that the cell is a late stage apoptotic cell, necrotic cell or dead cell.

In an example, the method further comprises detecting an apoptotic or necrotic cell, or a cell with a disrupted membrane. This example can be performed by any method known in the art such as detecting phosphotidylserine exposure (which can be detected by Annexin V binding).

Further described herein is a population of dendritic cells or precursors thereof for use in modulating an immune response to an antigen in a subject, said modulating comprising
i) obtaining a population of dendritic cells or precursors thereof,
ii) modulating the production and/or activity of a polypeptide which comprises:
   a) an amino acid sequence as provided in any one of SEQ ID NO's 1 to 8;
   b) an amino acid sequence which is at least 50% identical to any one or more of SEQ ID NO's 1 to 8; and/or
   c) a biologically active, soluble and/or antigenic fragment of a) or b),
iii) contacting the dendritic cells or precursors thereof with the antigen, and
iv) administering the dendritic cells or precursors thereof to the subject.

In an example, step iii) comprises contacting the dendritic cells or precursors thereof with a cell with a disrupted cell membrane, a cell infected with a pathogen, a dying cell, a dead cell, and/or a portion thereof, comprising said antigen.

In a further example, step iii) comprises
a) obtaining a cell comprising the antigen,
b) disrupting the cell membrane of the cell, and
c) contacting the product of step b) with the dendritic cells or precursors thereof.

In an example, the modulating further comprises, before step ii), enriching the population for cells expressing the polypeptide.

In an example, steps ii) and iii) are conducted concurrently.

In an example, the cell with a disrupted cell membrane, dying cell or dead cell is a cancer cell.

In another example, the production and/or activity of the polypeptide is increased. In an alternate example, the production and/or activity of the polypeptide is decreased.

In an example, the precursor is a monocyte.

In an example, an immune response to the antigen is increased.

In an example, the subject is an animal, eg a mammal such as a human, dog, cat, horse, cow, or sheep. In one example, the subject is a human.

Also described is use of a compound which modulates the binding of a polypeptide to a ligand of said polypeptide for the manufacture of a medicament for modulating the uptake and/or clearance of cells with a disrupted cell membrane, cells infected with a pathogen, dying cells or dead cells, or a portion thereof, or surrounding cells, in a subject, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Further described is use of a compound which modulates the binding of a polypeptide to a ligand of said polypeptide for the manufacture of a medicament for modulating the antigen recognition, processing and/or presentation of material derived from cells with a disrupted cell membrane, cells infected with a pathogen, dying cells or dead cells, or a portion thereof, or surrounding cells, in a subject, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Also described is use of a compound which modulates the binding of a polypeptide to a ligand of said polypeptide for the manufacture of a medicament for modulating an immune response to material derived from cells with a disrupted cell membrane, cells infected with a pathogen, dying cells or dead cells, or a portion thereof, or surrounding cells, in a subject, wherein the polypeptide comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii).

Further described is use of a compound which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii),
for the manufacture of a medicament for modulating the uptake and/or clearance of cells with a disrupted cell membrane, cells infected with a pathogen, dying cells or dead cells, or a portion thereof, or surrounding cells, in a subject.

Also described is use of a compound which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii),
for the manufacture of a medicament for modulating the antigen recognition, processing and/or presentation of material derived from cells with a disrupted cell membrane, cells infected with a pathogen, dying cells or dead cells, or a portion thereof, or surrounding cells, in a subject.

Further described is use of a compound which modulates the production of a polypeptide which comprises:
i) an amino acid sequence as provided in any one of SEQ ID NO's 1 to 8;
ii) an amino acid sequence which is at least 50% identical to any one or more of SEQ ID NO's 1 to 8; and/or
iii) a biologically active and/or antigenic fragment of i) or ii), for the manufacture of a medicament for modulating an immune response to material derived from cells with a disrupted cell membrane, cells infected with a pathogen, dying cells or dead cells, or a portion thereof, or surrounding cells, in a subject.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying figures.

### BRIEF DESCRIPTION OF THE ACCOMPANYING DRAWING

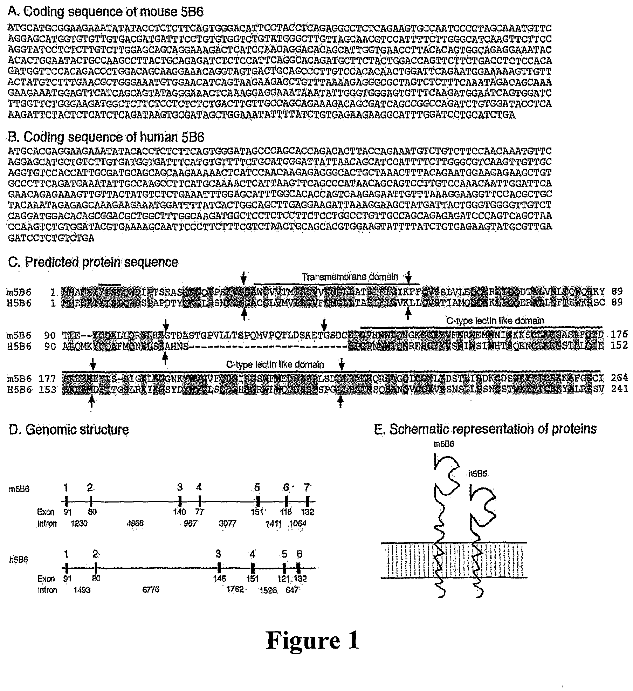

FIGURE 1. The genomic structure and predicted protein structure encoded by the mouse (m) and human (h) *5B6* genes. The full-length cDNA encoding (A) mouse and (B) human 5B6. (C) Protein sequence alignment of the predicted protein sequence encoded by mouse and human *5B6.* Sequence identity is highlighted in dark grey, similarity is shown in a light grey. Arrowheads denote exon boundaries. (D) Gene structures of mouse and human *5B6,* determined by alignment of the cDNA to the genomic sequence databases of the C57BL/6J mouse (UCSC assembly February 2006) and human databases (UCSC assembly March 2006) respectively, are represented schematically. Exons encoding the coding region of *5B6* genes are denoted by black boxes and the size (bp) of the exons and introns are shown below. (E) A schematic representation of the mouse and human 5B6 proteins.

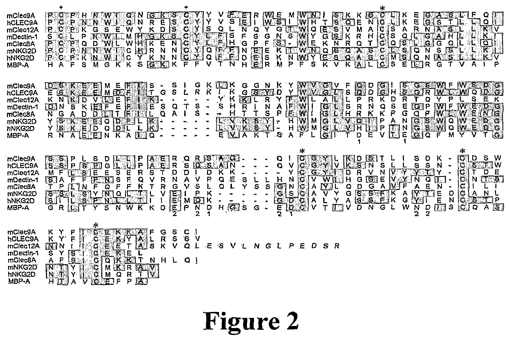

**FIGURE 2****.** Alignment of the CTLD of mouse and human 5B6 (Clec9A) to proteins that share sequence homology. Rat mannose binding protein A (MBP-A) is included for comparison as a classical C-type lectin domain that has functional carbohydrate recognition domains. Grey boxes indicate conserved residues, (+) indicates additional pair of cysteine residues involved in protein homodimeration, (*) marks the conserved cysteine residues that form disulfide bonds. The residues that ligate Ca²⁺ in the MBP-A are designated 1 and 2.

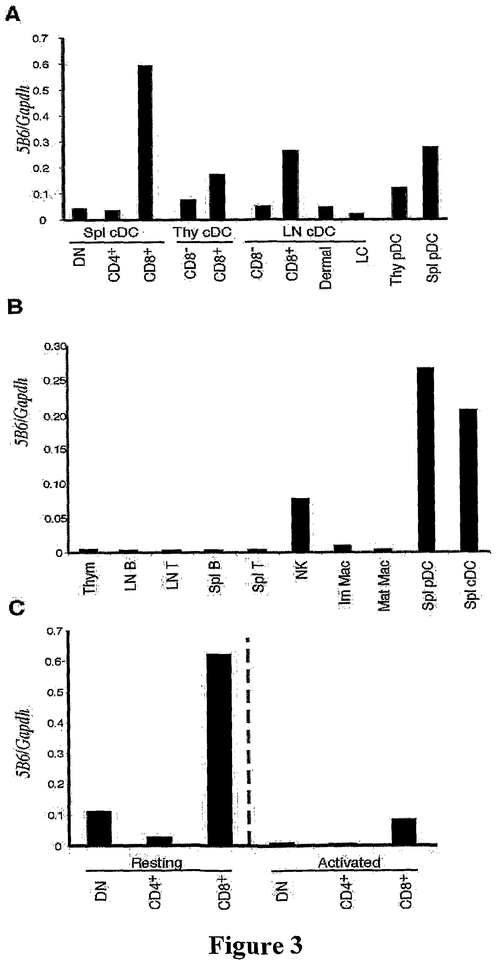

**FIGURE 3****.** Gene expression profiles of mouse *5B6.* Real-time PCR was used to study the expression profiles of the *5B6* gene relative to *Gapdh* in (A) lymphoid organ steady state DC including splenic cDC subsets; DN, CD4⁺ and CD8⁺, thymic cDC subsets; CD8⁻ and CD8⁺, LN cDC subsets; CD8⁻, CD8⁺, Dermal and Langerhans' cells (LC) and in thymic and splenic pDC. (B) Haemopoietic cells including thymocytes (thym), lymph node (LN) B and T cells, spleen (spl) B and T cells, NK cells, immature macrophages (im mac), mature macrophages (mat mac), splenic pDC and cDC. (C) Splenic cDC isolated from both steady state (resting) mice and after 3 hours *in vivo* activation with LPS and CpG.

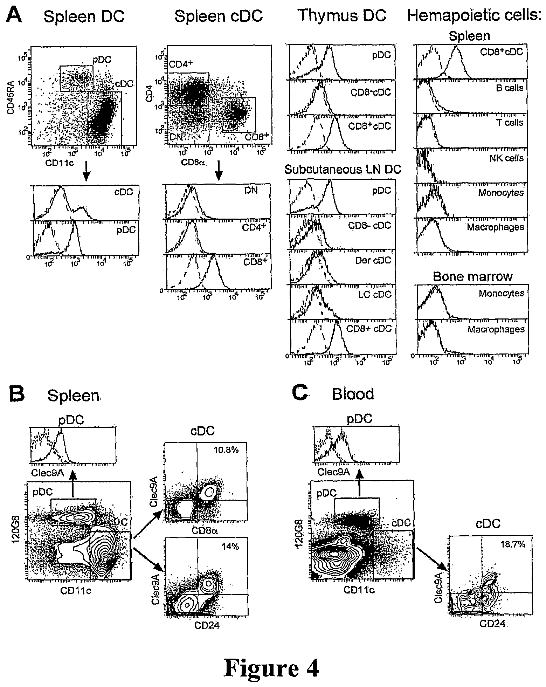

**FIGURE 4****.** Surface expression of m5B6 (Clec9A) protein on DCs and other hemopoietic cells. (A) The DCs were purified and surface labeled by 4-color immunofluorescent staining. DCs were stained with mAb against CD11c (N418-PeCy7), CD45RA (14.8-APC), CD8 (53-6.7-APC-Cy7) and m5B6 (10B4-biotin). Splenic DCs were also stained with CD4 (GK1.5-FITC), thymic DCs with Sirpα (p84-FITC), and subcutaneous LN DCs with CD205 (NLDC-145-FITC). Splenic cDCs were divided into CD4⁺cDCs (CD11^{hi}CD45RA⁻CD4⁺CD8⁻), DN cDCs (CD11^{hi}CD45RA⁻CD4⁻CD8⁻) and CD8⁺cDCs (CD11^{hi}CD45RA⁻CD8⁺CD4⁻); thymic cDCs were divided into CD8⁻cDCs (Sirpα^{hi}CD8^{lo}) and CD8⁺cDCs (Sirpa^{lo}CD8⁺); and LN cDCs into CD8⁻cDC (CD11c⁺CD205⁻CD8⁻), dermal cDCs (CD11c⁺CD205^{int}CD8⁻), Langerhans' cells (CD11c⁺CD205^{hi}CD8⁻) and CD8⁺cDCs (CD11c⁺CD205^{hi}CD8⁺), as described previously ³¹. pDCs were identified as CD11c^{int}CD45RA⁺. Splenocytes were stained with mAb against CD3 (KT3-1.1-FITC), CD19 (1D3-PeCy7), NK1.1 (PK136-PeCy7), CD49b (Hmα2-APC) and B cells (CD19⁺CD3⁻), T cells (CD19⁻CD3⁺) and NK cells (NK1.1⁺CD49b⁺CD3⁻) were identified. Splenic macrophages were enriched as indicated in Materials and Methods and stained with CD11b (M1/70-Cy5) and F4/80-FITC and defined as CD11b^{hi}F4/80⁺. Bone marrow cells and splenocytes were stained with mAb against CD11b (Ml/70-Cy5) and Ly6C (5075-3.6-FITC) and monocytes were defined as side-scatter^{lo}Ly6C^{hi}CD11b^{hi}. Bone marrow macrophages were Ly6C^{int}CD11b^{hi}. Cell populations were counterstained with SA-PE and analysed for m5B6 expression. The solid line represents m5B6 staining on gated cells, the dotted line represents staining of the gated cells with an isotype-matched control. (B) Enriched preparations of splenic DCs were stained with mAb against m5B6 (10B4-biotin), CD11c (N418-Quantum dot 655), CD8α (YTS-169-PercpCy5.5 and CD24 (M1/69-Alexa 633) and 120G8-FITC, then counterstained with SA-PE. pDCs (CD11c^{int}120G8⁺) and cDCs (CD11c^{hi}120G8⁻) were analysed for expression of m5B6. m5B6 expression correlated with CD8α and CD24 expression on cDCs. Most splenic pDCs expressed m5B6. (C) An enriched preparation of blood DCs was stained in parallel with the splenic DCs (B) using the same mAbs and analysed using identical gating strategies. Blood DCs do not express CD8α, but do express CD24. Similar to splenic DCs, blood DCs expressing CD24 also co-expressed 5B6 pDCs from the blood, like their splenic counterpart, expressed m5B6.

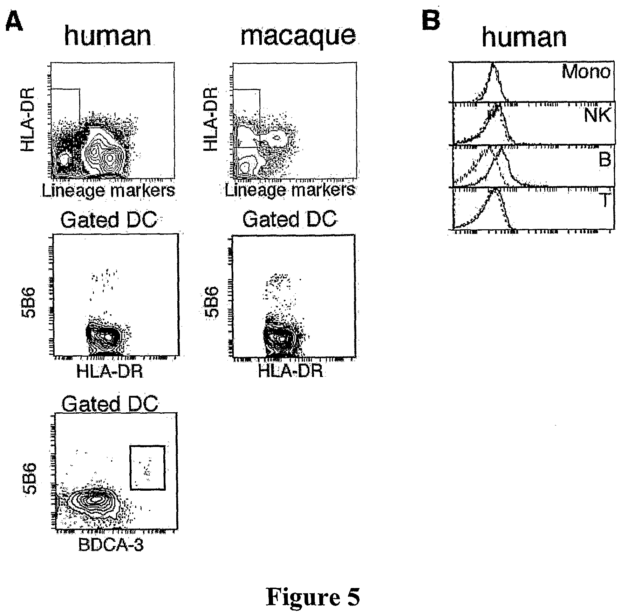

**FIGURE 5****.** Expression of 5B6 on human and macaque DC and haemopoietic cells. (A) Human and macaque peripheral blood mononuclear cells (PBMCs) were isolated and surface immunofluorescence labeled with mAb against HLADR, BDCA3, 5B6, and a PE-conjugated Lineage cocktail including CD3 (T cells), CD14 (monocytes), CD19 (B cells) and CD56 (Natural killer cells). Blood DC were gated as HLADR⁺Lineage (PE)⁻ and further analysed for their expression of 5B6 (human and macaque) and BDCA3 (human). (B) Human PBMC were surface immunofluorescence labeled with mAb against the required surface markers and 5B6. Monocytes (CD14⁺), NK cells (NKp46⁺), T cells (CD3⁺), and B cells (CD19⁺) were gated and analysed for their expression of 5B6 (solid line). The dotted line represents staining of the gated cells with an isotype matched control.

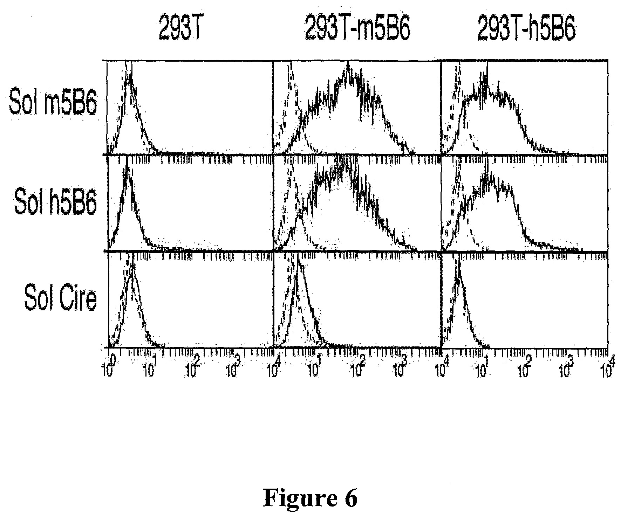

**FIGURE 6****.** Binding of soluble 5B6 to membrane bound 5B6 on transiently transfected 293T cells. 293T cells were transiently transfected with expression constructs encoding full length untagged m5B6 (283T-m5B6), h5B6 (293T-h5B6) or no DNA (293T). Two days later, cells were harvested and surface immunofluorescence labeled using soluble FLAG-tagged m5B6, h5B6 and Cire, and binding detected using biotinylated anti-Flag mAb 9H10 and Streptavidin PE. Live cells were gated on forward scatter and propidium iodide exclusion and analysed for their surface binding of soluble 5B6 (solid line) relative to control staining with anti-Flag Ab and streptavidin-PE (dashed line).

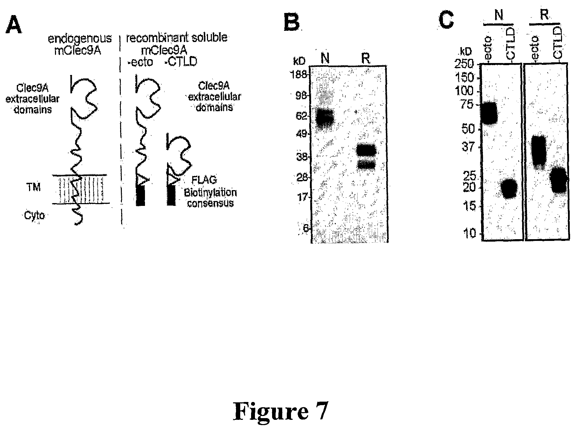

**FIGURE 7****.** Generation of soluble recombinant ectodomains of Clec9A. (A) A schematic representation of the endogenous and recombinant soluble mClec9A proteins. The endogenous protein includes the Clec9A extracellular domains, the transmembrane (TM) and the cytoplasmic (cyto) domains. Two forms of recombinant soluble mClec9A protein were generated: mClec9A-ecto which consists of the full Clec9A ectodomain, a FLAG tag and a biotinylation consensus sequence (predicted mol wt ∼27kDa); and mClec9A-CTLD which consists of the Clec9A-CTLD, FLAG tag and biotinylation consensus sequence (predicted mol wt ∼19.7 kDa). (B) Western blot analysis of endogenous mClec9A expression. DCs were produced from cultures of bone marrow with Flt3L (Naik et al., 2005) and DC lysates electrophoresed under non-reducing (N) and reducing (R) conditions. Blots were hybridised using anti-mClec9A Ab (24/04-10B4) and binding detected using HRP conjugated anti-rat Ig and Enhanced Chemiluminescence-Plus (Amersham). mClec9A was observed to migrate as a dimer under non-reducing conditions. (C) Western blot analysis of biotinylated recombinant soluble mClec9A protein. Biotinylated mClec9A-ecto and mClec9A-CTLD were electrophoresed under nonreducing (N) and reducing (R) conditions, and proteins detected using SA-HRP and Enhanced Chemiluminescence (Amersham). Recombinant mClec9A-ecto, like endogenous mClec9A, was observed to migrate as a dimer under nonreducing conditions and as a monomer under reducing conditions whereas mClec9A-CTLD migrated as a monomer under all conditions (B, C).

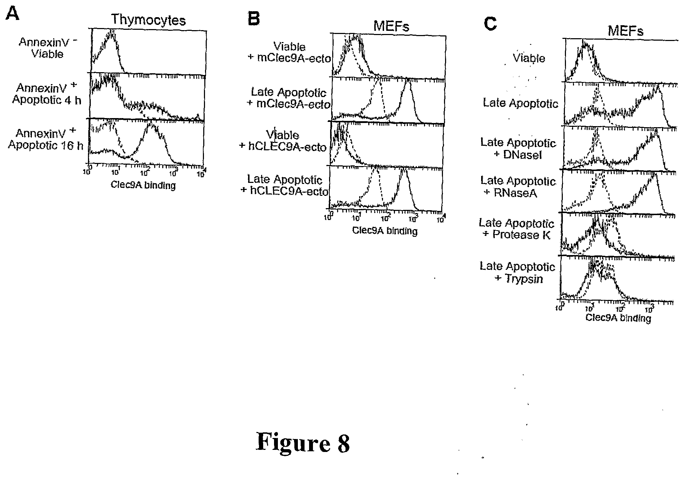

**FIGURE 8****.** Binding of Clec9A ectodomains to dead cells. (A) Thymocytes were γ-irradiated (5Gy) then cultured for 4 h or 16 h at 37°C in RPMI-1640 containing 10% FCS, to follow the progress of apoptotic death. Samples were incubated with biotinylated mClec9A-ecto (solid line), or biotinylated Cire-ecto as a background control (dashed line) and binding detected using SA-PE. Thymocytes were stained with Annexin V-FITC, analysed by flow cytometry, and gated as Annexin V⁻ (viable) cells or Annexin V⁺ (apoptotic) for analysis of Clec9A binding. At 4 h 41% of the Annexin V⁺ cells were PI⁺; at 16 h 90% of the Annexin V⁺ cells were PI⁺. The backgrounds observed using biotinylated Cire-ecto were similar to those observed with second stage reagents alone. (B) MEFs overexpressing Noxa to inactivate Mcl-1 (van Delft et al., 2006) were grown to approximately 80% confluence then induced to undergo apoptosis by treatment with 2.5µM ABT-737 for 16h. Control untreated MEFs (viable) and ABT-737 treated MEFs (late apoptotic) were harvested and incubated with mClec9A-ecto (solid line), hCLEC9A-ecto (solid line), or Cire-ecto (background control, dashed line). Binding was detected using biotinylated anti-FLAG mAb, SA-PE and flow cytometry. 90% of the untreated MEFs were viable based on normal forward scatter (FSC) and PI exclusion (PI⁻), whereas 95% of the ABT-737 treated MEFs were dead based on reduced FSC and positive PI staining. (C) Control untreated MEFs (viable) and ABT-737 treated MEFs (2.5µM ABT-737 for 16h; late apoptotic) were incubated in PBS alone or in the presence of DNaseI, RNaseA, protease K or trypsin. Cells were washed extensively to remove nucleases and proteases, then incubated with biotinylated mClec9A-ecto (solid line), or biotinylated Cire-ecto as a control (dashed line). Binding was detected using SA-PE and flow cytometry. 80% of the untreated MEFs were viable based on normal FSC and PI exclusion, whereas 97% of the ABT-737 treated MEFs were dead based on reduced FSC and positive PI staining.

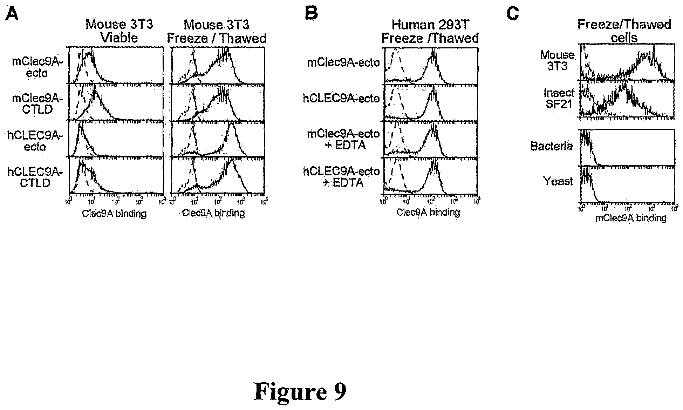

**FIGURE 9****.** Clec9A binding is mediated via the CTLD and is directed against a protein expressed by diverse species. (A) Viable or freeze-thawed mouse fibroblasts (3T3 cell line) were incubated with biotinylated mClec9A or hCLEC9A ectodomains or CTLD (solid line), or with biotinylated control (Cire-ecto, dashed line). (B) Human 293T cells were freeze-thawed then incubated with biotinylated mClec9A-ecto, hCLEC9A-ecto (solid line) or Cire (dashed line) in the absence or presence of 5mM EDTA (solid line). (C) Mouse 3T3 cells, insect SF21 cells, bacterial JM109 cells and yeast (*Pichia pastoris*) cells were freeze-thawed twice then incubated with biotinylated mClec9A-ecto (solid line), or biotinylated control (Cire-ecto, dashed line). Binding in (A)-(C) was detected using SA-PE and flow cytometry.

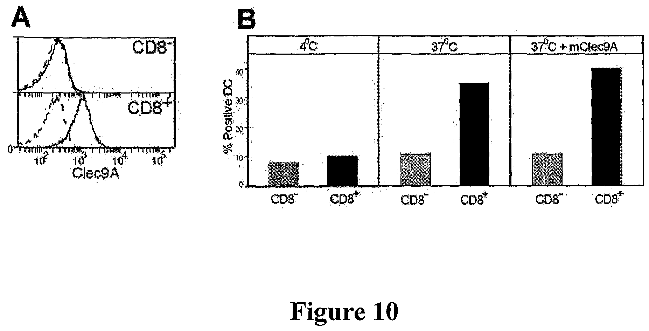

**FIGURE 10****.** Soluble mClec9A does not block uptake of dead cells by CD8⁺ DC. (A) Surface expression of Clec9A on splenic cDCs. DCs were blocked using rat Ig and anti FcR mAb (2.4G2) and surface labelled using mAb against CD11c (N418-PE-Cy7), CD45RA (14.8-APC), CD8 (53-6.7-APC-Cy7), CD172a (p84-FITC) and either Clec9A (24/04-10B4-biotin) or an isotype control-biotin (IgG2a-kappa; BD Pharmingen), then counterstained with SA-PE and analysed by flow cytometry. Splenic cDCs were gated as CD11c^{hi} CD45RA⁻ CD172a^{hi} CD8⁻ (CD8⁻) or as CD11c^{hi} CD45RA⁻ CD172a^{lo} CD8⁺ (CD8⁺) and analysed for their surface expression of Clec9A. The solid line represents mClec9A staining on gated cells, the dotted line represents staining of the gated cells using an isotype matched control. (B) Phagocytic uptake of dead cells by splenic cDCs. Splenocytes were freeze-thawed then labelled with PI, then incubated in the absence or presence of mClec9A-ecto. DCs were surface labelled with mAb against CD11c and CD8, then cocultured with the PI labelled splenocytes for 3h at 4°C or at 37°C. DCs were gated.as CD8⁺ (CD11c^{hi} CD8⁺) or CD8⁻ (CD11c^{hi}CD8⁻) and analysed for the percentage of cells that were PI positive as a measure of dead cell uptake.

### KEY TO THE SEQUENCE LISTING

SEQ ID NO:1- Human 5B6.
SEQ ID NO:2 - Murine 5B6.
SEQ ID NO:3 - Chimpanzee 5B6.
SEQ ID NO:4 - Rhesus monkey 5B6.
SEQ ID NO:5 - Dog 5B6.
SEQ ID NO:6 - Cow 5B6.
SEQ ID NO:7 - Horse 5B6.
SEQ ID NO: 8 - Rat 5B6.
SEQ ID NO:9 - Open reading frame encoding human 5B6.
SEQ ID NO:10 - Open reading frame encoding murine 5B6.
SEQ ID NO:11 - Open reading frame encoding chimpanzee 5B6.
SEQ ID NO: 12 - Open reading frame encoding rhesus monkey 5B6.
SEQ ID NO: 13 - Open reading frame encoding dog 5B6.
SEQ ID NO:14 - Open reading frame encoding cow 5B6.
SEQ ID NO:15 - Open reading frame encoding horse 5B6.
SEQ ID NO:16 - Open reading frame encoding rat 5B6.
SEQ ID NO's 17 to 28, 38 and 39- Oligonucleotide primers.
SEQ ID NO:29 - Antigenic fragment of murine 5B6.
SEQ ID NO:30 - Antigenic fragment of human 5B6.
SEQ ID NO:31 - Biotinylation consensus sequence.
SEQ ID NO:32 - Partial sequence of mouse Clec12a.
SEQ ID NO:33 - Partial sequence of mouse Dectin-1.
SEQ ID NO:34 - Partial sequence of mouse Clec8a.
SEQ ID NO:35 - Partial sequence of mouse NKG2D.
SEQ ID NO:36 - Partial sequence of human NKG2D.
SEQ ID NO:37 - Partial sequence of rat MBP-A.
SEQ ID N0:40 - Soluble mouse 5B6 including stalk.
SEQ ID NO:41 - Soluble human 5B6 including stalk.
SEQ ID NO:42 - Soluble mouse 5B6 without stalk.
SEQ ID NO:43 - Soluble human 5B6 without stalk.
SEQ ID NO:44 - Soluble flag tagged mouse 5B6 including stalk.
SEQ ID NO:45 - Soluble flag tagged human 5B6 including stalk.
SEQ ID NO:46 - Soluble flag tagged mouse 5B6 without stalk.
SEQ ID NO:47 - Soluble flag tagged human 5B6 without stalk.

### DETAILED DESCRIPTION OF THE INVENTION

### General Techniques and Definitions

Unless specifically defined otherwise, all technical and scientific terms used herein shall be taken to have the same meaning as commonly understood by one of ordinary skill in the art (e.g., in cell culture, molecular genetics, molecular biology, immunology, immunohistochemistry, protein chemistry, and biochemistry).

Unless otherwise indicated, the recombinant protein, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

The phrase "cells with a disrupted cell membrane, cells infected with a pathogen, dying cells or dead cells", and variations thereof, as used herein includes situations where the cell can have, where possible, one or more of these features. For example, the cell could have a disrupted membrane, be infected with a pathogen and be dying.

As used herein, the term "cells with a disrupted cell membrane" or "cell with a disrupted cell membrane" refers to cells where the integrity of the cell membrane has been compromised. This includes cells with pores, as well as damaged or ruptured cells.

As used herein, the term "dying cell" or "dying cells" refers to later stage apoptotic cells or necrotic cells. Preferably, the dying cell(s) are AnnexinV⁺ or they are propidium iodide (PI)⁺. For dying cells with a nuclei, it is preferred that they are AnnexinV⁺ and PI⁺. In a particularly preferred embodiment, the dying cells are at least AnnexinV⁺. In yet another embodiment, the necrotic cells are secondary necrotic cells.

As used herein, "early apoptotic cell" or "early apoptotic cells" includes cells that are AnnexinV⁺ and PI⁻.

As used herein, the term "dead cell" or "dead cells" refers to cell(s) that has passed a point of no return in the death process and which changes cannot be reversed. The cell(s) may have died through apoptosis or necrosis.

With regard to the phrase "cells infected with a pathogen", the term pathogen includes any organism which can infect a cell. Examples include, but are not limited to, viruses, protozoa and bacteria.

As used herein, the term "or portion thereof" refers to any part of cells with a disrupted cell membrane, cells infected with a pathogen, dying cells or dead cells which comprise a ligand of a polypeptide defined herein. Examples include, but are not limited to, blebs and cell homogenates/lysates.

As used herein, the term "uptake and/or clearance" of cells with a disrupted cell membrane, cells infected with a pathogen, dying cells or dead cells, or a portion thereof, refers to the removal of cellular material, such a proteins or fragments thereof, of the cells. In an embodiment, dendritic cells are responsible, at least in part, for the uptake and/or clearance of the cells. Preferably, the dendritic cells are 5B6⁺ (also known as Clec9A⁺).

As used herein, the term "surrounding cells" refers to cells in close proximity to one or more of cells with a disrupted cell membrane, cells infected with a pathogen, dying cells or dead cells.

As used herein, the terms "treating", "treat" or "treatment" include administering a therapeutically effective amount of a compound sufficient to reduce or eliminate at least one symptom of the specified condition.

As used herein, the terms "preventing", "prevent" or "prevention" include administering a therapeutically effective amount of a compound sufficient to stop or hinder the development of at least one symptom of the specified condition.

As used herein, the term "diagnosing" or variations thereof refers to the detection of a disease.

As used herein, the term "prognosing" or variations thereof refers to an assessment of the future outcome of a disease.

As used herein, the term "monitoring the status" or variations thereof refers to determining the stage of a disease. The status can be determined before, during and/or after a subject has been administered with a treatment for the disease.

As used herein, the term "5B6" and "Clec9A" refers to a polypeptide which comprises;
i) an amino acid sequence as provided in any one of SEQ ID NO's 1 to 8;
ii) an amino acid sequence which is at least 50% identical to any one or more of SEQ ID NO's 1 to 8; and/or
iii) a biologically active, soluble and/or antigenic fragment of i) or ii). Preferably, the polypeptide is at least expressed on a subset of dendritic cells.

As used herein, the "sample" can be any biological material suspected of having cells with a disrupted cell membrane, cells infected with a pathogen, dying cells or dead cells, or a portion thereof. Examples include, but are not limited to, blood, for example, whole peripheral blood, cord blood, foetus blood, bone marrow, plasma, serum, urine, cultured cells, saliva or urethral swab, lymphoid tissues, for example tonsils, peyers patches, appendix, thymus, spleen and lymph nodes, and any biopsy samples taken for routine screening, diagnostic or surgical reason such as tumour biopsy or bioposy of inflamed organs/ tissues. The sample may be tested directly or may require some form of treatment prior to testing. For example, a biopsy sample may require homogenization to produce a cell suspension prior to testing. Furthermore, to the extent that the biological sample is not in liquid form (for example, it may be a solid, semi-solid or a dehydrated liquid sample), it may require the addition of a reagent, such as a buffer, to mobilize the sample. The mobilizing reagent may be mixed with the sample prior to placing the sample in contact with a compound as defined herein.

As used herein, the terms "conjugate", "conjugated" or variations thereof are used broadly to refer to any form of covalent or non-covalent association between a compound useful for the invention and a therapeutic agent, or to placing a compound useful for the invention and a therapeutic agent in close proximity to each other such as in a liposome.

As used herein, the term "immune response" refers to an alteration in the reactivity of the immune system of a subject in response to an antigen and may involve antibody production, induction of cell-mediated immunity, complement activation and/or development of immunological tolerance.

As used herein, the term "candidate compound obtained from the cell" is used in its broadest context to the extent that it not only includes molecules, especially proteins, obtained directly from the cells, but includes molecules prepared from other sources but are known, or have to be shown, to be present in cells with a disrupted cell membrane, cells infected with a pathogen, dying cells or dead cells, or a portion thereof.

As used herein, the term "modulating the production and/or activity" refers to any mechanism which can be used to alter the level, or biological function, of a polypeptide as defined herein. Such methods are described herein, as well as in WO 2009/026660. In a preferred embodiment, activity is modulated by blocking the binding of the polypeptide to its ligand(s).

As used herein, the phrase "disrupting the cell membrane of the cell" refers to any method that compromises the integrity of the cell membrane. Examples of such methods include, but are not limited to, irradiation, exposure to a detergent and freeze/thawing. In an embodiment, the cell is killed by the method.

### Compounds

The present inventors have now shown, for the first time, that 5B6 (also referred to in the art as CLEC9A and HEEE9341) which is expressed in a subset of dendritic cells, binds a ligand on cells with a disrupted cell membrane, cells infected with a pathogen, dying cells and dead cells, or a portion thereof. This enables compounds which modulate the binding of 5B6 to the ligand, and/or compounds which modulate the production of 5B6 or the ligand to be used in a wide variety of diagnostic, prognostic and therapeutic procedures.

Compounds useful for the invention bind, preferably specifically bind, 5B6 or the ligand. The compound may be, for example, a purified and/or recombinant naturally occurring ligand or a synthetic ligand. The binding between a compound and 5B6, or a compound and the ligand, may be mediated by covalent or non-covalent interactions or a combination of covalent and non-covalent interactions. When the interaction of the compound and 5B6, a compound and the ligand, produces a non-covalently bound complex, the binding which occurs is typically electrostatic, hydrogen-bonding, or the result of hydrophilic/lipophilic interactions. In a preferred embodiment, the compound is a purified and/or recombinant polypeptide. Particularly preferred compounds are purified and/or recombinant antibodies or antigenic binding fragments thereof, or sulble forms of 5B6 described herein.

Although not essential, the compound may bind specifically to 5B6 or the ligand. The phrase "specifically binds", means that under particular conditions, the compound binds 5B6 or the ligand and does not bind to a significant amount to other, for example, proteins or carbohydrates. In one embodiment, the compound specifically binds 5B6 and not other molecules in a sample obtained from a subject comprising dendritic cells. In another embodiment, the compound specifically binds the ligand and not other molecules in a sample obtained from a subject comprising cells with a disrupted cell membrane, cells infected with a pathogen, dying cells or dead cells, or a portion thereof. Specific binding under such conditions may require an antibody that is selected for its specificity. In another embodiment, a compound is considered to "specifically binds" if there is a greater than 10 fold difference, and preferably a 25, 50 or 100 fold greater difference between the binding of the compound when compared to another protein.

A variety of immunoassay formats may be used to select antibodies specifically immunoreactive. For example, surface labelling and flow cytometric analysis or solid-phase ELISA immunoassays are routinely used to select antibodies specifically immunoreactive with a protein or carbohydrate. See Harlow & Lane (*supra*) for a description of immunoassay formats and conditions that can be used to determine specific immunoreactivity.

Antibodies may exist as intact immunoglobulins, or as modifications in a variety of forms including, for example, but not limited to, domain antibodies including either the VH or VL domain, a dimer of the heavy chain variable region (VHH, as described for a camelid), a dimer of the light chain variable region (VLL), Fv fragments containing only the light and heavy chain variable regions, or Fd fragments containing the heavy chain variable region and the CH1 domain. A scFv consisting of the variable regions of the heavy and light chains linked together to form a single-chain antibody (Bird et al., 1988; Huston et al., 1988) and oligomers of scFvs such as diabodies and triabodies are also encompassed by the term "antibody". Also encompassed are fragments of antibodies such as Fab, (Fab')₂ and FabFc₂ fragments which contain the variable regions and parts of the constant regions. CDR-grafted antibody fragments and oligomers of antibody fragments are also encompassed. The heavy and light chain components of an Fv may be derived from the same antibody or different antibodies thereby producing a chimeric Fv region. The antibody may be of animal (for example mouse, rabbit or rat) or human origin or may be chimeric (Morrison et al., 1984) or humanized (Jones et al., 1986), and UK 8707252). As used herein the term "antibody" includes these various forms. Using the guidelines provided herein and those methods well known to those skilled in the art which are described in the references cited above and in such publications as Harlow & Lane (*supra*) the antibodies for use in the present invention can be readily made.

The antibodies may be Fv regions comprising a variable light (V_{L}) and a variable heavy (V_{H}) chain. The light and heavy chains may be joined directly or through a linker. As used herein a linker refers to a molecule that is covalently linked to the light and heavy chain and provides enough spacing and flexibility between the two chains such that they are able to achieve a conformation in which they are capable of specifically binding the epitope to which they are directed. Protein linkers are particularly preferred as they may be expressed as an intrinsic component of the Ig portion of the fusion polypeptide.

In another embodiment, recombinantly produced single chain scFv antibody, preferably a humanized scFv, is used in the invention.

### Monoclonal Antibodies

Monoclonal antibodies directed against 5B6 epitopes or epitopes of the ligand can be readily produced by one skilled in the art. An example of a method for producing such antibodies using the 5B6 epitope RWLWQDGSSPSPGLLPAERSQSANQVC-OH) (SEQ ID NO:30) is provided in the Examples section.

The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against suitable epitopes can be screened for various properties; i.e. for isotype and epitope affinity.

Animal- derived monoclonal antibodies can be used for both direct *in vivo* and extracorporeal immunotherapy. However, it has been observed that when, for example, mouse-derived monoclonal antibodies are used in humans as therapeutic agents, the patient produces human anti-mouse antibodies. Thus, animal-derived monoclonal antibodies are not preferred for therapy, especially for long term use. With established genetic engineering techniques it is possible, however, to create chimeric or humanized antibodies that have animal-derived and human-derived portions. The animal can be, for example, a mouse or other rodent such as a rat.

If the variable region of the chimeric antibody is, for example, mouse-derived while the constant region is human-derived, the chimeric antibody will generally be less immunogenic than a "pure" mouse-derived monoclonal antibody. These chimeric antibodies would likely be more suited for therapeutic use, should it turn out that "pure" mouse-derived antibodies are unsuitable.

Methodologies for generating chimeric antibodies are available to those in the art. For example, the light and heavy chains can be expressed separately, using, for example, immunoglobulin light chain and immunoglobulin heavy chains in separate plasmids. These can then be purified and assembled *in vitro* into complete antibodies; methodologies for accomplishing such assembly have been described (see, for example, Sun et al., 1986). Such a DNA construct may comprise DNA encoding functionally rearranged genes for the variable region of a light or heavy chain of an antibody linked to DNA encoding a human constant region. Lymphoid cells such as myelomas or hybridomas transfected with the DNA constructs for light and heavy chain can express and assemble the antibody chains.

*In vitro* reaction parameters for the formation of IgG antibodies from reduced isolated light and heavy chains have also been described. Co-expression of light and heavy chains in the same cells to achieve intracellular association and linkage of heavy and light chains into complete H2L2 IgG antibodies is also possible. Such co-expression can be accomplished using either the same or different plasmids in the same host cell.

In another preferred embodiment of the present invention the antibody is humanized, that is, an antibody produced by molecular modeling techniques wherein the human content of the antibody is maximised while causing little or no loss of binding affinity attributable to the variable region of, for example, a parental rat, rabbit or murine antibody.

The methods described below are applicable to the humanisation of antibodies.

There are several factors to consider in deciding which human antibody sequence to use during the humanisation. The humanisation of light and heavy chains are considered independently of one another, but the reasoning is basically similar for each.

This selection process is based on the following rationale: A given antibody's antigen specificity and affinity is primarily determined by the amino acid sequence of the variable region CDRs. Variable domain framework residues have little or no direct contribution. The primary function of the framework regions is two hold the CDRs in their proper spatial orientation to recognize antigen. Thus the substitution of animal, for example, rodent CDRs into a human variable domain framework is most likely to result in retention of their correct spatial orientation if the human variable domain framework is highly homologous to the animal variable domain from which they originated. A human variable domain should preferably be chosen therefore that is highly homologous to the animal variable domain(s). A suitable human antibody variable domain sequence can be selected as follow.

Step 1. Using a computer program, search all available protein (and DNA) databases for those human antibody variable domain sequences that are most homologous to the animal-derived antibody variable domains. The output of a suitable program is a list of sequences most homologous to the animal-derived antibody, the percent homology to each sequence, and an alignment of each sequence to the animal-derived sequence. This is done independently for both the heavy and light chain variable domain sequences. The above analyses are more easily accomplished if only human immunoglobulin sequences are included.

Step 2. List the human antibody variable domain sequences and compare for homology. Primarily the comparison is performed on length of CDRs, except CDR3 of the heavy chain which is quite variable. Human heavy chains and Kappa and Lambda light chains are divided into subgroups; Heavy chain 3 subgroups, Kappa chain 4 subgroups, Lambda chain 6 subgroups. The CDR sizes within each subgroup are similar but vary between subgroups. It is usually possible to match an animal-derived antibody CDR to one of the human subgroups as a first approximation of homology. Antibodies bearing CDRs of similar length are then compared for amino acid sequence homology, especially within the CDRs, but also in the surrounding framework regions. The human variable domain which is most homologous is chosen as the framework for humanisation.

### The Actual Humanising Methodologies/Techniques

An antibody may be humanized by grafting the desired CDRs onto a human framework according to EP 239400. A DNA sequence encoding the desired reshaped antibody can therefore be made beginning with the human DNA whose CDRs it is wished to reshape. The animal-derived variable domain amino acid sequence containing the desired CDRs is compared to that of the chosen human antibody variable domain sequence. The residues in the human variable domain are marked that need to be changed to the corresponding residue in the animal to make the human variable region incorporate the animal-derived CDRs. There may also be residues that need substituting in, adding to or deleting from the human sequence.

Oligonucleotides are synthesized that can be used to mutagenize the human variable domain framework to contain the desired residues. Those oligonucleotides can be of any convenient size. One is normally only limited in length by the capabilities of the particular synthesizer one has available. The method of oligonucleotide-directed in vitro mutagenesis is well known.

Synthetic gene sequences, such as those encoding humanized antibodies or fragments thereof, can be commercially ordered through any of a number of service companies, including DNA 2.0 (Menlo Park, Calif.), Geneart (Regensburg, Germany), CODA Genomics (Irvine, Calif.), and GenScript, Corporation (Piscataway, N.J.).

Alternatively, humanisation may be achieved using the recombinant polymerase chain reaction (PCR) methodology of WO 92/07075. Using this methodology, a CDR may be spliced between the framework regions of a human antibody. In general, the technique of WO 92/07075 can be performed, using a template comprising two human framework regions, AB and CD, and between them, the CDR which is to be replaced by a donor CDR. Primers A and B are used to amplify the framework region AB, and primers C and D used to amplify the framework region CD. However, the primers B and C each also contain, at their 5' ends, an additional sequence corresponding to all or at least part of the donor CDR sequence. Primers B and C overlap by a length sufficient to permit annealing of their 5' ends to each other under conditions which allow a PCR to be performed. Thus, the amplified regions AB and CD may undergo gene splicing by overlap extension to produce the humanized product in a single reaction.

Following the mutagenesis reactions to reshape the antibody, the mutagenised DNAs can be linked to an appropriate DNA encoding a light or heavy chain constant region, cloned into an expression vector, and transfected into host cells, preferably mammalian cells. These steps can be carried out in routine fashion. A reshaped antibody may therefore be prepared by a process comprising:
(a) preparing a first replicable expression vector including a suitable promoter operably linked to a DNA sequence which encodes at least a variable domain of an Ig heavy or light chain, the variable domain comprising framework regions from a human antibody and the CDRs required for the humanized antibody;
(b) preparing a second replicable expression vector including a suitable promoter operably linked to a DNA sequence which encodes at least the variable domain of a complementary Ig light or heavy chain respectively;
(c) transforming a cell line with the first or both prepared vectors; and
(d) culturing said transformed cell line to produce said altered antibody.

Preferably the DNA sequence in step (a) encodes both the variable domain and each constant domain of the human antibody chain. The humanized antibody can be prepared using any suitable recombinant expression system. The cell line which is transformed to produce the altered antibody may be a Chinese Hamster Ovary (CHO) cell line or an immortalised mammalian cell line, which is advantageously of lymphoid origin, such as a myeloma, hybridoma, trioma or quadroma cell line. The cell line may also comprise a normal lymphoid cell, such as a B-cell, which has been immortalised by transformation with a virus, such as the Epstein-Barr virus. Most preferably, the immortalised cell line is a myeloma cell line or a derivative thereof.

The CHO cells used for expression of the antibodies may be dihydrofolate reductase (dhfr) deficient and so dependent on thymidine and hypoxanthine for growth. The parental dhfr⁻ CHO cell line is transfected with the DNA encoding the antibody and dhfr gene which enables selection of CHO cell transformants of dhfr positive phenotype. Selection is carried out by culturing the colonies on media devoid of thymidine and hypoxanthine, the absence of which prevents untransformed cells from growing and transformed cells from resalvaging the folate pathway and thus bypassing the selection system. These transformants usually express low levels of the DNA of interest by virtue of co-integration of transfected DNA of interest and DNA encoding dhfr. The expression levels of the DNA encoding the antibody may be increased by amplification using methotrexate (MTX). This drug is a direct inhibitor of the enzyme dhfr and allows isolation of resistant colonies which amplify their dhfr gene copy number sufficiently to survive under these conditions. Since the DNA sequences encoding dhfr and the antibody are closely linked in the original transformants, there is usually concomitant amplification, and therefore increased expression of the desired antibody.

Another preferred expression system for use with CHO or myeloma cells is the glutamine synthetase (GS) amplification system described in WO 87/04462. This system involves the transfection of a cell with DNA encoding the enzyme GS and with DNA encoding the desired antibody. Cells are then selected which grow in glutamine free medium and can thus be assumed to have integrated the DNA encoding GS. These selected clones are then subjected to inhibition of the enzyme GS using methionine sulphoximine (Msx). The cells, in order to survive, will amplify the DNA encoding GS with concomitant amplification of the DNA encoding the antibody.

Although the cell line used to produce the humanized antibody is preferably a mammalian cell line, any other suitable cell line, such as a bacterial cell line or a yeast cell line, may alternatively be used. In particular, it is envisaged that *E. coli*-derived bacterial strains could be used. The antibody obtained is checked for functionality. If functionality is lost, it is necessary to return to step (2) and alter the framework of the antibody.

Once expressed, the whole antibodies, their dimers, individual light and heavy chains, or other immunoglobulin forms can be recovered and purified according to standard procedures of the art, including ammonium sulfate precipitation, affinity columns, column chromatography and gel electrophoresis (See, generally, Scopes, R., Protein Purification, Springer-Verlag, N.Y. (1982)). Substantially pure immunoglobulins of at least about 90 to 95% homogeneity are preferred, and 98 to 99% or more homogeneity most preferred, for pharmaceutical uses. Once purified, partially or to homogeneity as desired, a humanized antibody may then be used therapeutically or in developing and performing assay procedures and immunofluorescent stainings (See, generally, Lefkovits and Pernis (editors), Immunological Methods, Vols. I and II, Academic Press, (1979 and 1981)).

Studies carried out by Greenwood et al. (1993) have demonstrated that recognition of the Fc region of an antibody by human effector cells can be optimised by engineering the constant region of the immunoglobulin molecule. This could be achieved by fusing the variable region genes of the antibody, with the desired specificity, to human constant region genes encoding immunoglobulin isotypes that have demonstrated effective antigen dependent cellular cytotoxicity (ADCC) in human subjects, for example the IgG1 and IgG3 isotypes (Greenwood and Clark, Protein Engineering of Antibody Molecules for Prophylactic and Therapeutic Applications in Man. Mike Clark (editor), Academic Titles, Section II, p.85-113, (1993)).

Antibodies with fully human variable regions can also be prepared by administering the antigen to a transgenic animal which has been modified to produce such antibodies in response to antigenic challenge, but whose endogenous loci have been disabled. Various subsequent manipulations can be performed to obtain either antibodies *per se* or analogs thereof (see, for example, US Patent No. 6,075,181).

### Preparation of Genes Encoding Antibodies or Fragments Thereof

Genes encoding antibodies, both light and heavy chain genes or portions thereof, e.g., single chain Fv regions, may be cloned from a hybridoma cell line. They may all be cloned using the same general strategy such as RACE using a coimmercially available kit, for example as produced by Clontech. Typically, for example, poly(A)⁺mRNA extracted from the hybridoma cells is reverse transcribed using random hexamers as primers. For Fv regions, the V_{H} and V_{L} domains are amplified separately by two polymerase chain reactions (PCR). Heavy chain sequences may be amplified using 5' end primers which are designed according to the amino-terminal protein sequences of the heavy chains respectively and 3' end primers according to consensus immunoglobulin constant region sequences (Kabat et al., Sequences of Proteins of Immunological Interest. 5th edition. U.S. Department of Health and Human Services, Public Health Service, National Institutes of Health, Bethesda, Md. (1991)). Light chain Fv regions are amplified using 5' end primers designed according to the amino-terminal protein sequences of light chains and in combination with the primer C-kappa. One of skill in the art would recognize that many suitable primers may be employed to obtain Fv regions.

The PCR products are subcloned into a suitable cloning vector. Clones containing the correct size insert by DNA restriction are identified. The nucleotide sequence of the heavy or light chain coding regions may then be determined from double stranded plasmid DNA using sequencing primers adjacent to the cloning site. Commercially available kits (e.g., the Sequenase™ kit, United States Biochemical Corp., Cleveland, Ohio, USA) may be used to facilitate sequencing the DNA. DNA encoding the Fv regions may be prepared by any suitable method, including, for example, amplification techniques such as PCR and LCR.

Chemical synthesis produces a single stranded oligonucleotide. This may be converted into double stranded DNA by hybridization with a complementary sequence, or by polymerization with a DNA polymerase using the single strand as a template. While it is possible to chemically synthesize an entire single chain Fv region, it is preferable to synthesize a number of shorter sequences (about 100 to 150 bases) that are later ligated together.

Alternatively, sub-sequences may be cloned and the appropriate subsequences cleaved using appropriate restriction enzymes. The fragments may then be ligated to produce the desired DNA sequence.

Once the Fv variable light and heavy chain DNA is obtained, the sequences may be ligated together, either directly or through a DNA sequence encoding a peptide linker, using techniques well known to those of skill in the art. In one embodiment, heavy and light chain regions are connected by a flexible peptide linker (e.g., (Gly₄Ser)₃) which starts at the carboxyl end of the heavy chain Fv domain and ends at the amino terminus of the light chain Fv domain. The entire sequence encodes the Fv domain in the form of a single-chain antigen binding protein.

### Therapeutic Agents

Compounds defined herein can be used to deliver a therapeutic agent. Examples of therapeutic agents include, but are not limited to, an antigen, a cytotoxic agent, a drug and/or pharmacological agent.

In some examples, the therapeutic agent may be a polypeptide fused to the compound. Fusion polypeptides comprising the compound may be prepared by methods known to one of skill in the art. For example, a gene encoding an Fv region is fused to a gene encoding a therapeutic agent. Optionally, the Fv gene is linked to a segment encoding a peptide connector. The peptide connector may be present simply to provide space between the compound and the therapeutic agent or to facilitate mobility between these regions to enable them to each attain their optimum conformation. The DNA sequence comprising the connector may also provide sequences (such as primer sites or restriction sites) to facilitate cloning or may preserve the reading frame between the sequence encoding the binding moiety and the sequence encoding the therapeutic agent. The design of such connector peptides is well known to those of skill in the art.

Generally producing fusion polypeptides involves, e.g., separately preparing the Fv light and heavy chains and DNA encoding any other protein to which they are fused and recombining the DNA sequences in a plasmid or other vector to form a construct encoding the particular desired fusion polypeptide. However, a simpler approach involves inserting the DNA encoding the particular Fv region into a construct already encoding the desired fused polypeptide. The DNA sequence encoding the Fv region is inserted into the construct using techniques well known to those of skill in the art.

Compounds e.g., recombinant single chain antibodies, may be fused to, or otherwise bound to the therapeutic agent by any method known and available to those in the art. The two components may be chemically bonded together by any of a variety of well-known chemical procedures. For example, the linkage may be by way of heterobifunctional cross-linkers, e.g., SPDP, carbodiimide or glutaraldehyde. Production of various immunotoxins, as well as chemical conjugation methods, are well-known within the art (see, for example, "Monoclonal Antibody-Toxin Conjugates: Aiming the Magic Bullet," Thorpe et al., Monoclonal Antibodies in Clinical Medicine, Academic Press, p. 168-190 (1982); Waldmann, 1991; Vitetta et al., 1987; Pastan et al., 1986; and Thorpe et al., 1987).

Examples of drugs and/or pharmacological agents include, but are not limited to, agents that promote DC activation (e.g. TLR ligands), agents that suppress DC activation or function (e.g. specific inhibitors or promotors of DC signalling molecules such as kinases and phosphatases), and agents that modulate DC death (e.g. promotors or suppressors of apoptosis). Such drugs and/or pharmacological agents are well known to those skilled in the art.

The skilled person will appreciate that there are a number of bacterial or plant polypeptide toxins that are suitable for use as cytotoxic agents. These polypeptides include, but are not limited to, polypeptides such as native or modified Pseudomonas exotoxin (PE), diphtheria toxin (DT), ricin, abrin, gelonin, momordin II, bacterial RIPs such as shiga and shiga-like toxin a-chains, luffin, atrichosanthin, momordin I, Mirabilis anti-viral protein, pokeweed antiviral protein, byodin 2 (US 5,597,569), gaporin, as well as genetically engineered variants thereof. Native PE and DT are highly toxic compounds that typically bring about death through liver toxicity. Preferably, PE and DT are modified into a form that removes the native targeting component of the toxin, e.g., domain Ia of PE and the B chain of DT.

Other suitable cytotoxic agents include, but are not limited to, agents such as bacterial or plant toxins, drugs, e.g., cyclophosphamide (CTX; cytoxan), chlorambucil (CHL; leukeran), cisplatin (CisP; CDDP; platinol), busulfan (myleran), melphalan, carmustine (BCNU), streptozotocin, triethylenemelamine (TEM), mitomycin C, and other alkylating agents; methotrexate (MTX), etoposide (VP-16; vepesid), 6-mercaptopurine (6MP), 6-thioguanine (6TG), cytarabine (Ara-C), 5-fluorouracil (5FU), dacarbazine (DTIC), 2-chlorodeoxyadenosine (2-CdA), and other antimetabolites; antibiotics including actinomycin D, doxorubicin (DXR; adriamycin), daunorubicin (daunomycin), bleomycin, mithramycin as well as other antibiotics; alkaloids such as vincristin (VCR) and vinblastine; as well as other anticancer agents including the cytostatic agents glucocorticoids such as dexamethasone (DEX; decadron) and corticosteroids such as prednisone and nucleotide enzyme inhibitors such as hydroxyurea.

Those skilled in the art will realize that there are numerous other radioisotopes and chemocytotoxic agents that can be coupled to compounds which bind 5B6 by well known techniques, and delivered to specifically destroy dendritic cells (see, e.g., U.S. 4,542,225). Examples of photo-activated toxins include dihydropyridine-and omega-conotoxin. Examples of cytotoxic reagents that can be used include ¹²⁵I, ¹³¹I, ¹¹¹In, ¹²³I, ⁹⁹mTc, and ³²P. The antibody can be labeled with such reagents using techniques known in the art. For example, see Wenzel and Meares, Radioimmunoimaging and Radioimmunotherapy, Elsevier, N.Y. (1983) for techniques relating to the radiolabeling of antibodies (see also, Colcher et al., 1986; "Order, Analysis, Results and Future Prospective of the Therapeutic Use of Radiolabeled Antibody in Cancer Therapy", Monoclonal Antibodies for Cancer Detection and Therapy, Baldwin et al. (editors), Academic Press, p. 303-16, (1985)).

In one example, the linker-chelator tiuexutan is conjugated to the compound by a stable thiourea covalent bond to provide a high-affinity chelation site for Indium-111 or Yttrium-90.

### Antigens

The term "antigen" is further intended to encompass peptide or protein analogs of known or wild-type antigens such as those described above. The analogs may be more soluble or more stable than wild type antigen, and may also contain mutations or modifications rendering the antigen more immunologically active. Also useful are peptides or proteins which have amino acid sequences homologous with a desired antigen's amino acid sequence, where the homologous antigen induces an immune response to the respective tumor or organism.

A "cancer antigen," as used herein is a molecule or compound (e.g., a protein, peptide, polypeptide, lipid, glycolipid, carbohydrate and/or DNA) associated with a tumor or cancer cell and which is capable of provoking an immune response when expressed on the surface of an antigen presenting cell in the context of an MHC molecule. Cancer antigens include self antigens, as well as other antigens that may not be specifically associated with a cancer, but nonetheless induce and/or enhance an immune response to and/or reduce the growth of a tumor or cancer cell when administered to an animal.

An "antigen from a pathogenic and/or infectious organism" as used herein, is an antigen of any organism and includes, but is not limited to, infectious virus, infectious bacteria, infectious parasites including protozoa (such as *Plasmodium sp.*) and worms and infectious fungi. Typically, the antigen is a protein or antigenic fragment thereof from the organism, or a synthetic compound which is identical to or similar to naturally-occurring antigen which induces an immune response specific for the corresponding organism. Compounds or antigens that are similar to a naturally-occurring organism antigens are well known to those of ordinary skill in the art. A non-limiting example of a compound that is similar to a naturally-occurring organism antigen is a peptide mimic of a polysaccharide antigen.

Specific examples of cancer antigens include, e.g., mutated antigens such as the protein products of the Ras p21 protooncogenes, tumor suppressor p53 and HER-2/neu and BCR-abl oncogenes, as well as CDK4, MUM1, Caspase 8, and Beta catenin; overexpressed antigens such as galectin 4, galectin 9, carbonic anhydrase, Aldolase A, PRAME, Her2/neu, ErbB-2 and KSA, oncofetal antigens such as alpha fetoprotein (AFP), human chorionic gonadotropin (hCG); self antigens such as carcinoembryonic antigen (CEA) and melanocyte differentiation antigens such as Mart 1/Melan A, gp100, gp75, Tyrosinase, TRP1 and TRP2; prostate associated antigens such as PSA, PAP, PSMA, PSM-P1 and PSM-P2; reactivated embryonic gene products such as MAGE 1, MAGE 3, MAGE 4, GAGE 1, GAGE 2, BAGE, RAGE, and other cancer testis antigens such as NY-ESO1, SSX2 and SCP1; mucins such as Muc-1 and Muc-2; gangliosides such as GM2, GD2 and GD3, neutral glycolipids and glycoproteins such as Lewis (y) and globo-H; and glycoproteins such as Tn, Thompson-Freidenreich antigen (TF) and sTn.

Cancer antigens and their respective tumor cell targets include, e.g., cytokeratins, particularly cytokeratin 8, 18 and 19, as antigens for carcinoma. Epithelial membrane antigen (EMA), human embryonic antigen (HEA-125), human milk fat globules, MBr1, MBr8, Ber-EP4, 17-1A, C26 and T16 are also known carcinoma antigens. Desmin and muscle-specific actin are antigens of myogenic sarcomas. Placental alkaline phosphatase, beta-human chorionic gonadotropin, and alpha-fetoprotein are antigens of trophoblastic and germ cell tumors. Prostate specific antigen is an antigen of prostatic carcinomas, carcinoembryonic antigen of colon adenocarcinomas. HMB-45 is an antigen of melanomas. In cervical cancer, useful antigens could be encoded by human papilloma virus. Chromagranin-A and synaptophysin are antigens of neuroendocrine and neuroectodermal tumors. Of particular interest are aggressive tumors that form solid tumor masses having necrotic areas.

Antigens derived from pathogens known to predispose to certain cancers may also be used. Pathogens of particular interest for use in the cancer vaccines provided herein include the hepatitis B virus (hepatocellular carcinoma), hepatitis C virus (heptomas), Epstein Barr virus (EBV) (Burkitt lymphoma, nasopharynx cancer, PTLD in immunosuppressed individuals), HTLVL (adult T cell leukemia), oncogenic human papilloma viruses types 16, 18, 33, 45 (adult cervical cancer), and the bacterium *Helicobacter pylori* (B cell gastric lymphoma). Other medically relevant microorganisms that may serve as antigens in mammals and more particularly humans are described extensively in the literature, e.g., C. G. A Thomas, Medical Microbiology, Bailliere Tindall, (1983).

Exemplary viral pathogens include, but are not limited to, infectious virus that infect mammals, and more particularly humans. Examples of infectious virus include, but are not limited to: *Retroviridae* (e.g., human immunodeficiency viruses, such as HIV-1 (also referred to as HTLV-III, LAV or HTLV-III/LAV, or HIV-III; and other isolates, such as HIV-LP; *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); *Flaviridae* (e.g. dengue viruses, encephalitis viruses, yellow fever viruses); *Coronoviridae* (e.g. coronaviruses such as the SARS coronavirus); *Rhabdoviradae* (e.g. vesicular stomatitis viruses, rabies viruses); Filoviridae (e.g. ebola viruses); *Paramyxoviridae* (e.g. parainfluenza viruses, mumps virus, measles virus, respiratory syncytial virus); *Orthomyxoviridae* (e.g. influenza viruses); *Bungaviridae* (e.g. Hantaan viruses, bunga viruses, phleboviruses and Nairo viruses); *Arena viridae* (hemorrhagic fever viruses); *Reoviridae* (e.g. reoviruses, orbiviurses and rotaviruses); *Birnaviridae*; *Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* herpes simplex virus (HSV) 1 and 2, varicella zoster virus, cytomegalovirus (CMV), herpes virus; *Poxyiridae* (variola viruses, vaccinia viruses, pox viruses); and *Iridoviridae* (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Also, gram negative and gram positive bacteria may be targeted by the subject compositions and methods in vertebrate animals. Such gram positive bacteria include, but are not limited to *Pasteurella sp., Staphylococci sp.,* and *Streptococcus sp.* Gram negative bacteria include, but are not limited to, *Escherichia coli, Pseudomonas sp.,* and *Salmonella sp.* Specific examples of infectious bacteria include but are not limited to: *Helicobacter pyloris, Borella burgdorferi, Legionella pneumophilia, Mycobacteria sp.* (e.g. *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter sp., Enterococcus sp., Haemophilus infuenzae, Bacillus antracis, Corynebacterium diphtheriae, Corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia,* and *Actinomyces israelli.*

Polypeptides of bacterial pathogens which may find use as sources of antigen in the subject compositions include but are not limited to an iron-regulated outer membrane protein, ("IROMP"), an outer membrane protein ("OMP"), and an A-protein of *Aeromonis salmonicida* which causes furunculosis, p57 protein of *Renibacterium salmoninarum* which causes bacterial kidney disease ("BKD"), major surface associated antigen ("msa"), a surface expressed cytotoxin ("mpr"), a surface expressed hemolysin ("ish"), and a flagellar antigen of *Yersiniosis*; an extracellular protein ("ECP"), an iron-regulated outer membrane protein ("IROMP"), and a structural protein of *Pasteurellosis;* an OMP and a flagellar protein of *Vibrosis anguillarum* and *V. ordalii*; a flagellar protein, an OMP protein, aroA, and purA of *Edwardsiellosis ictaluri* and *E. tarda*; and surface antigen of *Ichthyophthirius*; and a structural and regulatory protein of *Cytophaga columnari*; and a structural and regulatory protein of *Rickettsia.* Such antigens can be isolated or prepared recombinantly or by any other means known in the art.

Examples of pathogens further include, but are not limited to, infectious fungi and parasites that infect mammals, and more particularly humans. Examples of infectious fungi include, but are not limited to: *Cryptococcus neoformans, Histoplasma capsulatum, Coccidioides immitis, Blastomyces dermatitidis, Chlamydia trachomatis,* and *Candida albicans.*

Examples of parasites include intracellular parasites and obligate intracellular parasites. Examples of parasites include but are not limited to *Plasmodium falciparum, Plasmodium ovale, Plasmodium malariae, Plasmdodium vivax, Plasmodium knowlesi, Babesia microti, Babesia divergens, Tiypanosoma cruzi, Toxoplasma gondii, Trichinella spiralis, Leishmania major, Leishmania donovani, Leishmania braziliensis, Leishmania tropica, Tiypanosoma gambiense, Tiypanosoma rhodesiense, Wuchereria bancrofti, Brugia malayi, Brugia timori, Ascaris lumbricoides, Onchocerca volvulus* and *Schistosoma mansoni.*

Other medically relevant microorganisms that serve as antigens in mammals and more particularly humans are described extensively in the literature, e.g., see C. G. A Thomas, Medical Microbiology, Bailliere Tindall, (1983). In addition to the treatment of infectious human diseases and human pathogens, the compositions are useful for treating infections of nonhuman mammals. Exemplary non-human pathogens include, but are not limited to, mouse mammary tumor virus ("MMTV"), Rous sarcoma virus ("RSV"), avian leukemia virus ("ALV"), avian myeloblastosis virus ("AMV"), murine leukemia virus ("MLV"), feline leukemia virus ("FeLV"), murine sarcoma virus ("MSV"), gibbon ape leukemia virus ("GALV"), spleen necrosis virus ("SNV"), reticuloendotheliosis virus ("RV"), simian sarcoma virus ("SSV"), Mason-Pfizer monkey virus ("MPMV"), simian retrovirus type 1 ("SRV-1 "), lentiviruses such as HIV-1, HIV-2, SIV, Visna virus, feline immunodeficiency virus ("FIV"), and equine infectious anemia virus ("EIAV"), T-cell leukemia viruses such as HTLV-1, HTLV-II, simian T-cell leukemia virus ("STLV"), and bovine leukemia virus ("BLV"), and foamy viruses such as human foamy virus ("HFV"), simian foamy virus ("SFV") and bovine foamy virus ("BFV").

### Detectable Labels

Compounds may be employed in a range of detection systems. For example, the compound may be used in methods for imaging an internal region of a subject and/or diagnosing the presence or absence of a disease in a subject.

It will be apparent to those skilled in the art that the diagnostic, prognostic and/or monitoring methods of the present invention involve a degree of quantification to determine levels of 5B6, 5B6 expressing cells, ligand and/or ligand expressing cells present in patient samples. Such quantification is readily provided by the inclusion of appropriate control samples.

Preferably, internal controls are included in the methods of the present invention. A preferred internal control is one or more samples taken from one or more healthy individuals.

Compounds useful for the present invention when used diagnostically may be linked to a diagnostic reagent such as a detectable label to allow easy detection of binding events *in vitro* or *in vivo.* Suitable labels include radioisotopes, or nonradioactive labels such as biotin, enzymes, chemiluminescent molecules, fluorophores, dye markers or other imaging reagents for detection and/or vocalisation of target molecules. Alternatively, a second labelled antibody or avidin (for example) which binds the compound can be used for detection.

In the case of an enzyme immunoassay, an enzyme can be conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, β-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above.

In another example, fluorescent compounds, such as but not limited to fluorecein and rhodamine amongst others, may be chemically coupled to, for examples, antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope.

By further way of non-limiting example, the compounds coupled to imaging agents can be used in the detection of 5B6 or ligand expression in histochemical tissue sections. The compound may be covalently or non-covalently coupled to a suitable supermagnetic, paramagnetic, electron dense, echogenic, radioactive, or nonradioactive labels such as biotin or avidin.

### Labelled Cell Detection and Isolation

Cells with a disrupted cell membrane, cells infected with a pathogen, dying cells or dead cells can be detected in a sample by a variety of techniques well known in the art, including cell sorting, especially fluorescence-activated cell sorting (FACS), by using an affinity reagent bound to a substrate (e.g., a plastic surface, as in panning), or by using an affinity reagent bound to a solid phase particle which can be isolated on the basis of the properties of the beads (e.g., colored latex beads or magnetic particles). Naturally, the procedure used to detect the cells will depend upon how the cells have been labelled.

In one example, any detectable substance which has the appropriate characteristics for the cell sorter may be used (e.g., in the case of a fluorescent dye, a dye which can be excited by the sorter's light source, and an emission spectra which can be detected by the cell sorter's detectors). In flow cytometry, a beam of laser light is projected through a liquid stream that contains cells, or other particles, which when struck by the focussed light give out signals which are picked up by detectors. These signals are then converted for computer storage and data analysis, and can provide information about various cellular properties. Cells labelled with a suitable dye are excited by the laser beam, and emit light at characteristic wavelengths. This emitted light is picked up by detectors, and these analogue signals are converted to digital signals, allowing for their storage, analysis and display.

Many larger flow cytometers are also "cell sorters", such as fluorescence-activated cell sorters (FACS), and are instruments which have the ability to selectively deposit cells from particular populations into tubes, or other collection vessels. In a particularly preferred embodiment, the cells are isolated using FACS. This procedure is well known in the art and described by, for example, Melamed et al., Flow Cytometry and Sorting, Wiley-Liss, Inc., (1990); Shapiro, Practical Flow Cytometry, 4th Edition, Wiley-Liss, Inc., (2003); and Robinson et al., Handbook of Flow Cytometry Methods, Wiley-Liss, Inc. (1993).

In order to sort cells, the instruments electronics interprets the signals collected for each cell as it is interrogated by the laser beam and compares the signal with sorting criteria set on the computer. If the cell meets the required criteria, an electrical charge is applied to the liquid stream which is being accurately broken into droplets containing the cells. This charge is applied to the stream at the precise moment the cell of interest is about to break off from the stream, then removed when the charged droplet has broken from the stream. As the droplets fall, they pass between two metal plates, which are strongly positively or negatively charged. Charged droplets get drawn towards the metal plate of the opposite polarity, and deposited in the collection vessel, or onto a microscope slide, for further examination.

The cells can automatically be deposited in collection vessels as single cells or as a plurality of cells, e.g. using a laser, e.g. an argon laser (488 nm) and for example with a Flow Cytometer fitted with an Autoclone unit (Coulter EPICS Altra, Beckman-Coulter, Miami, Fla., USA). Other examples of suitable FACS machines useful for the methods of the invention include, but are not limited to, MoFlo™ High-speed cell sorter (Dako-Cytomation ltd), FACS Aria™ (Becton Dickinson), FACS Diva (Becton Dickinson), ALTRA™ Hyper sort (Beckman Coulter) and CyFlow™ sorting system (Partec GmbH).

For the detection of cells with a disrupted cell membrane, cells infected with a pathogen, dying cells or dead cells from a sample using solid-phase particles, any particle with the desired properties may be utilized. For example, large particles (e.g., greater than about 90-100 µm in diameter) may be used to facilitate sedimentation. Preferably, the particles are "magnetic particles" (i.e., particles which can be collected using a magnetic field). Labelled cells are retained in the column (held by the magnetic field), whilst unlabelled cells pass straight through and are eluted at the other end. Magnetic particles are now commonly available from a variety of manufacturers including Dynal Biotech (Oslo, Norway) and Milteni Biotech GmbH (Germany). An example of magnetic cell sorting (MACS) is provided by Al-Mufti et al. (1999).

Laser-capture microdissection can also be used to selectively detect labelled cells on a slide using methods of the invention. Methods of using laser-capture microdissection are known in the art (see, for example, U.S. 20030227611 and Bauer et al., 2002).

### Identification of Compounds that Bind 5B6 or its Ligand

Methods of screening test compounds are described which can identify a compound that binds to 5B6, or its ligand.

Inhibitors of 5B6, or its ligand, activity are screened by resort to assays and techniques useful in identifying drugs capable of binding to the 5B6 polypeptide, or its ligand, and thereby inhibiting its biological activity. Such assays include the use of mammalian cell lines (for example, CHO cells or 293T cells) for phage display system for expressing the 5B6 polypeptide, or its ligand, and using a culture of transfected mammalian or *E. coli* or other microorganism to produce the proteins for binding studies of potential binding compounds.

As another example, a method for identifying compounds which specifically bind to a 5B6 polypeptide, or its ligand, can include simply the steps of contacting a selected cell expressing 5B6, or its ligand, with a test compound to permit binding of the test compound to 5B6, or its ligand, and determining the amount of test compound, if any, which is bound to the 5B6 or its ligand. Such a method involves the incubation of the test compound and the 5B6 polypeptide, or its ligand, immobilized on a solid support. Typically, the surface containing the immobilized compound is permitted to come into contact with a solution containing the protein and binding is measured using an appropriate detection system. Suitable detection systems are known in the art, some of which are described herein.

Computer modeling and searching technologies permit identification of compounds that can bind 5B6 or its ligand. The three dimensional geometric structure of 5B6, or its ligand, or the active site thereof can be determined. This can be done by known methods, including X-ray crystallography, which can determine a complete molecular structure.

Methods of computer based numerical modeling can be used to complete the structure (e. g., in examples wherein an incomplete or insufficiently accurate structure is determined) or to improve its accuracy. Any method recognized in the art may be used, including, but not limited to, parameterized models specific to particular biopolymers such as proteins or nucleic acids, molecular dynamics models based on computing molecular motions, statistical mechanics models based on thermal ensembles, or combined models.

The three-dimensional structure of 5B6, or its ligand, can be used to identify antagonists or agonists through the use of computer modeling using a docking program such as GRAM, DOCK, or AUTODOCK (Dunbrack et al., 1997). Computer programs can also be employed to estimate the attraction, repulsion, and steric hindrance of a candidate compound to the polypeptide. Generally the tighter the fit (e.g., the lower the steric hindrance, and/or the greater the attractive force) the more potent the potential agonist or antagonist will be since these properties are consistent with a tighter binding constant. Furthermore, the more specificity in the design of a potential agonist or antagonist the more likely that it will not interfere with other proteins.

Initially a potential compound could be obtained, for example, by screening a random peptide library produced by a recombinant bacteriophage or a chemical library. A compound selected in this manner could be then be systematically modified by computer modeling programs until one or more promising potential compounds are identified.

Such computer modeling allows the selection of a finite number of rational chemical modifications, as opposed to the countless number of essentially random chemical modifications that could be made, and of which any one might lead to a useful agonist or antagonist. Each chemical modification requires additional chemical steps, which while being reasonable for the synthesis of a finite number of compounds, quickly becomes overwhelming if all possible modifications needed to be synthesized. Thus through the use of the three-dimensional structure and computer modeling, a large number of these compounds can be rapidly screened on the computer monitor screen, and a few likely candidates can be determined without the laborious synthesis of untold numbers of compounds.

For most types of models, standard molecular force fields, representing the forces between constituent atoms and groups, are necessary, and can be selected from force fields known in physical chemistry. Exemplary forcefields that are known in the art and can be used in such methods include, but are not limited to, the Constant Valence Force Field (CVFF), the AMBER force field and the CHARM force field. The incomplete or less accurate experimental structures can serve as constraints on the complete and more accurate structures computed by these modeling methods.

Further examples of molecular modeling systems are the CHARMm and QUANTA programs (Polygen Corporation, Waltham, MA). CHARMm performs the energy minimization and molecular dynamics functions. QUANTA performs the construction, graphic modelling and analysis of molecular structure. QUANTA allows interactive construction, modification, visualization, and analysis of the behaviour of molecules with each other.

### Microorganism Deposit Details

Hybridoma 20/05-3A4-26-16-Clone 5 and Hybridoma 23/05-4C6-29-3-Clone 5 are hybridomas secreting monoclonal Ab to human C-type lectin clone 5B6.

Hybridoma 24/04-10B4-24-8-FACS 9-5 and Hybridoma 42/04-42D2-66-4-1-Clone 4 are hybridomas secreting monoclonal Ab to mouse C-type lectin clone 5B6.

Antibodies 24/04-10B4, 42/04-42D2, 20/05-3A4 and 23/05-4C6 are produced by hydridoma cell lines deposited with the European Collection of Cell Cultures (ECACC) 24/04-10B4-24-8, 42/04-42D2-66-4-1, 20/05-3A4-26-16, 23/05-4C6-29-3 on 11 December 2007 under Deposit Reference Numbers 07121101, 07121102, 07121103, and 07121104 respectively.

Higher antibody secreting subclones of the above hybridomas (24/04-10B4-24-8-FACS 9-5, 42/04-42D2-66-4-1-Clone 4, 20/05-3A4-26-16-Clone 5, 23/05-4C6-29-3-Clone 5) were deposited with the ECACC on 29 April 2008, and designated the following numbers;
- Hybridoma 24/04-10B4-24-8-FACS 9-5 - Accession no. 08042901 • Hybridoma 42/04-42D2-66-4-1 CLONE 4 - Accession no. 08042902 • Hybridoma 20/05-3A4-26-16-CLONE 5 - Accession no. 08042903, and
- Hybridoma 23/05-4C6-29-3- CLONE 5 - Accession no. 08042904.

These deposits were made under the provisions of the Budapest Treaty on the International Recognition of the Deposit of Microorganisms for the Purpose of Patent Procedure and the Regulations thereunder. This assures maintenance of viable cultures for 30 years from the date of deposit. The organisms will be made available by the ECACC under the terms of the Budapest Treaty which assures permanent and unrestricted availability of the progeny of the culture to the public upon issuance of the pertinent patent.

The assignee of the present application has agreed that if the culture deposit should die or be lost or destroyed when cultivated under suitable conditions, it will be promptly replaced on notification with a viable specimen of the same culture. Availability of a deposited strain is not to be construed as a license to practice the invention in contravention of the rights granted under the authority of any government in accordance with its patent laws.

### Diseases Associated with Cells with a Disrupted Cell Membrane, Cells Infected with a Pathogen, Dying Cells or Dead Cells

The diseases associated with cells with a disrupted cell membrane, cells infected with a pathogen, dying cells or dead cells include the following:
1) Diseases in which apoptosis is induced in response to a signal generated by a cell of an immune system responsible for biophylaxis, for example, graft versus host disease (GVHD) and autoimmune diseases (systemic lupus erythematosus (SLE), rheumatoid arthritis (RA), scleroderma, Sjogren's syndrome, multiple sclerosis, insulin dependent diabetes mellitus, ulcerative colitis).
2) Diseases in which cell death is induced by viral infection or apoptosis is induced by reaction of a cell of an immune system with a cell infected by a virus or parasitic infection, for example, virus associated hemophagocytic syndrome (VAHS) and other viral infections (HCV, HIV, influenza virus).
3) Diseases in which cell death is induced by an abnormal apoptosis signal, for example, neurodegenerative diseases (Alzheimer's disease, Parkinson's disease).
4) Leukemia, for example, acute lymphatic leukemia.
5) Diseases in which apoptosis is artificially induced by, for example, radiation exposure or medication (anticancer drug etc.)
6) Systemic inflammatory reaction syndrome (SIRS), diseases in which organ disorder occurs because the immune system is nonspecifically activated in response to invasion to a living body and thus control of cytokine production becomes impossible (HPS, severe pancreatitis).
7) Diseases where there is a lack of cell death such as cancer.
8) Injury, particularly post-injury recovery.

Cell death progressing in a living body can be determined using the present invention, and hence progress of these diseases can be monitored. In particular, the invention is useful for GVHD, human immunodeficiency virus (HIV), hemophagocytic syndrome (HPS), especially virus associated hemophagocytic syndrome (VAHS), acute lymphatic leukemia, influenzal encephalitis, encephalopathy, and malaria.

### Polypeptides

The terms "polypeptide" and "protein" are generally used interchangeably and refer to a single polypeptide chain which may or may not be modified by addition of non-amino acid groups. It would be understood that such polypeptide chains may associate with other polypeptides or proteins or other molecules such as co-factors. The terms "proteins" and "polypeptides" as used herein also include variants, mutants, biologically active fragments, modifications, analogous and/or derivatives of the polypeptides described herein.

The % identity of a polypeptide is determined by GAP (Needleman and Wunsch, 1970) analysis (GCG program) with a gap creation penalty=5, and a gap extension penalty=0.3. The query sequence is at least 25 amino acids in length, and the GAP analysis aligns the two sequences over a region of at least 25 amino acids. More preferably, the query sequence is at least 50 amino acids in length, and the GAP analysis aligns the two sequences over a region of at least 50 amino acids. More preferably, the query sequence is at least 100 amino acids in length and the GAP analysis aligns the two sequences over a region of at least 100 amino acids. Even more preferably, the query sequence is at least 200 amino acids in length and the GAP analysis aligns the two sequences over a region of at least 200 amino acids. Even more preferably, the GAP analysis aligns the two sequences over their entire length.

As used herein a "biologically active fragment" is a portion of a polypeptide as described herein which maintains a defined activity of the full-length polypeptide. Biologically active fragments can be any size as long as they maintain the defined activity. Preferably, biologically active fragments are at least 100 amino acids in length.

As used herein, an "antigenic fragment" is a portion of a polypeptide as described herein which can be administered to an animal, for example a mouse or rabbit, to induce the production of antibodies that will bind the polypeptide.

As used herein, an "antigenic binding fragment" refers to a portion of an antibody as defined herein that is capable of binding the same antigen as the full length molecule.

As used herein, a "soluble fragment" refers to a portion of a polypeptide which is lacking a membrane spanning region. In a preferred embodiment, the soluble fragment does not comprise at least the about 40, at least about 50, at least about 55, or at least about 100, N-terminal residues of any one of SEQ ID NO's 1 to 8. In a further preferred embodiment, the soluble fragment comprises the C-type lectin-like domain of a polypeptide which comprises:
i) an amino acid sequence as provided in any one of SEQ ID NO's 1 to 8; or
ii) an amino acid sequence which is at least 50% identical to any one or more of SEQ ID NO's 1 to 8. In a further embodiment, the soluble fragment comprises:
   i) an amino acid sequence as provided in any one of SEQ ID NO's 40 to 47; or
   ii) an amino acid sequence which is at least 50% identical to any one or more of SEQ ID NO's 40 to 47,
wherein the soluble fragment does not comprise at least the about 40 N-terminal residues of any one of SEQ ID NO's 1 to 8.

With regard to a defined polypeptide, it will be appreciated that % identity figures higher than those provided above will encompass preferred embodiments. Thus, where applicable, in light of the minimum % identity figures, it is preferred that the polypeptide comprises an amino acid sequence which is at least 50%, more preferably at least 55%, more preferably at least 60%, more preferably at least 65%, more preferably at least 70%, more preferably at least 75%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

Amino acid sequence mutants of a polypeptide described herein can be prepared by introducing appropriate nucleotide changes into a nucleic acid defined herein, or by *in vitro* synthesis of the desired polypeptide. Such mutants include, for example, deletions, insertions or substitutions of residues within the amino acid sequence. A combination of deletion, insertion and substitution can be made to arrive at the final construct, provided that the final polypeptide product possesses the desired characteristics.

Mutant (altered) polypeptides can be prepared using any technique known in the art. For example, a polynucleotide described herein can be subjected to *in vitro* mutagenesis. Such *in vitro* mutagenesis techniques may include sub-cloning the polynucleotide into a suitable vector, transforming the vector into a "mutator" strain such as the *E. coli* XL-1 red (Stratagene) and propagating the transformed bacteria for a suitable number of generations. In another example, the polynucleotides defined herein are subjected to DNA shuffling techniques as broadly described by Harayama (1998). Products derived from mutated/altered DNA can readily be screened using techniques described herein to determine if they are able to confer the desired phenotype.

In designing amino acid sequence mutants, the location of the mutation site and the nature of the mutation will depend on characteristic(s) to be modified. The sites for mutation can be modified individually or in series, e.g., by (1) substituting first with conservative amino acid choices and then with more radical selections depending upon the results achieved, (2) deleting the target residue, or (3) inserting other residues adjacent to the located site.

Amino acid sequence deletions generally range from about 1 to 15 residues, more preferably about 1 to 10 residues and typically about 1 to 5 contiguous residues.

Substitution mutants have at least one amino acid residue in the polypeptide molecule removed and a different residue inserted in its place. The sites of greatest interest for substitutional mutagenesis include sites identified as important for function. Other sites of interest are those in which particular residues obtained from various strains or species are identical (see, for example, Figure 1), and/or those in which particular residues obtained from related proteins are identical (see, for example, Figure 2). These positions may be important for biological activity. These sites, especially those falling within a sequence of at least three other identically conserved sites, are preferably substituted in a relatively conservative manner. Such conservative substitutions are shown in Table 1.

Furthermore, if desired, unnatural amino acids or chemical amino acid analogues can be introduced as a substitution or addition into a polypeptides described herein. Such amino acids include, but are not limited to, the D-isomers of the common amino acids, 2,4-diaminobutyric acid, α-amino isobutyric acid, 4-aminobutyric acid, 2-aminobutyric acid, 6-amino hexanoic acid, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogues in general.

**Table 1 - Exemplary substitutions.**

| **Original Residue** | **Exemplary Substitutions** |
|---|---|
| Ala (A) | val; leu; ile; gly |
| Arg (R) | lys |
| Asn (N) | gln; his |
| Asp (D) | glu |
| Cys (C) | ser |
| Gln (Q) | asn; his |
| Glu (E) | asp |
| Gly (G) | pro, ala |
| His (H) | asn; gln |
| Ile (I) | leu; val; ala |
| Leu (L) | ile; val; met; ala; phe |
| Lys (K) | arg |
| Met (M) | leu; phe |
| Phe (F) | leu; val; ala |
| Pro (P) | gly |
| Ser (S) | thr |
| Thr (T) | ser |
| Trp (W) | tyr |
| Tyr (Y) | trp; phe |
| Val (V) | ile; leu; met; phe; ala |

Also included within the scope of the invention are the use of polypeptides which are differentially modified during or after synthesis, e.g., by biotinylation, benzylation, glycosylation, acetylation, phosphorylation, amidation, derivatization by known protecting/blocking groups, proteolytic cleavage, linkage to an antibody molecule or other cellular ligand, etc. These modifications may serve to increase the stability and/or bioactivity of the polypeptide.

Polypeptides described herein can be produced in a variety of ways, including production and recovery of natural polypeptides, production and recovery of recombinant polypeptides, and chemical synthesis of the polypeptides. In one example, an isolated polypeptide is produced by culturing a cell capable of expressing the polypeptide under conditions effective to produce the polypeptide, and recovering the polypeptide. A preferred cell to culture is a recombinant cell as described herein. Effective culture conditions include, but are not limited to, effective media, bioreactor, temperature, pH and oxygen conditions that permit polypeptide production. An effective medium refers to any medium in which a cell is cultured to produce a polypeptide. Such medium typically comprises an aqueous medium having assimilable carbon, nitrogen and phosphate sources, and appropriate salts, minerals, metals and other nutrients, such as vitamins. Cells can be cultured in conventional fermentation bioreactors, tissue culture flasks, shake flasks, test tubes, microtiter dishes, and petri plates. Culturing can be carried out at a temperature, pH and oxygen content appropriate for a recombinant cell. Such culturing conditions are within the expertise of one of ordinary skill in the art.

### Polynucleotides

The term "polynucleotide" is used interchangeably herein with the term "nucleic acid".

The % identity of a polynucleotide is determined by GAP (Needleman and Wunsch, 1970) analysis (GCG program) with a gap creation penalty=5, and a gap extension penalty=0.3. Unless stated otherwise, the query sequence is at least 45 nucleotides in length, and the GAP analysis aligns the two sequences over a region of at least 45 nucleotides. Preferably, the query sequence is at least 150 nucleotides in length, and the GAP analysis aligns the two sequences over a region of at least 150 nucleotides. More preferably, the query sequence is at least 250 nucleotides in length and the GAP analysis aligns the two sequences over a region of at least 250 nucleotides. Even more preferably, the GAP analysis aligns the two sequences over their entire length.

With regard to the defined polynucleotides, it will be appreciated that % identity figures higher than those provided above will encompass preferred embodiments. Thus, where applicable, in light of the minimum % identity figures, it is preferred that a polynucleotide defined herein comprises a sequence which is at least 50%, more preferably at least 55%, more preferably at least 60%, more preferably at least 65%, more preferably at least 70%, more preferably at least 75%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1 %, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

As used herein, the term "hybridizes" refers to the ability of two single stranded nucleic acid molecules being able to form at least a partially double stranded nucleic acid through hydrogen bonding.

As used herein, the phrase "stringent conditions" refers to conditions under which a polynucleotide, probe, primer and/or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (e.g., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel et al. (*supra*), 6.3.1-6.3.6, as well as the Examples described herein. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6xSSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2.xSSC, 0.01% BSA at 50°C. In another embodiment, a nucleic acid sequence that is hybridizable to one or more of the nucleic acid molecule comprising the nucleotide sequence of SEQ ID NO's 9 to 16, under conditions of moderate stringency is provided. A non-limiting example of moderate stringency hybridization conditions are hybridization in 6xSSC, 5xDenhardt's solution, 0.5% SDS and 100 mg/ml denatured salmon sperm DNA at 55°C, followed by one or more washes in 1xSSC, 0.1% SDS at 37°C. Other conditions of moderate stringency that may be used are well-known within the art, see, e.g., Ausubel et al. (*supra*), and Kriegler, Gene Transfer and Expression, A Laboratory Manual, Stockton Press, (1990). In yet another example, a nucleic acid that is hybridizable to the nucleic acid molecule comprising any one or more of the nucleotide sequences of SEQ ID NO's 9 to 16, under conditions of low stringency, is provided. A non-limiting example of low stringency hybridization conditions are hybridization in 35% formamide, 5xSSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C., followed by one or more washes in 2xSSC, 25 mM Tris-HCl (pH 7.4), 5 mM EDTA, and 0.1% SDS at 50°C. Other conditions of low stringency that may be used are well known in the art, see, e.g., Ausubel et al. (*supra*) and Kriegler (*supra*).

Polynucleotides may possess, when compared to naturally occurring molecules, one or more mutations which are deletions, insertions, or substitutions of nucleotide residues. Mutants can be either naturally occurring (that is to say, isolated from a natural source) or synthetic (for example, by performing site-directed mutagenesis on the nucleic acid).

Usually, monomers of a polynucleotide or oligonucleotide are linked by phosphodiester bonds or analogs thereof to form oligonucleotides ranging in size from a relatively short monomeric units, e.g., 12-18, to several hundreds of monomeric units. Analogs of phosphodiester linkages include: phosphorothioate, phosphorodithioate, phosphoroselenoate, phosphorodiselenoate, phosphoroanilothioate, phosphoranilidate and phosphoramidate.

### Antisense Polynucleotides

The term "antisense polynucleotide" shall be taken to mean a DNA or RNA, or combination thereof, molecule that is complementary to at least a portion of a specific mRNA molecule encoding a polypeptide and capable of interfering with a post-transcriptional event such as mRNA translation. The use of antisense methods is well known in the art (see for example, G. Hartmann and S. Endres, Manual of Antisense Methodology, Kluwer (1999)). The use of antisense techniques in plants has been reviewed by Bourque, 1995 and Senior, 1998. Bourque, 1995 lists a large number of examples of how antisense sequences have been utilized in plant systems as a method of gene inactivation. She also states that attaining 100% inhibition of any enzyme activity may not be necessary as partial inhibition will more than likely result in measurable change in the system. Senior (1998) states that antisense methods are now a very well established technique for manipulating gene expression.

An antisense polynucleotide will hybridize to a target polynucleotide under physiological conditions. As used herein, the term "an antisense polynucleotide which hybridises under physiological conditions" means that the polynucleotide (which is fully or partially single stranded) is at least capable of forming a double stranded polynucleotide with mRNA encoding a protein, such as those provided in any one of SEQ ID NOs 1 to 8 under normal conditions in a cell, preferably a human cell.

Antisense molecules may include sequences that correspond to the structural genes or for sequences that effect control over the gene expression or splicing event. For example, the antisense sequence may correspond to the targeted coding region of the target gene, or the 5'-untranslated region (UTR) or the 3'-UTR or combination of these. It may be complementary in part to intron sequences, which may be spliced out during or after transcription, preferably only to exon sequences of the target gene. In view of the generally greater divergence of the UTRs, targeting these regions provides greater specificity of gene inhibition.

The length of the antisense sequence should be at least 19 contiguous nucleotides, preferably at least 50 nucleotides, and more preferably at least 100, 200, 500 or 1000 nucleotides. The full-length sequence complementary to the entire gene transcript may be used. The length is most preferably 100-2000 nucleotides. The degree of identity of the antisense sequence to the targeted transcript should be at least 90% and more preferably 95-100%. The antisense RNA molecule may of course comprise unrelated sequences which may function to stabilize the molecule.

### Catalytic Polynucleotides

The term catalytic polynucleotide/nucleic acid refers to a DNA molecule or DNA-containing molecule (also known in the art as a "deoxyribozyme") or an RNA or RNA-containing molecule (also known as a "ribozyme") which specifically recognizes a distinct substrate and catalyzes the chemical modification of this substrate. The nucleic acid bases in the catalytic nucleic acid can be bases A, C, G, T (and U for RNA).

Typically, the catalytic nucleic acid contains an antisense sequence for specific recognition of a target nucleic acid, and a nucleic acid cleaving enzymatic activity (also referred to herein as the "catalytic domain"). The types of ribozymes that are particularly useful in this invention are the hammerhead ribozyme (Haseloff and Gerlach, 1988; Perriman et al., 1992) and the hairpin ribozyme (Shippy et al., 1999).

The ribozymes and DNA encoding the ribozymes can be chemically synthesized using methods well known in the art. The ribozymes can also be prepared from a DNA molecule (that upon transcription, yields an RNA molecule) operably linked to an RNA polymerase promoter, e.g., the promoter for T7 RNA polymerase or SP6 RNA polymerase. When the vector also contains an RNA polymerase promoter operably linked to the DNA molecule, the ribozyme can be produced *in vitro* upon incubation with RNA polymerase and nucleotides. In a separate example, the DNA can be inserted into an expression cassette or transcription cassette. After synthesis, the RNA molecule can be modified by ligation to a DNA molecule having the ability to stabilize the ribozyme and make it resistant to RNase.

As with antisense polynucleotides described herein, catalytic polynucleotides should also be capable of hybridizing a target nucleic acid molecule (for example an mRNA encoding any polypeptide provided in SEQ ID NOs 1 to 8) under "physiological conditions", namely those conditions within a cell (especially conditions in an animal cell such as a human cell).

### RNA interference

RNA interference (RNAi) is particularly useful for specifically inhibiting the production of a particular protein. Although not wishing to be limited by theory, Waterhouse et al. (1998) have provided a model for the mechanism by which dsRNA (duplex RNA) can be used to reduce protein production. This technology relies on the presence of dsRNA molecules that contain a sequence that is essentially identical to the mRNA of the gene of interest or part thereof, in this case an mRNA encoding a polypeptide according to the invention. Conveniently, the dsRNA can be produced from a single promoter in a recombinant vector or host cell, where the sense and anti-sense sequences are flanked by an unrelated sequence which enables the sense and anti-sense sequences to hybridize to form the dsRNA molecule with the unrelated sequence forming a loop structure. The design and production of suitable dsRNA molecules is well within the capacity of a person skilled in the art, particularly considering Waterhouse et al. (1998), Smith et al. (2000), WO 99/32619, WO 99/53050, WO 99/49029, and WO 01/34815.

In one example, a DNA is introduced that directs the synthesis of an at least partly double stranded RNA product(s) with homology to the target gene to be inactivated. The DNA therefore comprises both sense and antisense sequences that, when transcribed into RNA, can hybridize to form the double-stranded RNA region. In one example, the sense and antisense sequences are separated by a spacer region that comprises an intron which, when transcribed into RNA, is spliced out. This arrangement has been shown to result in a higher efficiency of gene silencing. The double-stranded region may comprise one or two RNA molecules, transcribed from either one DNA region or two. The presence of the double stranded molecule is thought to trigger a response from an endogenous plant system that destroys both the double stranded RNA and also the homologous RNA transcript from the target plant gene, efficiently reducing or eliminating the activity of the target gene.

The length of the sense and antisense sequences that hybridise should each be at least 19 contiguous nucleotides, preferably at least 30 or 50 nucleotides, and more preferably at least 100, 200, 500 or 1000 nucleotides. The full-length sequence corresponding to the entire gene transcript may be used. The lengths are most preferably 100-2000 nucleotides. The degree of identity of the sense and antisense sequences to the targeted transcript should be at least 85%, preferably at least 90% and more preferably 95-100%. The RNA molecule may of course comprise unrelated sequences which may function to stabilize the molecule. The RNA molecule may be expressed under the control of a RNA polymerase II or RNA polymerase III promoter. Examples of the latter include tRNA or snRNA promoters.

Preferred small interfering RNA ('siRNA') molecules comprise a nucleotides sequence that is identical to about 19-21 contiguous nucleotides of the target mRNA. Preferably, the target mRNA sequence commences with the dinucleotide AA, comprises a GC-content of about 30-70% (preferably, 30-60%, more preferably 40-60% and more preferably about 45%-55%), and does not have a high percentage identity to any nucleotide sequence other than the target in the genome of the animal (preferably human) in which it is to be introduced, e.g., as determined by standard BLAST search.

### microRNA

MicroRNA regulation is a clearly specialized branch of the RNA silencing pathway that evolved towards gene regulation, diverging from conventional RNAi/PTGS. MicroRNAs are a specific class of small RNAs that are encoded in gene-like elements organized in a characteristic inverted repeat. When transcribed, microRNA genes give rise to stem-looped precursor RNAs from which the microRNAs are subsequently processed. MicroRNAs are typically about 21 nucleotides in length. The released miRNAs are incorporated into RISC-like complexes containing a particular subset of Argonaute proteins that exert sequence-specific gene repression (see, for example, Millar and Waterhouse, 2005; Pasquinelli et al., 2005; Almeida and Allshire, 2005).

### Cosuppression

Another molecular biological approach that may be used is co-suppression. The mechanism of co-suppression is not well understood but is thought to involve post-transcriptional gene silencing (PTGS) and in that regard may be very similar to many examples of antisense suppression. It involves introducing an extra copy of a gene or a fragment thereof into a plant in the sense orientation with respect to a promoter for its expression. The size of the sense fragment, its correspondence to target gene regions, and its degree of sequence identity to the target gene are as for the antisense sequences described above. In some instances the additional copy of the gene sequence interferes with the expression of the target plant gene. Reference is made to WO 97/20936 and EP 0465572 for methods of implementing co-suppression approaches.

### Recombinant Vectors

Recombinant vectors can include at least one polynucleotide molecule described herein, and/or a polynucleotide encoding a polypeptide as described herein, inserted into any vector capable of delivering the polynucleotide molecule into a host cell. Such a vector contains heterologous polynucleotide sequences, that is polynucleotide sequences that are not naturally found adjacent to polynucleotide molecules described herein and that preferably are derived from a species other than the species from which the polynucleotide molecule(s) are derived. The vector can be either RNA or DNA, either prokaryotic or eukaryotic, and typically is a transposon (such as described in US 5,792,294), a virus or a plasmid.

One type of recombinant vector comprises the polynucleotide(s) operably linked to an expression vector. The phrase operably linked refers to insertion of a polynucleotide molecule into an expression vector in a manner such that the molecule is able to be expressed when transformed into a host cell. As used herein, an expression vector is a DNA or RNA vector that is capable of transforming a host cell and of effecting expression of a specified polynucleotide molecule. The expression vector can also be capable of replicating within the host cell. Expression vectors can be either prokaryotic or eukaryotic, and are typically viruses or plasmids. Expression vectors include any vectors that function (i.e., direct gene expression) in recombinant cells, including in bacterial, fungal, endoparasite, arthropod, animal, and plant cells. Vectors can also be used to produce the polypeptide in a cell-free expression system, such systems are well known in the art.

"Operably linked" as used herein refers to a functional relationship between two or more nucleic acid (e.g., DNA) segments. Typically, it refers to the functional relationship of transcriptional regulatory element to a transcribed sequence. For example, a promoter is operably linked to a coding sequence, such as a polynucleotide defined herein, if it stimulates or modulates the transcription of the coding sequence in an appropriate host cell and/or in a cell-free expression system. Generally, promoter transcriptional regulatory elements that are operably linked to a transcribed sequence are physically contiguous to the transcribed sequence, i.e., they are *cis-*acting. However, some transcriptional regulatory elements, such as enhancers, need not be physically contiguous or located in close proximity to the coding sequences whose transcription they enhance.

In particular, expression vectors contain regulatory sequences such as transcription control sequences, translation control sequences, origins of replication, and other regulatory sequences that are compatible with the recombinant cell and that control the expression of polynucleotide molecules. In particular, recombinant molecules include transcription control sequences. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the recombinant cells. A variety of such transcription control sequences are known to those skilled in the art. Transcription control sequences include those which function in bacterial, yeast, arthropod, nematode, plant or animal cells, such as, but not limited to, tac, lac, trp, trc, oxy-pro, omp/lpp, rrnB, bacteriophage lambda, bacteriophage T7, T7lac, bacteriophage T3, bacteriophage SP6, bacteriophage SP01, metallothionein, alpha-mating factor, Pichia alcohol oxidase, alphavirus subgenomic promoters (such as Sindbis virus subgenomic promoters), antibiotic resistance gene, baculovirus, *Heliothis zea* insect virus, vaccinia virus, herpesvirus, raccoon poxvirus, other poxvirus, adenovirus, cytomegalovirus (such as intermediate early promoters), simian virus 40, retrovirus, actin, retroviral long terminal repeat, Rous sarcoma virus, heat shock, phosphate and nitrate transcription control sequences as well as other sequences capable of controlling gene expression in prokaryotic or eukaryotic cells.

### Host Cells

Also described herein is a recombinant cell comprising a host cell transformed with one or more recombinant molecules described herein or progeny cells thereof. Transformation of a polynucleotide molecule into a cell can be accomplished by any method by which a polynucleotide molecule can be inserted into the cell. Transformation techniques include, but are not limited to, transfection, electroporation, microinjection, lipofection, adsorption, and protoplast fusion. A recombinant cell may remain unicellular or may grow into a tissue, organ or a multicellular organism. Transformed polynucleotide molecules can remain extrachromosomal or can integrate into one or more sites within a chromosome of the transformed (i.e., recombinant) cell in such a manner that their ability to be expressed is retained.

Suitable host cells to transform include any cell that can be transformed with a polynucleotide. Host cells either can be endogenously (i.e., naturally) capable of producing polypeptides described herein or can be capable of producing such polypeptides after being transformed with at least one polynucleotide molecule as described herein. Host cells can be any cell capable of producing at least one protein defined herein, and include bacterial, fungal (including yeast), parasite, nematode, arthropod, animal and plant cells. Examples of host cells include *Salmonella*, *Escherichia*, *Bacillus, Listeria, Saccharomyces, Spodoptera*, *Mycobacteria, Trichoplusia,* BHK (baby hamster kidney) cells, CHO cells, 293 cells, EL4 cells, MDCK cells, CRFK cells, CV-1 cells, COS (e.g., COS-7) cells, and Vero cells. Further examples of host cells are *E. coli*, including *E. coli* K-12 derivatives; *Salmonella typhi*; *Salmonella typhimurium,* including attenuated strains; *Spodoptera frugiperda*; *Trichoplusia ni*; and non-tumorigenic mouse myoblast G8 cells (e.g., ATCC CRL 1246).

Recombinant DNA technologies can be used to improve expression of a transformed polynucleotide molecule by manipulating, for example, the number of copies of the polynucleotide molecule within a host cell, the efficiency with which those polynucleotide molecules are transcribed, the efficiency with which the resultant transcripts are translated, and the efficiency of post-translational modifications. Recombinant techniques useful for increasing the expression of polynucleotide molecules include, but are not limited to, operatively linking polynucleotide molecules to high-copy number plasmids, integration of the polynucleotide molecule into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of polynucleotide molecules to correspond to the codon usage of the host cell, and the deletion of sequences that destabilize transcripts.

### Gene Therapy

Therapeutic polynucleotide molecules described herein may be employed by expression of such polynucleotides in treatment modalities often referred to as "gene therapy". For example, polynucleotides encoding human 5B6 (for example SEQ ID NO:1), a polynucleotide encoding the ligand of 5B6, or a polynucleotide that up-regulates or down-regulates the production of 5B6 or the ligand thereof in a cell, may be employed in gene therapy techniques for the treatment of disease. Thus, cells from a patient may be engineered with a polynucleotide, such as a DNA or RNA, to encode a polypeptide *ex vivo.* The engineered cells can then be provided to a patient to be treated with the polynucleotide. In this example, cells may be engineered *ex vivo*, for example, by the use of a retroviral plasmid vector containing RNA encoding a polypeptide described herein can be used to transform, for example, stem cells or differentiated stem cells. Such methods are well-known in the art and their use will be apparent from the teachings herein.

Further, cells may be engineered *in vivo* for expression of a polypeptide *in vivo* by procedures known in the art. For example, a polynucleotide encoding a polypeptide as described herein may be engineered for expression in a replication defective retroviral vector or adenoviral vector or other vector (e.g., poxvirus vectors). The expression construct may then be isolated. A packaging cell is transduced with a plasmid vector containing RNA encoding a polypeptide as described herein such as a soluble fragment of human 5B6, such that the packaging cell now produces infectious viral particles containing the gene of interest. These producer cells may be administered to a patient for engineering cells *in vivo* and expression of the polypeptide *in vivo.* These and other methods for administering a polypeptide should be apparent to those skilled in the art.

Retroviruses from which the retroviral plasmid vectors hereinabove-mentioned may be derived include, but are not limited to, Moloney Murine Leukemia Virus, Spleen Necrosis Virus, Rous Sarcoma Virus, Harvey Sarcoma Virus, Avian Leukosis Virus, Gibbon Ape Leukemia Virus, Human Immunodeficiency Virus, Adenovirus, Myeloproliferative Sarcoma Virus, and Mammary Tumor Virus. In one example, the retroviral plasmid vector is derived from Moloney Murine Leukemia Virus.

Such vectors will include one or more promoters for expressing the polypeptide. Suitable promoters which may be employed include, but are not limited to, the retroviral LTR; the SV40 promoter; and the human cytomegalovirus (CMV) promoter. Cellular promoters such as eukaryotic cellular promoters including, but not limited to, the histone, RNA polymerase III, the metallothionein promoter, heat shock promoters, the albumin promoter, the 5B6 promoter, human globin promoters and β-actin promoters, can also be used. Additional viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The retroviral plasmid vector can be employed to transduce packaging cell lines to form producer cell lines. Examples of packaging cells which may be transfected include, but are not limited to, the PE501, PA317, Y-2, Y-AM, PA12, T19-14X, VT-19-17-H2, YCRE, YCRIP, GP+E-86, GP+envAm12, and DAN cell lines as described by Miller (1990). The vector may be transduced into the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line will generate infectious retroviral vector particles, which include the nucleic acid sequence(s) encoding the polypeptide (for example). Such retroviral vector particles may then be employed to transduce eukaryotic cells, either *in vitro* or *in vivo.* The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, myocytes (particularly skeletal muscle cells), endothelial cells, and bronchial epithelial cells.

Genetic therapies may involve a transient (temporary) presence of the gene therapy polynucleotide in the patient or the permanent introduction of a polynucleotide into the patient.

Genetic therapies, like the direct administration of agents discussed herein, may be used alone or in conjunction with other therapeutic modalities.

### Pharmaceutical Compositions, Dosages, and Routes of Administration

Compositions comprising the compound together with an acceptable carrier or diluent are useful.

Therapeutic compositions can be prepared by mixing the desired component (such as compound that binds 5B6) having the appropriate degree of purity with optional pharmaceutically acceptable carriers, excipients, or stabilizers (Remington's Pharmaceutical Sciences, 16th edition, Osol, A.ed. (1980)), in the form of lyophilized formulations, aqueous solutions or aqueous suspensions. Acceptable carriers, excipients, or stabilizers are preferably nontoxic to recipients at the dosages and concentrations employed, and include buffers such as Tris, HEPES, PIPES, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; salt-forming counter-ions such as sodium; and/or non-ionic surfactants such as TWEEN™, PLURONICS™ or polyethylene glycol (PEG).

Additional examples of such carriers include ion exchangers, alumina, aluminum stearate, lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, colloidal silica, magnesium trisilicate, polyvinyl pyrrolidone, and cellulose-based substances.

Therapeutic compositions to be used for *in vivo* administration should be sterile. This is readily accomplished by filtration through sterile filtration membranes, prior to or following lyophilization and reconstitution. The composition may be stored in lyophilized form or in solution if administered systemically. If in lyophilized form, it is typically formulated in combination with other ingredients for reconstitution with an appropriate diluent at the time for use. An example of a liquid formulation is a sterile, clear, colorless unpreserved solution filled in a single-dose vial for subcutaneous injection.

Therapeutic compositions generally are placed into a container having a sterile access port, for example, an intravenous solution bag or vial having a stopper pierceable by a hypodermic injection needle. The compositions are preferably administered subcutaneously, intramuscularly or parenterally, for example, as intravenous injections or infusions or administered into a body cavity.

The compound may be administered in an amount of about 0.001 to 2000 mg/kg body weight per dose, and more preferably about 0.01 to 500 mg/kg body weight per dose. Repeated doses may be administered as prescribed by the treating physician.

Single or multiple administrations of the compositions are administered depending on the dosage and frequency as required and tolerated by the patient. The dosage and frequency will typically vary according to factors specific for each patient depending on the specific therapeutic or prophylactic agents administered, the severity and type of disease or immune response required, the route of administration, as well as age, body weight, response, and the past medical history of the patient. Suitable regimens can be selected by one skilled in the art by considering such factors and by following, for example, dosages reported in the literature and recommended in the Physician's Desk Reference, 56th ed., (2002). Generally, the dose is sufficient to treat or ameliorate symptoms or signs of disease without producing unacceptable toxicity to the patient.

In an example, the composition comprises liposomes or membrane vesicles. Examples of such liposomes are described in US 2007/0026057, Leserman (2004) and van Broekhoven et al. (2004). In these instances the compound can be used to target the liposome to enhance the delivery of an agent of interest. As outlined in US 2007/0026047, processes for the preparation of membrane vesicles are described in WO 00/64471.

Compositions for detection of cells with a disrupted cell membrane, cells infected with a pathogen, dying cells or dead cells, or a portion thereof, modulating an immune response, and/or antigen recognition, processing and/or presentation, are conventionally administered parenterally, by injection, for example, subcutaneously, intramuscularly or intravenously. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the composition is lyophilised, the lyophilised material may be reconstituted prior to administration, e.g. as a suspension. Reconstitution is preferably effected in buffer. Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

In any treatment regimen, the therapeutic composition may be administered to a patient either singly or in a cocktail containing other therapeutic agents or compositions.

### EXAMPLES

### Example 1- Cloning and Expression of 5B6

### Materials and Methods

### Mice

Mice were bred under specific pathogen free conditions at The Walter and Eliza Hall Institute (WEHI). Female mice were used at 6 - 12 weeks of age; alternatively, gender aged-matched cohorts were generated. Animals were handled according to the guidelines of the National Health and Medical Research Council of Australia. Experimental procedures were approved by the Animal Ethics Committee, WEHI.

### Sequence Identification of 5B6

Sequencing was performed using the Big Dye Terminator version 3.1 (Applied Biosystems, Victoria, Australia) and 200ng plasmid DNA, and subjected to electrophoresis on an ABI 3730x1 96-capillary automated DNA sequencer. Comparison of sequences to the expressed sequence tag, cDNA and protein databases was performed by basic local alignment search tool (BLAST) using National Center for Biotechnology Information (www.ncbi.nlm.nih.gov). Genomic localisation was performed by BLAST alignment to the mouse assembly (February 2006) and human assembly (March 2006) using University of California Santa Cruz, Genome Browser (www.genome.ucsc.edu/).

### Quantitative RT-PCR

RNA (up to 1 µg) was DNase treated with RQ1 DNase (Promega) then reverse transcribed into cDNA using random primers (Promega) and Superscript II reverse transcriptase (Gibco BRL, Geithersburg, MD). Real-time reverse transcription PCR (RT-PCR) was performed to determine the expression of *5B6* and *Gapdh* in hemopoietic cells using the Quantitect SYBR Green PCR kit (Qiagen) and a Light cycler (Roche, Victoria; Australia). The specific primers for real-time RT-PCR were as follows: *5B6*; 5'-TGTGACTGCTCCCACAACTGGA-3' (SEQ ID NO:17); 5'-TTTGCACCAATCACAGCACAGA-3' (SEQ ID NO:18), *Gapdh*; *5'-*CATTTGCAGTGGCAAAGTGGAG-3' (SEQ ID NO:19); 5'-GTCTCGCTCCTGGAAGATGGTG-3' (SEQ ID NO:20). An initial activation step for 15 min at 95°C was followed by 40 cycles of: 15s at 94°C (denaturation), 20-30s at 50-60°C (annealing) and 10-12s at 72°C (extension), followed by melting point analysis. The expression level for each gene was determined using a standard curve prepared from 10⁻²-10⁻⁶ pg of specific DNA fragment, and was expressed as a ratio relative to *Gapdh.*

### Recombinant surface expression of 5B6

Full length mouse and human 5B6 (m5B6 and h5B6) were isolated by PCR amplification from splenic DC cDNA using Advantage cDNA polymerase (Clontech) and the following primers: [5B6: 5'-GCCATTTCTTGTACCAACCTACTCCT-3' (SEQ ID NO:21); 5'-CGGTGTGGTATGGATCGTCACTT-3' (SEQ ID NO:22)], [HE: 5'-AGCCTCCTGTGTGGACTGCTTT-3' (SEQ ID NO:23); 5'-TTCATGGCCCACATTTTGGTTT-3' (SEQ ID NO:24)], and the resultant products were subcloned into pGemT easy plasmid (Promega). m5B6 and h5B6 were expressed on the surface of Chinese hamster ovary (CHO) cells as C-terminal (extracellular) FLAG-tagged proteins and on the surface of mouse EL4 cells as a fusion protein where green fluorescent protein (GFP) was fused to the N-terminal cytoplasmic domain of 5B6. To generate the FLAG tagged proteins, 5B6 encoding cDNA was amplified using Advantage high fidelity polymerase (Clontech), restriction digested with AscI and Mlu-1 and subcloned into a pEF-Bos vector modified to contain the FLAG epitope (kindly donated by Dr T. Willson; WEHI).

CHO cells were co-transfected with the pEF-Bos-5B6 lectin and a pGK-neo plasmid containing the neomycin phosphotransferase gene by electoporation (Gene Pulsar, Biorad, NSW, Australia) and transfectants selected with 1 mg/ml G418 (Geneticin, Life Technologies). 5B6 lectin-positive cells were stained with a rat anti-FLAG mAb, followed by an anti-rat Ig-PE (Caltag), and then isolated by flow cytometric sorting. GFP-tagged proteins were generated by amplifying the 5B6 lectin encoding cDNA, restriction digesting with EcoRI and subcloning into pEGFP-C2 vector (Clontech), before electroporation into EL4 cells and selection with 1mg/ml G418. 5B6-positive cells were isolated by flow cytometric sorting of GFP positive cells. Full length untagged proteins were generated by amplifying the 5B6 lectin encoding cDNA, restriction digesting with EcoRI and subcloning into a pIRES-Neo vector, before electroporation into CHO cells and selection with 1mg/ml G418.

### Generation of mAb against C-type lectins,

Wistar rats were immunised three to four times with 50 µg Keyhole Limpet Hemocyanin (KLH)-conjugated peptide: 5B6 mouse peptide (H-DGSSPLSDLLPAERQRSAGQIC-OH) (SEQ ID NO:29), human peptide (H-RWLWQDGSSPSPGLLPAERSQSANQVC-OH) (SEQ ID NO:30), or 1 x 10⁷ CHO cells expressing 5B6-FLAG at 4 week intervals, and given a final boost 4 days before fusion with Sp2/0 myeloma cells. Hybridomas secreting specific mAb were identified by flow cytometric analysis of supernatants using CHO cells expressing C-type 5B6-FLAG and EL4 cells expressing GFP-5B6. Hybridomas were generated that displayed specific reactivity to each of mouse 5B6 and human 5B6.

In summary, the following mAb were generated and utilised in this study, two rat mAb 24/04-10B4 (from peptide immunisation) and 42/04-42D2 (from CHO-5B6-FLAG immunisation) were raised against mouse 5B6 (m5B6). Two rat antibodies 20/05-3A4 and 23/05-4C6 (from h5B6 peptide immunisation) were raised against h5B6.

### Isolation and flow cytometric analysts of DCs

DC isolations from lymphoid organs were performed as previously described (Vremec et al., 2000). Briefly, tissues were mechanically chopped, digested with collagenase and DNAse and treated with ethylenediamine tetraacetic acid (EDTA). Low-density cells were enriched by density centrifugation (1.077 g/cm³ Nycodenz, Axis-Shield, Oslo, Norway). Non-DC-lineage cells were coated with mAb (KT3-1.1, anti-CD3; T24/31.7, anti-Thy1; TER119, anti-erythrocytes; ID3, anti-CD19; and 1A8, anti-Ly6G) then removed using anti-rat Ig magnetic beads (Biomag beads, QIAGEN, Victoria, Australia). Blood DCs were enriched by removing red blood cells (RBC) (0.168M NH₄Cl; 5 min at 4°C) and depletion of irrelevant cells as above, except the mAb cocktail also contained the mAb F4/80. DC-enriched populations were blocked using rat Ig and anti-FcR mAb (2.4G2), then stained with fluorochrome-conjugated mAb against CD11c (N418), CD205 (NLDC-145), CD4 (GK1.5), CD8 (YTS169.4), CD24 (M1/69), 120G8 or CD45RA (14.8), Sirpα (p84) and m5B6 (24/04-10B4-biotin).

cDCs were selected as CD11c^{hi}CD45RA⁻ or CD11c^{hi}120G8⁻; splenic cDC were further subdivided into CD4⁺cDC (CD11c^{hi}CD45RA⁻CD4⁺ CD8⁻), double negative (DN) cDC (CD11c^{hi}CD45RA⁻CD4⁻CD8⁻) and CD8⁺cDC (CD11c^{hi}CD45RA⁻CD8⁺ CD4⁻); thymic DCs were subdivided into CD8⁻cDC (Sirpα^{hi}CD8^{lo}) and CD8⁺cDC (Sirpα^{lo}CD8^{hi}); and LN cDC were subdivided into CD8⁻cDC (CD11c^{hi}CD205⁻CD8⁻), dermal DC (CD11c⁺CD205^{int}CD8⁻), Langerhans' cells (CD11c⁺CD205^{hi}CD8⁻) and CD8⁺cDC (CD11c⁺CD205^{hi}CD8⁺), as described previously (Lahoud et al., 2006). pDCs were separated as CD11c^{int}CD45RA⁺ or CD11c^{int}120G8⁺. Biotin staining was detected using streptavidin (SA)- phycoerythrin (PE). The expression of m5B6 on the various DC populations was analysed and compared to isotype control staining (IgG2a, BD Pharmingen, San Diego, CA, USA). Flow cytometric analysis was performed on an LSR II (Becton Dickinson, Franklin Lakes, NJ, USA), excluding autofluorescent and propidium iodide (PI) positive dead cells.

### Isolation and flow cytometric analysis of human blood DCs and hemopoietic cells

Peripheral blood mononuclear cells (PBMC) were isolated from human blood using Ficoll-Pacque-PLUS (GE Healthcare, Rydalmere, NSW, Australia) density separation. Blood donors gave with informed consent and collection was approved by Human Research Ethics Committee, Melbourne Health. The PBMC were blocked using rat Ig and anti-FcR mAb (2.4G2) then stained with mAb against HLA-DR (L243; Becton Dickinson), and a cocktail of PE-conjugated mAb against lineage markers, namely CD3 (BW264156; T cells), CD14 (Tuk4; monocytes), CD19 (6D5; B cells) and CD56 (AF12-7H3; NK cells). Blood DCs were gated as HLA-DR^{hi}, lineage⁻ cells and further segregated based on their expression of BDCA-1 (ADJ-8E3), BDCA-3 (AD5-14H2), BDCA-4 (AD5-17F6) and CD16 (VEP13). PBMC were also used as a source of other hemopoietic cells that were isolated using mAb against CD3 (BW264156; T cells), CD19 (6D5; B cells), CD56 (AF12-7H3), and NKp46 (9E2) (CD56⁺NKp46⁺; NK cells) and CD14 (Tuk4; monocytes). Staining and flow cytometric analysis for the expression of h5B6 (20/05-3A4) was performed, excluding PI positive dead cells. Unless otherwise specified, all anti-human mAb were purchased Miltenyi Biotec (North Ryde, NSW, Australia).

### Isolation and analysis of 5B6 on mouse hemopoietic cells

Spleen cell suspensions were prepared as for DC isolation (Vremec et al., 2000). Cells were stained with mAb against CD3 (KT3-1.1), CD19 (ID3), NK1.1 (PK136), CD49b (Hmα2; eBioscience, San Diego, CA, USA) then B cells (CD19⁺CD3⁻), T cells (CD19⁻CD3⁺) and NK cells (CD49b⁺NK1.1⁺CD3⁻) were selected. Splenic macrophages were first enriched by a 1.082 g/cm³ density centrifugation (Nycodenz) and immunomagnetic bead depletion of CD3⁺ T cells and CD19⁺ B cells; the enriched cells were stained with mAb against CD11b (M1/70) and F4/80, then macrophages were gated as CD11b^{hi}F4/80⁺. Bone marrow macrophages and monocytes were first enriched as for spleen, then stained with CD11b (M1/70) and Ly6C (5075-3.6); monocytes were then gated as side-scatter^{lo}Ly6C^{hi}CD11b^{hi} and macrophages as Ly6C^{int}CD11b^{hi}. All cells were blocked using rat Ig and anti-FcR mAb (2.4G2) before immunofluorescence staining with the various mAb cocktails including anti-5B6 mAb (10B4-biotin). Biotin staining was detected using streptavidin-PE. Samples were analysed for their expression of 5B6 on an LSR II (Becton Dickinson), excluding PI positive dead cells.

### Recombinant expression of soluble 5B6

To generate soluble 5B6, cDNA containing the hinge and ectodomain regions was amplified using Advantage high fidelity 2 polymerase (Clontech) and the following primers [m5B6: 5'-TAGTAGACGCGTGAGCAGCAGGAAAGACTCATC-3' (SEQ ID NO:25); 5'-TAGTAGACGCGTTCAGATGCAGGATCCAAATGC-3'] (SEQ ID NO:26), [H5B6:5'-TAGTAGACGCGTCAGCAGCAAGAAAAACTCATC-3' (SEQ ID NO:27); 5'-TAGTAGACGCGTTCAGACAGAGGATCTCAACGC-3'] (SEQ ID NO:28). The amplified cDNA was restriction digested with Mlu-1 and subcloned into the Mlu-1 site of a pEF-Bos vector modified to contain the biotinylation consensus sequence (a peptide consensus sequence NSGLHHILDAQKMVWNHR (SEQ ID NO:31) recognised specifically by *E coli* biotin holoenzyme synthetase BirA and the FLAG epitope. The resulting lectin fusion constructs thus included (in order of N-terminus): the IL3 signal sequence (to ensure secretion), the biotinylation consensus peptide sequence, a FLAG-tag, the hinge region and the lectin domain. Recombinant proteins were expressed by transient transfection of 293T cells (a human renal epithelial cell line stably transfected with polyoma/ SV40 large T antigen) in DMEM-10% FCS with 8 micrograms DNA/ 75cm2 flask using Fugene . After 8h, the media was removed, the cells washed twice, then incubated for 36-60h in 10ml X-Vivo-10 protein-free/ serum-free media (BioWhittaker, Walkersville, MD). The media containing the secreted recombinant protein was harvested, and recombinant protein from the culture supernatant concentrated 100-fold using a 10,000 mwt cutoff centrifugal device (Nanosep 10K Omega, PALL Life Sciences). The concentrated protein was then used directly or enzymatically biotinylated using BIR enzyme (Avidity, Denver, CO).

### Binding assays to investigate the binding of soluble 5B6 to membrane bound 5B6

293T cells were transiently transfected with expression constructs encoding full length untagged 5B6 in pIRES Neo. Two-three days later, cells were harvested and surface immunofluorescence labeled using either (1) soluble FLAG-tagged biotinylated m5B6, h5B6 and Cire, and detected with Streptavidin PE, or (2) soluble FLAG-tagged 5B6, biotinylated anti-FLAG mAb 9H10, and Streptavidin-PE. Live cells were gated on forward and side scatter, or by propidium iodide exclusion and analysed for their surface binding of soluble 5B6. The specificity of the binding of soluble 5B6 was demonstrated by comparison to binding to other soluble FLAG-tagged C-type lectins, such as Cire.

### ELISA

Recombinant soluble protein secretion was assayed by capture/ two-site ELISA. Briefly, 96-well polyvinylchloride microtitre plates (Costar, Broadway, Cambridge, UK) were coated with purified capture mAb, namely, anti-FLAG 9H10 12.5ug/ml (generated in-house). Culture supernatants were detected using the biotinylated anti-m5B6 antibody (24/04-10B4) - (2ug/ml), Streptavidin-HRP and ABTS substrate. Biotinylated recombinant soluble protein was assayed by capture/ two-site ELISA. Briefly, 96-well polyvinylchloride microtitre plates (Costar, Broadway, Cambridge, UK) were coated with purified capture mAb, namely, anti-FLAG 9H10 12.5ug/ml (generated in-house). Culture supernatants were detected using Streptavidin-HRP and ABTS substrate.

### Results

### Comparison of gene expression patterns between splenic DC subsets

Gene expression profile analysis identified a murine cDNA clone that is preferentially expressed by the CD8⁺ cDC subset relative to the CD8- cDC. This clone, termed 5B6, represented a fragment of a "hypothetical C-type lectin", a gene found on chromosome 6, that was differentially expressed in CD8⁺ DC (Riken 9830005G06, (recently named C-type lectin domain family 9, member A, (Clec9a) Genbank accession AK036399.1, Unigene ID Mm.391518). Furthermore, analysis of the public databases revealed a human orthologue for 5B6 (HEEE9341) on chromosome 12, recently renamed CLEC9A. Orthologs have been identified to exist in other animals such as chimpanzees (Genbank accession XP_001143778), Rhesus monkeys (XP_001114857), dogs (Genbank accession XP_854151), cows (XP_873119), horses (up_001493987) and rats (Genbank accession XP_578403).

### Identification, characterisation and cloning of the C-type lectins

The inventors amplified the full-length cDNA encoding mouse and human 5B6 by PCR and sequenced the genes (Figure 1A and 1B).

The full-length coding sequence of mouse *5B6*, encoded by 7 exons spanning 13.4 kb of genomic DNA (Figure 1D), contains a single open reading frame (ORF) (795 bp) encoding a protein of 264 amino acid (aa) (Figure 1C). Human *5B6* coding sequence, is encoded by 6 exons spanning 12.9 kb of genomic DNA (Figure 1D), similarly contains a single ORF encoding a protein of 241 aa (Figure 1C).

The mouse and human *5B6* gene each encode a putative transmembrane protein with a single C-type lectin domain in its extracellular region, a transmembrane region and a cytoplasmic tail containing the YXXL residues, which is a potential signalling motif (Fuller et al., 2007) (Figure 1C). Human 5B6 has shorter hinge region than mouse. An alignment of the mouse and human protein sequences is demonstrated in Fig. 1C (53% identical; 69% similar). A schematic representation of the proposed mouse and human 5B6 protein structure is shown in Figure 1E.

Using NCBI Blast protein analysis, it was determined that m5B6 shares most sequence similarity with mouse Dectin-1 (Clec7A), Clec12B, and NKG2D, whereas h5B6 is most similar to LOX-1 (Clec8A), Clec12B, and DCAL-2 (Clec12A). The CTLD of 5B6, like the classical C-type lectin the rat mannose binding protein A (MBP-A), has four conserved cysteine residues that form two disulfide bonds (Figure 2). Furthermore, 5B6 possesses two additional cysteine residues in the neck region allowing protein homodimerization (Weis et al., 1998). Critically, the residues involved in Ca²⁺ binding in classical C-type lectins are not present in mouse and human 5B6 (Figure 2).

### Expression of C-type lectin genes

Microarray analysis predicted 5B6 to be expressed at 3.5 fold higher levels in CD8⁺ DC relative to CD8⁻ DC, and at 2.6-fold higher levels in CD8⁺ DC relative to the DN DC. Hence, the inventors designed primers and investigated the expression of 5B6, by quantitative RT-PCR, in mouse splenic cDC subsets. It was confirmed that 5B6 was preferentially expressed by the CD8⁺ cDC; splenic CD8⁺ DC expressed 22-fold more mRNA than splenic CD4⁺ cDC (Figure 3A).

The inventors examined the expression of mouse and human *5B6* genes across a panel of haemopoietic cell types by quantitative real-time RT-PCR. *5B6* mRNA expression was specific to DC, both cDC and pDC, with moderate levels of mRNA expression in NK cells (Figure 3B). It was preferentially expressed in splenic CD8⁺ DC relative to CD8⁻ cDC. It was also differentially expressed in the thymic CD8⁺ cDC and the LN CD8⁺DEC205^{hi} cDC (Figure 3A). Furthermore, the gene expression in all three splenic cDC populations was reduced 3 h after *in vivo* activation with CpG and LPS, ligands to Toll like receptor 9 and 4 respectively (Figure 3C).

### Surface expression of mouse 5B6 protein

To investigate the protein expression of m5B6 and h5B6, the inventors generated mAbs that recognised protein on the surface of 5B6-transfected cells by flow cytometry. Staining of a panel of freshly isolated mouse hemopoietic cells with the mAb 10B4 indicated that m5B6 was expressed on a subset of cDCs and on most pDCs (Figure 4A). Strikingly, m5B6 protein was not detected on most other hemopoietic cells investigated, including T cells, most B cells, monocytes and macrophages. Nor was it detected on the NK cells that expressed some mRNA (Figure 4A). However, a small (3 %) proportion of B cells, displayed clear positive staining for m5B6. Only around 3 % of bone marrow cells showed any staining with 10B4, and most of this was weak. Thus, in the hemopoietic system, m5B6 surface expression appears mainly restricted to DCs (Figure 4A). In addition, staining of frozen sections with the mAb 10B4 revealed no staining beyond that attributed to DCs (data not shown).

Surface levels of m5B6 were then compared on splenic, LN and thymic cDCs. m5B6 was expressed by the CD8⁺cDCs of spleen, thymus and LN (Figure 4A). Most splenic, thymic and LN CD8⁻cDCs and the migratory cDCs (dermal DCs and Langerhans' cells) were negative for m5B6 expression (Figure 4A, B). However, a small proportion of CD8⁻cDCs showed above background staining; this could be attributed to a small proportion of DCs of the CD8⁺cDC lineage not yet expressing CD8α, known to be present within this CD8⁻cDC gating. No m5B6 staining was detected on a preparation of inflammatory CD11^{int}CD11b^{hi} DCs from inflamed mouse spleens (Naik et al., 2006) (data not shown). These DC surface expression profiles were consistent with the gene expression observed by quantitative RT-PCR (Figure 3).

### Surface expression of mouse 5B6 on mouse blood DC

Mouse blood contains very few mature DC (CD11c^{hi}) compared to the DC found within the spleen and these few blood DC lack the expression of CD8 (O'Keeffe et al., 2003). However, in the mouse, CD24 expression has correlated with the expression of CD8. A small portion of mature DC within the blood express this marker; presumably these cells are on their way to becoming CD8⁺. To determine the expression of 5B6 on blood DC, we isolated them and stained them with CD24 and 5B6. DC expressing CD24 (which are destined to become CD8⁺DC) also express 5B6 (Figure 4C).

### Surface expression of macaque and human 5B6

To investigate the surface expression of human 5B6 (h5B6), we generated two monoclonal antibodies (20/05-3A4; 23/05-4C6) that recognised native protein on the surface of h5B6-transfectant cells, as measured by flow cytometry (data not shown). Staining of freshly isolated peripheral blood cells, from humans or from macaque monkeys, indicated that 5B6 was expressed on a subset of DC (Figure 5). In particular, a small subset of HLADR⁺ DCs were positive for h5B6 (Figure 5A). Most other human blood cells did not show positive staining, but low level staining was obtained on human blood B cells (Figure 5B). To determine if the 5B6-expressing DCs resembled those seen in mouse blood, the blood DCs were also stained with BDCA-1, BDCA-3 and BDCA-4. Staining with mAb 3A4 was restricted to the minor BDCA-3⁺ DC subset (proposed equivalents of mouse CD8⁺ cDC¹⁷), and absent from BDCA-4⁺ subset (data not shown). This suggests h5B6 is present on a cDC type similar to the mouse CD24⁺, CD8⁺ DC lineage (Galibert et al., 2005), but in contrast to the mouse, not on pDCs.

### Soluble 5B6 can interact with membrane bound 5B6 in a cross-species manner

To identify binding partners for the 5B6 molecule, the inventors generated the soluble FLAG-tagged m5B6 and h5B6, and a control soluble FLAG-tagged C-type lectin Cire. The soluble 5B6 was screened for binding to 293T cells expressing membrane bound m5B6 and h5B6 following transient transfections with full length untagged 5B6 constructs in a pIresNeo vector. Soluble mouse 5B6 was able to bind to live 293T cells expressing both the membrane bound mouse 5B6 and human 5B6 but showed minimal or no binding to the mock (no DNA) transfected 293T cells (Figure 6). Similarly, soluble human 5B6 was able to bind to live 293T cells expressing both the membrane bound mouse 5B6 and human 5B6 but showed no binding to mock transfected 293T cells. In contrast the control soluble molecule Cire showed only minimal binding to the control or transfectant cell lines. Thus, soluble 5B6 can interact with membrane bound 5B6 in a cross-species manner.

### Example 2 - 5B6 ligand expressed by dying and dead cells

### Materials and Methods

### Nomenclature

Throughout this Example 5B6 is referred to as Clec9A.

### Mice

Female C57BL/6J Wehi mice, 8-12 weeks of age, were bred under specific pathogen free conditions at The Walter and Eliza Hall Institute (WEHI). Animals were handled according to the guidelines of the National Health and Medical Research Council of Australia. Experimental procedures were approved by the Institutional Animal Ethics Committee, WEHI.

### Recombinant expression of soluble Clec9A

Two versions of soluble Clec9A were generated, a full Clec9A ectodomain (Clec9A-ecto; stalk and CTLD), and a Clec9A CTLD only (Clec9A-CTLD). Soluble ectodomain mouse Clec9A is provided as SEQ ID NO:40, soluble ectodomain human Clec9A is provided as SEQ ID NO:41, soluble CTLD only mouse Clec9A is provided as SEQ ID NO:42, and soluble CTLD only human Clec9A is provided as SEQ ID NO:43.

cDNA containing the required ectodomain region was amplified from the original Clec9A cDNA sequence (Caminschi et al., 2008) using Advantage high fidelity 2 polymerase (Clontech) or HotStar HiFidelity polymerase (Qiagen) and the following primers: mClec9A-ecto-forward: 5'-TAGTAGACGCGTGAGCAGCAGGAAAGACTCATC-3' (SEQ ID NO:25); mClec9A-CTLD-forward: 5'-TAGTAGACGCGTGGTAGTGACTGCAGCCCTTGT-3' (SEQ ID NO:38); mClec9A-reverse 5'-TAGTAGACGCGTTCAGATGCAGGATCCAAATGC-3' (SEQ ID NO:26). hCLEC9A-ecto-forward: 5'-TAGTAGACGCGTCAGCAGCAAGAAAAACTCATC-3' (SEQ ID NO:27); hCLEC9A-CTLD-forward: 5'-TAGTAGACGCGTAACAGCAGTCCTTGTCCAAACAAT -3' (SEQ ID NO:39); hCLEC9A-reverse: 5'-TAGTAGACGCGTTCAGACAGAGGATCTCAACGC-3' (SEQ ID NO:28).

The amplified cDNA was subcloned into a pEF-Bos vector modified to contain the biotinylation consensus sequence (a peptide consensus sequence NSGLHHILDAQKMVWNHR (SEQ ID NO:31) recognised specifically by *E coli* biotin holoenzyme synthetase BirA) and the FLAG epitope. The resulting fusion constructs thus included (in order of N-terminus): the IL3 signal sequence (to ensure secretion), the biotinylation consensus peptide sequence, a FLAG-tag, and Clec9A cDNA fragment. Tagged soluble ectodomain mouse Clec9A is provided as SEQ ID NO:44, tagged soluble ectodomain human Clec9A is provided as SEQ ID NO:45, tagged soluble CTLD only mouse Clec9A is provided as SEQ ID NO:46, and tagged soluble CTLD only human Clec9A is provided as SEQ ID NO:47.

Recombinant proteins were expressed by transient transfection of mammalian cells and culture in protein-free/ serum-free media: 293T cells followed by culture in X-Vivo-10 media (BioWhittaker) or FreeStyle 293F cells cultured in FreeStyle Expression Media (Invitrogen). Media containing the secreted recombinant protein was assayed for the presence of soluble mClec9A by reactivity with anti-mouse Clec9A mAb (24/04-10B4). The secreted recombinant protein was concentrated 100-fold using a 10,000 mol wt cutoff centrifugal device (Millipore) and either used directly or enzymatically biotinylated using BIR enzyme (Avidity). Where required, Clec9A soluble proteins were purified by affinity chromomatography using an anti-FLAG M2 agarose resin (Sigma) and elution with 100µg/ml FLAG peptide (Auspep), and further purified by size-exclusion chromatography using a pre-packed Superdex 200 column (GE Healthcare).

### Mouse embryonic fibroblasts

Mouse embryonic fibroblasts (MEF) expressing Noxa (van Delft et al., 2006) were seeded a day previously and grown to approximately 80% confluence then induced to undergo apoptosis by treatment with 2.5µM ABT-737. After 16h. cells were harvested using Cell Dissociation Buffer (Enzyme-free PBS-based; Gibco).

### Binding assays using soluble Clec9A

Binding assays were performed in binding buffer (PBS containing 0.2%BSA/ and 0.02% sodium azide), on ice. Cells were washed 3 times with PBS to remove serum proteins, then resuspended in binding buffer. Cells were incubated with either (1) biotinylated soluble Clec9A and controls, and detected with SA-PE, or (2) soluble FLAG-tagged Clec9A, biotinylated anti-FLAG mAb 9H10, then detected with SA-PE. Live cells were gated on forward and side scatter, or by propidium iodide (PI) exclusion, whereas dead cells were gated on forward and side scatter, or by PI inclusion. Analysis of soluble Clec9A binding was performed by flow cytometry using a FACScan (Becton Dickinson). The specificity of the binding was demonstrated by comparison to binding to other soluble FLAG-tagged C-type lectins, mouse Cire/mDCSign (Caminschi et al., 2001) and Clec12A (Pyz et al., 2008).

### Phagocytic uptake of dead cell by DCs from lymphoid organs

Splenic DC were isolated as previously described (Caminschi et al., 2008) and assayed for uptake of dead cells (modified from Schnorrer et al., 2006). In brief, DC were labelled with antibodies to CD11c (N418-allophycocyanin (APC)) and CD8 (YTS169.4-FITC). Splenocytes were subjected to two rounds of freezing then thawing (30s on dry ice followed by 30s at 37°C) then labelled with 250ng/ml PI for 10 min at 4°C, and the excess PI dye washed away. Labelled freeze-thawed splenocytes (1 x 10⁶ cells/ well) were incubated with soluble proteins, mClec9A-ecto, mClec9A-CTLD or the control protein Cire, at 25µg/ml for 30 min at 4°C, before the addition of 2.5 x 10⁵ DC in modified RPMI-1640 medium containing 10%FCS, 100 U/ml penicillin, 100µg/ml streptomycin, and 10⁻⁴ M 2-ME. The cocultures were incubated for 3 h at 37°C to enable phagocytosis, or at 4°C (control) then harvested for flow cytometric analysis using an LSRII (Becton Dickinson). DC were gated as CD11c⁺CD8⁺ or CD11c⁺CD8⁻ cells and the proportion of DC that were PI⁺ calculated. Uptake at 4°C was a measure of binding of dead splenocytes to the DC surface, whereas the additional uptake at 37°C was a measure of phagocytic uptake.

### Results and Discussion

### Generation of soluble Clec9A ectodomains

To seek Clec9A ligands, the inventors first generated tagged soluble forms of the ectodomains of both the mouse and human molecules. Constructs were made encoding either the full Clec9A ectodomain (Clec9A-ecto; stalk region and Clec9A CTLD), or the Clec9A CTLD only (Clec9A-CTLD), and including a FLAG-tag and a biotinylation consensus sequence (Brown et al., 1998) (Figure 7A). Recombinant soluble mClec9A and hCLEC9A proteins were expressed in mammalian cells using protein-free / serum-free media. They were either enzymatically biotinylated using BirA (Avidity) enzyme (Figure 7C) and detected with Streptavidin-PE (SA-PE), or they were detected using anti-FLAG reagents. As controls, similar constructs were generated for the other C-type lectins, mouse Cire/ mDCSign (Caminschi et al., 2001) and Clec12A (Pyz et al., 2008).

### Mouse and human Clec9A bind to dead cells

A variety of mouse and human cells were screened to determine if soluble Clec9A could bind to the cell surface; low or minimal binding was observed with normal cells but we observed Clec9A binding to cells that were dead based on staining with the nuclear dye propidium iodide (PI) which stains cell nuclei once the cell membrane is damaged. Therefore, the inventors investigated the binding of mClec9A to thymocytes induced to undergo apoptosis using γ-irradiation.

Thymocytes were stained with Annexin V, an early marker of apoptosis, and with PI to mark cells damaged to the point of having disrupted cell membranes. mClec9A-ecto strongly bound to some apoptotic mouse thymocytes, but not to their viable counterparts; notably binding was restricted to late stage apoptotic/secondary necrotic cells (Annexin V⁺PI⁺) but not to early stage Annexin V⁺ apoptotic cells (Figure 8A).

The present inventors further investigated mouse embryonic fibroblasts (MEF) induced to undergo apoptosis induced by the BH3 mimetic drug ABT-737. They found that both mClec9A and hCLEC9A strongly bound to late stage apoptotic MEFs, but not their live counterparts (Figure 8B). Pretreatment of apoptotic cells with proteases (trypsin, protease K), but not with nucleases, reduced Clec9A binding in a dose dependent manner, suggesting the ligand was a protein or protein-associated molecule (Figure 8C). The level of binding to dead cells was higher than any "nonspecific" binding seen with soluble forms of other C-type lectins tested, namely Cire (Figure 8A, B and C) and Clec12A (data not shown).

The possibility that cell membrane rupture was all that was required to reveal the ligand was tested by staining with Clec9A immediately after freezing and thawing cells, as a model of primary necrosis. Clec9A bound to frozen and thawed 3T3 cells (Figure 9A), to other frozen and thawed mouse cells, as well as to fixed and permeabilised cells and to frozen mouse tissue sections (data not shown). The binding included the exposed external surface of the dead cells, since it was also seen under fluorescence microscopy when fluorescent beads coated with mClec9A were used instead of soluble mClec9A (data not shown). Treatment of viable cells with trypsin prior to freeze-thawing did not eliminate Clec9A binding (data not shown), indicating the ligand was initially within the cells.

In summary, the data indicates that the ligand(s) for Clec9A are normally contained within viable cells, and are only exposed after membrane disruption, such as occurs in late apoptosis or necrosis.

### Binding to dead cells is via the C-type lectin domain

To investigate the requirements for Clec9A binding to dead cells, the shorter form of the soluble recombinant proteins (CTLD only) was compared to the soluble full length ectodomains (stalk + CTLD). The mClec9A-CTLD and hCLEC9A-CTLD were both monomeric, compared to the homodimeric Clec9A ectodomains, indicating the stalk region was required for homodimerisation (Figure 7). However, the mClec9A-CTLD and hCLEC9A-CTLD both showed similar levels of binding to dead cells as the full length ectodomains, indicating that the monomeric CTLD is sufficient for binding (Figure 9A). The mClec9A-ecto and mClec9A-CTLD showed similar levels of binding even when limiting dilutions of Clec9A were used, indicating that the ectodomain and CTLD bound with similarly to the dead cells.

Since binding was unaffected by EDTA (Figure 9B), divalent metal ions such as calcium are not required for binding.

### Species conservation of Clec9A binding to dead cells

As indicated in Figures 8 and 9A, both mouse and human Clec9A bound to dead mouse cells. Binding was seen to all dead mouse cell types tested, whether from cultured cell lines or freshly isolated cells. Mouse and human Clec9A also bound equally well to dead human cells (Figure 9B), as well as to hamster and monkey cells (data not shown). Binding was also seen to frozen and thawed insect cells, but not to bacteria or yeast (Figure 9C). Thus, recognition of dead cells by Clec9A is conserved across evolution and the ligand(s) are expressed by most animal and even insect cells.

### Does Clec9A mediate uptake of dead cells by DC?

Clec9A is expressed on splenic CD8⁺ DCs, and not on CD8⁻ DCs, as reported previously (Caminschi et al., 2008; Sancho et al., 2008) and confirmed in Figure 10A. CD8⁺ DCs have been reported previously to be more efficient at phagocytosis of dead cells (Iyoda et al., 2002; Schulz et al, 2002; Schnorrer et al., 2006). The inventors therefore investigated whether uptake of dead cells could be blocked using an excess of soluble Clec9A. As previously reported (Iyoda et al., 2002; Schulz and Sousa, 2002), the CD8⁺ DC were more efficient than their CD8⁻ counterparts at phagocytosis of dead splenocytes that had been labelled with the nuclear dye PI (Figure 10B). However, the addition of soluble mClec9A, whether the full ectodomain (mClec9A-ecto; Figure 10B) or CTLD only (data not shown) had no noticeable effect on CD8⁺ DC uptake of dead cells. Similar results were observed using dead splenocytes that had been labelled with the lipophilic membrane dye PKH26 (data not shown).

### Conclusions

Clec9A binds strongly to late apoptotic or necrotic cells; it recognises a component or components that are expressed by cells of diverse origins and tissue types, but are not accessible until the cell membrane is disrupted. Clec9A can therefore serve to distinguish early apoptotic from necrotic cells. This is considered to be an important distinction in the biology of DCs, as uptake of early apoptotic cells has been reported to promote an immunosuppressive environment to self-Ag whereas necrosis or failed clearance of apoptotic cells has been reported to promote immunogenic responses. Clec9A is selectively expressed on CD8⁺ DCs, which are specialised for the uptake and processing of Ag from dead cells, so it is likely to have a role in this process.

### Example 3 - Identification of the m5B6 ligand

CD8+DC ingest dead cells more efficiently than other DC types (Iyoda et al., 2002). m5B6, expressed on CD8+ DC, specifically binds dead cells, but not early stage apoptotic cells. Molecules used by CD8+ DC to differentiate between early stage apoptotic cells and necrotic cells are of prime importance because uptake of early stage apoptotic cells by DC induces tolerance, but uptake of necrotic cells induces immunity (Sauter et al., 2000). Thus differential recognition of these states by receptors on DC is crucial to the immune system. Importantly, only CD8+ DC are capable of inducing efficient CD8 T cell responses to exogenous Ag (Belz et al., 2004).

Some related C-type lectins have had their ligands identified, and some have multiple ligands (eg.LOX-1/Clec8a Dectin-1/Clec7a). The identity of 5B6 ligand(s) will be determined using a panel of immunochemical and proteomic techniques.

In a first approach, cells will be metabolically labelled using 35S, induce cell death, then incubate with soluble FLAG-tagged m5B6. Excess free Clec9A will be washed away and the cells incubated in the presence or absence of a chemical crosslinker. Cells will be lysed, and the complex affinity purified using either anti-FLAG M2 or anti-5B6-affinity resin. Bound proteins will be eluted with an excess of FLAG or 5B6 peptide.

In a complementary approach, a large batch of sol-5B6 will be purified and conjugated to NHS-activated Sepharose resin. Lysates from at least 5 x 10⁷ EL4 cells will be incubated with 5B6 affinity resin, and bound proteins eluted. Eluted proteins will be analysed by SDS-PAGE, transferred to PVDF membrane and visualised using a phosphorimager. To identify positive bands, this procedure will be scaled-up and eluates analysed by SDS-PAGE and Sypro Ruby/ Coomassie blue staining. Bands will be excised and proteins identified using mass-spectrometry. In brief, protein bands will be digested with trypsin (Moritz et al., 1996), separated by capillary chromatography (Moritz et al., 1992) and sequenced using an on-line electrospray ionisation ion-trap mass-spectrometer (Simpson et al., 2000).

In a further complementary approach, rats will be immunized with dead cells and perform a fusion to generate hybridomas by standard protocols. Hybridomas will be screened for Ab that bind to dead cells and block the binding of sol-5B6 as assayed by flow cytometry. The inventors will clone and purify the blocking Ab, and use it to immunoprecipitate the ligand to enable identification by mass-spectrometry.

Myc-tagged expression constructs for potential ligands will be generated, transiently transfected into 293 T cells and induce cell death, before incubation with 5B6+ DC or 5B6 transfectant cells. The inventors will lyse the cells, immunoprecipitate 5B6-complexes using anti-Clec9A mAb and analyse for coprecipitation of the myc-tagged ligand by Western blot. Alternatively, the inventors will perform direct Western blots of 5B6 complexes. In a complementary approach, the inventors will express potential candidates as recombinant soluble molecules and confirm their binding to 5B6 transfectants.

Antibodies which bind the 5B6 ligand will be generated using standard procedures.

### REFERENCES

Almeida and Allshire (2005) TRENDS Cell Biol 15: 251-258.
Al-Mufti et al (1999) Am. J. Med. Genet. 85:66-75.
Bauer et al (2002) Int J Legal Med 116:39-42.
Belz et al (2004). Proc Natl Acad Sci U S A 101:8670-8675.
Belz et al (2005) J Immunol 175, 196-200 (2005).
Binder et al (2004) Tissue Antigens 64, 442-51 (2004).
Bird et al (1988) Blood 80:1418-1422.
Bourque (1995) Plant Sci. 105:125-149.
Brown et al (1998) J Exp Med 188,2083-90.
Caminschi et al (2001) Mol Immunol 38:365-373.
Caminschi et al (2008) Blood 112:3264-3273.
Colcher et al (1986) Methods Enzymol. 121:802-816.
Delneste (2004) Biochem Soc Trans 32, 633-5.
Dunbrack et al (1997) Folding and Design, 2:R27-42.
Fuller et al (2007) J Biol Chem 282:12397-12409.
Galibert et al (2005) J Biol Chem 280:21955-21964.
Greenwood et al (1993) Eur J Immunol 23: 1098-1104.
Harayama (1998) Trends Biotech. 16:76-82.
Haseloff and Gerlach (1988) Nature 334:585-591.
Heath and Valladangos (2005) Seminars in Immunology 17: 262-272.
Henri et al (2001) J Immunol 167, 741-748 (2001).
Hochrein et al (2001) J Immunol 166:5448-5455.
Hoeffel et al (2007) Immunity 27, 481-92.
Hume (2008) Nat Immunol 9, 12-4.
Huston et al (1988) Proc Natl Acad Sci USA 85:5879-5883.
Iyoda et al (2002) J Exp Med 195, 1289-302.
Jones et al (1986) Nature 321:522-525.
Kenna et al (2008) Blood 111, 2091-100 (2008).
Lahoud et al (2006) J Immunol 177:372-382.
Leserman (2004) J Liposome Res 14:175-189.
Miller (1990) Blood 76:271-278.
Millar and Waterhouse (2005) Funct Integr Genomics 5:129-135.
Moritz et al (1992) J Chromatogr 599:119-130.
Moritz et al (1996) Electrophoresis 17:907-917.
Morrison et al (1984) Proc Natl Acad Sci USA 81:6851-6855.
Nagata (2007) Immunol Rev 220,237-50 (2007).
Naik et al (2006) Nat Immunol 7:663-671.
Needleman and Wunsch. (1970). J. Mol. Biol., 48, 443-453.
O'Keeffe et al (2002) J Exp Med 196:1307-1319.
O'Keeffe et al (2003) Blood 101:1453-1459.
Pasquinelli et al (2005) Curr Opin Genet Develop 15: 200-205.
Pastan et al (1986) Cell 47: 641-648.
Peng et al (2007) J Autoimmun 29:303-309.
Perriman et al (1992) Gene 113: 157-16.
Pyz et al (2008) Eur J Immunol 38:1157-1163.
Sancho et al (2008) J Clin Invest 118:2098-2110.
Sauter et al (2000) J Exp Med 191, 423-434.
Schnorrer et al (2006) Proc Natl Acad Sci U S A 103:10729-10734.
Schulz & Sousa (2002) Immunology 107:183-189.
Senior (1998) Biotech. Genet. Engin. Revs. 15:79-119.
Shippy et al (1999) Mol. Biotech. 12: 117-129.
Shortman and Naik (2007). Nat Rev Immunol 7:19-30.
Simpson et al (2000) Electrophoresis 21:1707-1732.
Smith et al (2000) Nature 407: 319-320.
Steinman et al (2000) J Exp Med 191,411-6.
Steinman et al (2003) Annu Rev Immunol 21:685-711.
Sun et al (1986) Hybridoma 5S1:517-520.
Thorpe et al (1987) Cancer Res 47: 5924-31.
van Broekhoven et al (2004) Cancer Res 64:4357-4365.
van Delft et al (2006) Cancer Cell 10, 389-99
Vandenabeele et al (2001) Blood 97:1733-1741.
Vitetta et al (1987) Science 238: 1098 -1104.
Vremec et al (2000) J Immunol 164:2978-2986.
Waldmann (1991) Science 252: 1657-1662.
Waterhouse et al (1998) Proc. Natl. Acad. Sci. USA 95: 13959-13964.
Weis et al (1998) Immunol Rev. 163:19-34.

## Claims

1. In vitro use of a compound for detecting a cell with a disrupted cell membrane, a cell infected with a pathogen, a dying cell or a dead cell or distinguishing between an early stage apoptotic cell and a late stage apoptotic cell, necrotic cell or dead cell, wherein
(1) the compound is a polypeptide which comprises:
i) an amino acid sequence as provided in any one of SEQ ID NO's 1 to 8;
ii) an amino acid sequence which is at least 50% identical to any one or more of SEQ ID NO's 1 to 8 and which is able to detect a cell with a disrupted cell membrane, a cell infected with a pathogen, a dying cell or a dead cell or distinguish between an early stage apoptotic cell and a late stage apoptotic cell, necrotic cell or dead cell; and/or
iii) a biologically active, soluble and/or antigenic fragment of i) or ii) and which is able to detect a cell with a disrupted cell membrane, a cell infected with a pathogen, a dying cell or a dead cell or distinguish between an early stage apoptotic cell and a late stage apoptotic cell, necrotic cell or dead cell, or
(2) the compound is an antibody or antigenic binding fragment thereof,
(A) wherein the antibody is a monoclonal antibody, humanized antibody, single chain antibody, diabody, triabody, or tetrabody, and/or
(B) wherein the antibody is 24/04-10B4, 42/04-42D2, 20/05-3A4 or 23/05-4C6, wherein antibodies 24/04-10B4, 42/04-42D2, 20/05-3A4 and 23/05-4C6 are produced by hydridoma cell lines deposited with the European Collection of Cell Cultures (ECACC) 24/04-10B4-24-8, 42/04-42D2-66-4-1, 20/05/3A4-26-16, 23/05-4C6-29-3 on 11 December 2007 under Deposit Reference Numbers 07121101, 07121102, 07121103, and 07121104 respectively, or by higher antibody secreting subclones of the hybridomas (24/04-10B4-24-8-FACS 9-5, 42/04-42D2-66-4-1-Clone 4, 20/05-3A4-26-16-Clone 5, 23/05-4C6-29-3-Clone 5) deposited with the ECACC on 29 April 2008, and designated Accession numbers 08042901, 08042902, 08042903, and 08042904, respectively.

2. In vitro use of a compound for diagnosing, prognosing and/or monitoring the status of a disease associated with cells with a disrupted cell membrane, cells infected with a pathogen, dying cells or dead cells, wherein
(1) the compound is a polypeptide which comprises:
i) an amino acid sequence as provided in any one of SEQ ID NO's 1 to 8;
ii) an amino acid sequence which is at least 50% identical to any one or more of SEQ ID NO's 1 to 8 and which is able to detect a cell with a disrupted cell membrane, cells infected with a pathogen, dying cells or dead cells; and/or
iii) a biologically active, soluble and/or antigenic fragment of i) or ii) and which is able to detect a cell with a disrupted cell membrane, cells infected with a pathogen, dying cells or dead cells, or
(2) the compound is an antibody or antigenic binding fragment thereof,
(A) wherein the antibody is a monoclonal antibody, humanized antibody, single chain antibody, diabody, triabody, or tetrabody, and/or
(B) wherein the antibody is 24/04-10B4, 42/04-42D2, 20/05-3A4 or 23/05-4C6, wherein antibodies 24/04-10B4, 42/04-42D2, 20/05-3A4 and 23/05-4C6 are produced by hydridoma cell lines deposited with the European Collection of Cell Cultures (ECACC) 24/04-10B4-24-8, 42/04-42D2-66-4-1, 20/05/3A4-26-16, 23/05-4C6-29-3 on 11 December 2007 under Deposit Reference Numbers 07121101, 07121102, 07121103, and 07121104 respectively, or by higher antibody secreting subclones of the hybridomas (24/04-10B4-24-8-FACS 9-5, 42/04-42D2-66-4-1-Clone 4, 20/05-3A4-26-16-Clone 5, 23/05-4C6-29-3-Clone 5) deposited with the ECACC on 29 April 2008, and designated Accession numbers 08042901, 08042902, 08042903, and 08042904, respectively.

3. The use according to claim 1 or 2, wherein the compound is detectably labelled.

4. The use according to claim 2 or 3, wherein the disease is selected from: graft versus host disease (GVHD), an autoimmune disease, an infection, a neurodegenerative disease, systemic inflammatory reaction syndrome (SIRS), cancer and injury.

## Patentansprüche

1. In vitro-Verwendung einer Verbindung zum Nachweisen einer Zelle mit einer aufgebrochenen Zellmembran, einer mit einem Pathogen infizierten Zelle, einer sterbenden Zelle oder einer toten Zelle oder Unterscheiden zwischen einer apoptotischen Zelle früher Stufe und einer apoptotischen Zelle später Stufe, nekrotischer Zelle oder toter Zelle, wobei
(1) die Verbindung ein Polypeptid ist, welches umfasst:
i) eine Aminosäuresequenz, wie in einer von SEQ ID NRn. 1 bis 8 bereitgestellt;
ii) eine Aminosäuresequenz, welche mindestens 50% mit einer oder mehreren von SEQ ID NRn. 1 bis 8 identisch ist und welche eine Zelle mit einer aufgebrochenen Zellmembran, eine mit einem Pathogen infizierten Zelle, eine sterbende Zelle oder eine tote Zelle nachweisen oder zwischen einer apoptotischen Zelle früher Stufe und einer apoptotischen Zelle später Stufe, nekrotischer Zelle oder toter Zelle unterscheiden kann; und/oder
iii) ein biologisch aktives, lösliches und/oder antigenes Fragment von i) oder ii) und welches eine Zelle mit einer aufgebrochenen Zellmembran, eine mit einem Pathogen infizierte Zelle, eine sterbende Zelle oder eine tote Zelle nachweisen oder zwischen einer apoptotischen Zelle früher Stufe und einer apoptotischen Zelle später Stufe, nekrotischer Zelle oder toter Zelle unterscheiden kann, oder
(2) die Verbindung ein Antikörper oder Antigen bindendes Fragment davon ist,
(A) wobei der Antikörper ein monoklonaler Antikörper, humanisierter Antikörper, einkettiger Antikörper, Diabody, Triabody oder Tetrabody ist, und/oder
(B) wobei der Antikörper 24/04-10B4, 42/04-42D2, 20/05-3A4 oder 23/05-4C6 ist, wobei Antikörper 24/04-10B4, 42/04-42D2, 20/05-3A4 und 23/05-4C6 durch Hybridom-Zelllinien erzeugt werden, hinterlegt bei der European Collection of Cell Cultures (ECACC) 24/04-10B4-24-8, 42/04-42D2-66-4-1, 20/05/3A4-26-16, 23/05-4C6-29-3 am 11. Dezember 2007 unter Hinterlegungs-Bezugsnummern 07121101, 07121102, 07121103 bzw. 07121104 oder durch höhere Antikörper sekretierende Subklone der Hybridoma (24/04-10B4-24-8-FACS 9-5, 42/04-42D2-66-4-1-Klon 4, 20/05-3A4-26-16-Klon 5, 23/05-4C6-29-3-Klon 5) hinterlegt bei der ECACC am 29. April 2008, und bezeichnet mit Zugangsnummern 08042901, 08042902, 08042903 bzw. 08042904.

2. In vitro-Verwendung einer Verbindung zum Diagnostizieren, Prognostizieren und/oder Verfolgen des Status einer Krankheit, verbunden mit Zellen mit einer aufgebrochenen Zellmembran, mit einem Pathogen infizierten Zellen, sterbenden Zellen oder toten Zellen, wobei
(1) die Verbindung ein Polypeptid ist, welches umfasst:
i) eine Aminosäuresequenz, wie in einer von SEQ ID NRn. 1 bis 8 bereitgestellt;
ii) eine Aminosäuresequenz, welche mindestens 50% mit einer oder mehreren von SEQ ID NRn. 1 bis 8 identisch ist und welche eine Zelle mit einer aufgebrochenen Zellmembran, mit einem Pathogen infizierte Zellen, sterbende Zellen oder tote Zellen nachweisen kann; und/oder
iii) ein biologisch aktives, lösliches und/oder antigenes Fragment von i) oder ii) und welches eine Zelle mit einer aufgebrochenen Zellmembran, mit einem Pathogen infizierte Zellen, sterbende Zellen oder tote Zellen nachweisen kann, oder
(2) die Verbindung ein Antikörper oder Antigen bindendes Fragment davon ist,
(A) wobei der Antikörper ein monoklonaler Antikörper, humanisierter Antikörper, einkettiger Antikörper, Diabody, Triabody oder Tetrabody ist, und/oder
(B) wobei der Antikörper 24/04-10B4, 42/04-42D2, 20/05-3A4 oder 23/05-4C6 ist, wobei Antikörper 24/04-10B4, 42/04-42D2, 20/05-3A4 und 23/05-4C6 durch Hybridom-Zelllinien erzeugt werden, hinterlegt bei der European Collection of Cell Cultures (ECACC) 24/04-10B4-24-8, 42/04-42D2-66-4-1, 20/05/3A4-26-16, 23/05-4C6-29-3 am 11. Dezember 2007 unter Hinterlegungs-Bezugsnummern 07121101, 07121102, 07121103 bzw. 07121104, oder durch höhere Antikörper sekretierende Subklone der Hybridoma (24/04-10B4-24-8-FACS 9-5, 42/04-42D2-66-4-1-Klon 4, 20/05-3A4-26-16-Klon 5, 23/05-4C6-29-3-Klon 5), hinterlegt bei der ECACC am 29. April 2008, und bezeichnet mit Zugangsnummern 08042901, 08042902, 08042903 bzw. 08042904.

3. Verwendung nach Anspruch 1 oder 2, wobei die Verbindung nachweisbar markiert ist.

4. Verwendung nach Anspruch 2 oder 3, wobei die Krankheiten ausgewählt sind aus: Transplantat-gegen-Wirts-Krankheit (GVHD), einer Autoimmunkrankheit, einer Infektion, einer neurodegenerativen Krankheit, systemischem Entzündungsreaktionssyndrom (SIRS), Krebs und einer Verletzung.

## Revendications

1. Utilisation in vitro d'un composé pour détecter une cellule avec une membrane cellulaire perturbée, une cellule infectée par un pathogène, une cellule mourante ou une cellule morte ou faire la distinction entre une cellule apoptotique à un stade précoce et une cellule apoptotique à un stade tardif, une cellule nécrotique ou une cellule morte, dans laquelle
(1) le composé est un polypeptide qui comprend :
i) une séquence d'acide aminé telle que prévue dans l'une quelconque des SEQ ID NO's 1 à 8 ;
ii) une séquence d'acide aminé qui est au moins 50 % identique à l'une quelconque ou plusieurs des SEQ ID NO's 1 à 8 et qui est capable de détecter une cellule avec une membrane cellulaire perturbée, une cellule infectée par un pathogène, une cellule mourante ou une cellule morte ou faire la distinction entre une cellule apoptotique à un stade précoce et une cellule apoptotique à un stade tardif, une cellule nécrotique ou une cellule morte ; et/ou
iii) un fragment biologiquement actif, soluble et/ou antigénique de i) ou ii) et qui est capable de détecter une cellule avec une membrane cellulaire perturbée, une cellule infectée par un pathogène, une cellule mourante ou une cellule morte ou faire la distinction entre une cellule apoptotique à un stade précoce et une cellule apoptotique à un stade tardif, une cellule nécrotique ou une cellule morte, ou
(2) le composé est un anticorps ou un fragment de liaison antigénique de celui-ci,
(A) dans lequel l'anticorps est un anticorps monoclonal, un anticorps humanisé, un anticorps à chaine unique, un diabody, un triabody, ou un tétrabody, et/ou
(B) dans lequel l'anticorps est 24/04-10B4, 42/04-42D2, 20/05-3A4 ou 23/05-4C6, dans lequel les anticorps 24/04-10B4, 42/04-42D2, 20/05-3A4 et 23/05-4C6 sont produits par des lignées cellulaires d'hybridomes déposées avec la collection européenne de cultures cellulaires (ECACC) 24/04-10B4-24-8, 42/04-42D2-66-4-1, 20/05/3A4-26-16, 23/05-4C6-29-3 le 11 décembre 2007 respectivement sous les numéros de référence de dépôt 07121101, 07121102, 07121103, et 07121104, ou par des sous-clones de sécrétion d'anticorps supérieurs des hybridomes (24/04-10B4-24-8-FACS 9-5, 42/04-42D2-66-4-1-Clone 4, 20/05-3A4-26-16-Clone 5, 23/05-4C6-29-3-Clone 5) déposés avec la ECACC le 29 avril 2008, et respectivement les numéros d'accès désignés 08042901, 08042902, 08042903, et 08042904.

2. Utilisation in vitro d'un composé pour diagnostiquer, pronostiquer et/ou surveiller le statut d'une maladie associée avec des cellules avec une membrane cellulaire perturbée, des cellules infectées par un pathogène, des cellules mourantes ou des cellules mortes, dans laquelle
(1) le composé est un polypeptide qui comprend :
i) une séquence d'acide aminé telle que prévue dans l'une quelconque des SEQ ID NO's 1 à 8 ;
ii) une séquence d'acide aminé qui est au moins 50 % identique à l'une quelconque ou plusieurs des SEQ ID NO's 1 à 8 et qui est capable de détecter une cellule avec une membrane cellulaire perturbée, des cellules infectées par un pathogène, des cellules mourantes ou des cellules mortes ; et/ou
iii) un fragment biologiquement actif, soluble et/ou antigénique de i) ou ii) et qui est capable de détecter une cellule avec une membrane cellulaire perturbée, des cellules infectées par un pathogène, des cellules mourantes ou des cellules mortes, ou
(2) le composé est un anticorps ou un fragment de liaison antigénique de celui-ci,
(A) dans lequel l'anticorps est un anticorps monoclonal, un anticorps humanisé, un anticorps à chaine unique, un diabody, un triabody, ou un tétrabody, et/ou
(B) dans lequel l'anticorps est 24/04-10B4, 42/04-42D2, 20/05-3A4 ou 23/05-4C6, dans lequel les anticorps 24/04-10B4, 42/04-42D2, 20/05-3A4 et 23/05-4C6 sont produits par des lignées cellulaires d'hybridomes déposées avec la collection européenne de cultures cellulaires (ECACC) 24/04-10B4-24-8, 42/04-42D2-66-4-1, 20/05/3A4-26-16, 23/05-4C6-29-3 le 11 décembre 2007 respectivement sous les numéros de référence de dépôt 07121101, 07121102, 07121103, et 07121104, ou par des sous-clones de sécrétion d'anticorps supérieurs des hybridomes (24/04-10B4-24-8-FACS 9-5, 42/04-42D2-66-4-1-Clone 4, 20/05-3A4-26-16-Clone 5, 23/05-4C6-29-3-Clone 5) déposés avec la ECACC le 29 avril 2008, et respectivement les numéros d'accès désignés 08042901, 08042902, 08042903, et 08042904.

3. Utilisation selon la revendication 1 ou 2, dans laquelle le composé est marqué de manière détectable.

4. Utilisation selon la revendication 2 ou 3, dans laquelle la maladie est sélectionnée parmi : la maladie du greffon contre l'hôte (GVHD), une maladie auto-immune, une infection, une maladie neurodégénérative, un syndrome de réaction inflammatoire systémique (SIRS), un cancer et une lésion.


### SEQUENCE LISTING

<110> Walter and Eliza Hall Institute of Medical Research
   The Burnet Institute
<120> Methods of detecting cells with a disrupted cell membrane, cells
   infected with a pathogen, dying cells or dead cells
<130> 508096
<150> US 61/052,983
   <151> 2008-05-13
<150> US 61/120,801
   <151> 2008-12-08
<160> 47
<170> PatentIn version 3.5
<210> 1
   <211> 241
   <212> PRT
   <213> Homo sapiens
<400> 1
   Met His Glu Glu Glu Ile Tyr Thr Ser Leu Gln Trp Asp Ser Pro Ala
   1               5                   10                  15
   Pro Asp Thr Tyr Gln Lys Cys Leu Ser Ser Asn Lys Cys Ser Gly Ala
               20                  25                  30
   Cys Cys Leu Val Met Val Ile Ser Cys Val Phe Cys Met Gly Leu Leu
           35                  40                  45
   Thr Ala Ser Ile Phe Leu Gly Val Lys Leu Leu Gln Val Ser Thr Ile
       50                  55                  60
   Ala Met Gln Gln Gln Glu Lys Leu Ile Gln Gln Glu Arg Ala Leu Leu
   65                  70                  75                  80
   Asn Phe Thr Glu Trp Lys Arg Ser Cys Ala Leu Gln Met Lys Tyr Cys
                   85                  90                  95
   Gln Ala Phe Met Gln Asn Ser Leu Ser Ser Ala His Asn Ser Ser Pro
               100                 105                 110
   Cys Pro Asn Asn Trp Ile Gln Asn Arg Glu Ser Cys Tyr Tyr Val Ser
           115                 120                 125
   Glu Ile Trp Ser Ile Trp His Thr Ser Gln Glu Asn Cys Leu Lys Glu
       130                 135                 140
   Gly Ser Thr Leu Leu Gln Ile Glu Ser Lys Glu Glu Met Asp Phe Ile
   145                 150                 155                 160
   Thr Gly Ser Leu Arg Lys Ile Lys Gly Ser Tyr Asp Tyr Trp Val Gly
                   165                 170                 175
   Leu Ser Gln Asp Gly His Ser Gly Arg Trp Leu Trp Gln Asp Gly Ser
               180                 185                 190
   Ser Pro Ser Pro Gly Leu Leu Pro Ala Glu Arg Ser Gln Ser Ala Asn
           195                 200                 205
   Gln Val Cys Gly Tyr Val Lys Ser Asn Ser Leu Leu Ser Ser Asn Cys
       210                 215                 220
   Ser Thr Trp Lys Tyr Phe Ile Cys Glu Lys Tyr Ala Leu Arg Ser Ser
   225                 230                 235                 240
   Val
<210> 2
   <211> 264
   <212> PRT
   <213> Mus musculus
<400> 2
   Met His Ala Glu Glu Ile Tyr Thr Ser Leu Gln Trp Asp Ile Pro Thr
   1 5 10 15
   Ser Glu Ala Ser Gln Lys Cys Gln Ser Pro Ser Lys Cys Ser Gly Ala
   20 25 30
   Trp Cys Val Val Thr Met Ile Ser Cys Val Val Cys Met Gly Leu Leu
   35 40 45
   Ala Thr Ser Ile Phe Leu Gly Ile Lys Phe Phe Gln Val Ser Ser Leu
   50 55 60
   Val Leu Glu Gln Gln Glu Arg Leu Ile Gln Gln Asp Thr Ala Leu Val
   65 70 75 80
   Asn Leu Thr Gln Trp Gln Arg Lys Tyr Thr Leu Glu Tyr Cys Gln Ala
   85 90 95
   Leu Leu Gln Arg Ser Leu His Ser Gly Thr Asp Ala Ser Thr Gly Pro
   100 105 110
   Val Leu Leu Thr Ser Pro Gln Met Val Pro Gln Thr Leu Asp Ser Lys
   115 120 125
   Glu Thr Gly Ser Asp Cys Ser Pro Cys Pro His Asn Trp Ile Gln Asn
   130 135 140
   Gly Lys Ser Cys Tyr Tyr Val Phe Glu Arg Trp Glu Met Trp Asn Ile
   145 150 155 160
   Ser Lys Lys Ser Cys Leu Lys Glu Gly Ala Ser Leu Phe Gln Ile Asp
   165 170 175
   Ser Lys Glu Glu Met Glu Phe Ile Ser Ser Ile Gly Lys Leu Lys Gly
   180 185 190
   Gly Asn Lys Tyr Trp Val Gly Val Phe Gln Asp Gly Ile Ser Gly Ser
   195 200 205
   Trp Phe Trp Glu Asp Gly Ser Ser Pro Leu Ser Asp Leu Leu Pro Ala
   210 215 220
   Glu Arg Gln Arg Ser Ala Gly Gln Ile Cys Gly Tyr Leu Lys Asp Ser
   225 230 235 240
   Thr Leu Ile Ser Asp Lys Cys Asp Ser Trp Lys Tyr Phe Ile Cys Glu
   245 250 255
   Lys Lys Ala Phe Gly Ser Cys Ile
   260
<210> 3
   <211> 241
   <212> PRT
   <213> Pan troglodytes
<400> 3
   Met His Glu Glu Glu Ile Tyr Thr Ser Leu Gln Trp Asp Ser Pro Ala
   1               5                   10                  15
   Pro Asp Thr Tyr Gln Lys Cys Leu Ser Ser Asn Lys Cys Ser Gly Ala
               20                  25                  30
   Cys Cys Leu Val Met Val Ile Ser Cys Val Phe Cys Met Gly Leu Leu
           35                  40                  45
   Thr Ala Ser Ile Phe Leu Gly Val Lys Leu Leu Gln Val Ser Thr Ile
       50                  55                  60
   Ala Met Gln Gln Gln Glu Lys Leu Ile Gln Gln Glu Arg Ala Leu Leu
   65                  70                  75                  80
   Asn Phe Thr Glu Trp Lys Arg Ser Cys Ala Leu Gln Met Lys Tyr Cys
                   85                  90                  95
   Gln Ala Phe Met Gln Asn Ser Leu Ser Ser Ala His Asn Ser Ser Pro
               100                 105                 110
   Cys Pro Asn Ser Trp Ile Gln Asn Arg Glu Ser Cys Tyr Tyr Val Ser
           115                 120                 125
   Glu Ile Trp Ser Ile Trp His Thr Ser Gln Glu Asn Cys Leu Lys Glu
       130                 135                 140
   Gly Ser Thr Leu Leu Gln Ile Glu Ser Lys Glu Glu Met Asp Phe Ile
   145                 150                 155                 160
   Thr Gly Ser Leu Arg Lys Ile Lys Gly Ser Tyr Asp Tyr Trp Val Gly
                   165                 170                 175
   Leu Ser Gln Asp Gly His Ser Gly Arg Trp Leu Trp Gln Asp Gly Ser
               180                 185                 190
   Ser Pro Ser Pro Gly Leu Leu Pro Val Glu Arg Ser Gln Ser Ala Asn
           195                 200                 205
   Gln Val Cys Gly Tyr Met Lys Ser Asn Ser Leu Leu Ser Ser Asn Cys
       210                 215                 220
   Ser Thr Trp Lys Tyr Phe Ile Cys Glu Lys Tyr Ala Leu Arg Ser Ser
   225                 230                 235                 240
   Val
<210> 4
   <211> 241
   <212> PRT
   <213> Macaca mulatta
<400> 4
   Met His Glu Glu Glu Ile Tyr Thr Ser Leu Gln Trp Asp Ser Pro Ala
   1               5                   10                  15
   Pro Asn Thr Tyr Gln Lys Cys Leu Ser Ser Asn Lys Cys Ser Gly Ala
               20                  25                  30
   Trp Cys Leu Val Met Ala Ile Ser Cys Ile Phe Cys Met Gly Leu Leu
           35                  40                  45
   Thr Ala Ser Ile Phe Leu Gly Val Lys Leu Leu Gln Val Ser Thr Ile
       50                  55                  60
   Ala Met Gln Gln Gln Glu Lys Leu Ile Gln Gln Glu Arg Ala Leu Leu
   65                  70                  75                  80
   Asn Phe Thr Glu Trp Lys Arg Ser His Val Leu Gln Met Lys Phe Cys
                   85                  90                  95
   Gln Thr Phe Met Gln Ser Ser Phe Ser Ser Ala His Asn Cys Ser Pro
               100                 105                 110
   Cys Pro Asn Asn Trp Ile Gln Asn Arg Glu Ser Cys Tyr Tyr Val Ser
           115                 120                 125
   Glu His Trp Lys Ile Trp His Thr Ser Gln Glu Asn Cys Leu Lys Glu
       130                 135                 140
   Gly Ser Thr Leu Leu Gln Ile Glu Ser Glu Glu Glu Met Asp Phe Ile
   145                 150                 155                 160
   Thr Gly Ser Leu Arg Lys Ile Arg Gly Ser Tyr Asp Tyr Trp Val Gly
                   165                 170                 175
   Leu Ser Gln Asp Gly His Ser Gly Arg Trp Leu Trp Gln Asp Gly Ser
               180                 185                 190
   Ser Pro Ser Pro Gly Leu Leu Pro Val Glu Ile Ser Gln Ser Thr Asn
           195                 200                 205
   Gln Val Cys Gly Tyr Ile Lys Asn Ser Ser Leu Leu Ser Ser Asn Cys
       210                 215                 220
   Ser Thr Trp Lys Tyr Phe Ile Cys Glu Lys Tyr Ala Leu Arg Ser Ser
   225                 230                 235                 240
   Val
<210> 5
   <211> 241
   <212> PRT
   <213> Canis familiaris
<400> 5
   Met Gln Glu Glu Glu Thr Tyr Thr Ser Leu Arg Trp Asp Ser Pro Thr
   1               5                   10                  15
   Pro Ser Phe Tyr Gln Lys His Leu Ser Ser Thr Lys Tyr Ser Gly Ala
               20                  25                  30
   Trp Cys Leu Val Thr Val Ile Thr Cys Ile Leu Cys Val Gly Ser Ile
           35                  40                  45
   Ala Thr Ser Val Phe Leu Gly Leu Lys Leu Phe Gln Val Ser Thr Ile
       50                  55                  60
   Ala Met Lys Gln Arg Glu Lys Leu Ile Leu Gln Asp Arg Ala Leu Leu
   65                  70                  75                  80
   Asn Phe Thr Gln Trp Glu Arg Asn His Asn Leu Gln Met Lys Tyr Cys
                   85                  90                  95
   Gln Thr Leu Met Gln Asn Ser Phe Ser Ser Ala His Asn Cys Ser Pro
               100                 105                 110
   Cys Pro Asp Asn Trp Ile Gln Asn Gly Glu Ser Cys Tyr His Val Phe
           115                 120                 125
   Glu Asn Trp Lys Ile Trp His Thr Ser Lys Glu Asp Cys Leu Lys Glu
       130                 135                 140
   Gly Ser Asn Leu Leu Gln Ile Asp Ser Lys Glu Glu Met Asp Phe Ile
   145                 150                 155                 160
   Thr Gly Ser Leu Lys Lys Val Lys Ser Gly Phe Asp Tyr Trp Val Gly
                   165                 170                 175
   Leu Ser Gln Asp Gly Leu Ser Lys Pro Trp Leu Trp Gln Asp Gly Ser
               180                 185                 190
   Ser Pro Ser Pro Asp Leu Ser Pro Val Gln Thr Leu Gln Ser Thr Asn
           195                 200                 205
   Gln Leu Cys Gly Tyr Leu Lys Asp Lys Phe Leu Ser Ser Ala Asn Cys
       210                 215                 220
   Ser Ile Trp Lys Tyr Phe Ile Cys Glu Lys Tyr Ala Leu Arg Ser Ser
   225                 230                 235                 240
   Asn
<210> 6
   <211> 241
   <212> PRT
   <213> Bos taurus
<400> 6
   Met Gln Glu Asp Glu Ile Tyr Thr Ser Leu Gln Trp Asp Thr Pro Thr
   1 5 10 15
   Ser Asn Pro Tyr Gln Lys His Leu Ser Ser Thr Lys Asn Ser Gly Val
   20 25 30
   Trp Cys Leu Val Met Val Ile Leu Cys Ile Phe Cys Ile Gly Ser Leu
   35 40 45
   Ala Thr Ser Ile Phe Leu Gly Ile Lys Leu Phe Gln Met Ser Thr Thr
   50 55 60
   Ile Met Lys Gln Gln Glu Lys Leu Ile Gln Gln Glu Arg Ala Leu Leu
   65 70 75 80
   Asn Phe Thr Gln Trp Lys Arg Asn Pro Asn Leu Gln Met Thr Tyr Cys
   85 90 95
   Gln Thr Leu Met Gln Lys Ser Leu Ser Ser Ala Tyr Asn Cys Ser Pro
   100 105 110
   Cys Pro Asp Asn Trp Ile Gln Asn Gly Glu Ser Cys Tyr His Val Phe
   115 120 125
   Glu Ser Trp Thr Phe Trp His Thr Ser Arg Lys Asp Cys Trp Lys Lys
   130 135 140
   Gly Ser Asp Leu Leu Gln Ile Glu Ser Lys Glu Glu Met Asp Phe Ile
   145 150 155 160
   Thr Gly Ser Leu Lys Lys Ile Lys Arg Asn Tyr Asp Tyr Trp Val Gly
   165 170 175
   Leu Ser Gln Asn Gly Ser Asn Gln Pro Trp Leu Trp Gln Asp Gly Ser
   180 185 190
   Ser Pro Ser Ala Asp Leu Leu Pro Arg Gln Gly Pro Gln Ser Thr Asn
   195 200 205
   Gln Val Cys Gly Tyr Leu Arg Asp Asn Asp Leu Ser Ser Ala Asn Cys
   210 215 220
   Ser Val Trp Lys Tyr Phe Ile Cys Glu Lys Tyr Ala Leu Arg Ser Ser
   225 230 235 240
   Thr
<210> 7
   <211> 240
   <212> PRT
   <213> Equus caballus
<400> 7
   Met Gln Glu Glu Glu Met Tyr Thr Ser Leu Gln Trp Asp Asn Pro Thr
   1 5 10 15
   Ser Asn Pro Tyr Gln Lys Asn Leu Pro Ser Lys Cys Ser Gly Thr Arg
   20 25 30
   Cys Leu Val Ile Val Ile Ser Cys Ile Phe Cys Met Gly Leu Leu Thr
   35 40 45
   Thr Ser Ile Phe Leu Gly Ile Lys Leu Phe Gln Val Ser Ala Ile Ala
   50 55 60
   Val Lys Gln Gln Glu Lys Leu Ile Gln Gln Glu Arg Thr Leu Leu Asn
   65 70 75 80
   Phe Thr Gln Cys Asn Arg Asn His Asp Phe Gln Met Lys Cys Cys Gln
   85 90 95
   Ile Leu Met Lys Asn Ser Leu Asn Ser Ala His His Cys Ser Pro Cys
   100 105 110
   Pro Asp Asn Trp Ile Gln Asn Gly Glu Ser Cys Tyr Tyr Val Phe Glu
   115 120 125
   Asn His Lys Thr Trp His Thr Ser Lys Gln Val Cys Leu Lys Glu Gly
   130 135 140
   Ser Asn Leu Leu Gln Ile Asp Asn Lys Glu Glu Met Asp Phe Ile Thr
   145 150 155 160
   Gly Ser Leu Lys Arg Ile Lys Ser Ser Tyr Asp Tyr Trp Val Gly Leu
   165 170 175
   Ser Gln Asp Gly Leu Ser Gly Pro Trp Leu Trp Gln Asp Gly Ser Ser
   180 185 190
   Leu Ser Pro Asp Leu Trp Pro Val Gln Arg Pro Gln Ser Pro Asn Leu
   195 200 205
   Val Cys Gly Tyr Leu Lys Asn Lys Ile Leu Phe Ser Ala Asn Cys Ser
   210 215 220
   Ser Trp Lys His Phe Ile Cys Glu Lys Tyr Ala Leu Arg Ser Cys Ile
   225 230 235 240
<210> 8
   <211> 241
   <212> PRT
   <213> Rattus norvegicus
<400> 8
   Met His Glu Glu Glu Ile Tyr Thr Ser Leu Gln Trp Asp Ile Pro Thr
   1               5                   10                  15
   Ser Glu Ala Ser Gln Lys Cys Pro Ser Leu Ser Lys Cys Pro Gly Thr
               20                  25                  30
   Trp Cys Ile Val Thr Val Ile Ser Cys Val Val Cys Val Gly Leu Leu
           35                  40                  45
   Ala Ala Ser Ile Phe Leu Gly Ile Lys Phe Ser Gln Val Ser Ser Leu
       50                  55                  60
   Val Met Glu Gln Arg Glu Arg Leu Ile Arg Gln Asp Thr Ala Leu Leu
   65                  70                  75                  80
   Asn Leu Thr Glu Trp Gln Arg Asn His Thr Leu Gln Leu Lys Ser Cys
                   85                  90                  95
   Gln Ala Ser Leu Gln Arg Ser Leu Arg Ser Gly Ser Asn Cys Asn Pro
               100                 105                 110
   Cys Pro Pro Asn Trp Ile Gln Asn Gly Lys Ser Cys Tyr Tyr Ala Phe
           115                 120                 125
   Asp Arg Trp Glu Thr Trp Asn Asn Ser Lys Lys Ser Cys Leu Lys Glu
       130                 135                 140
   Gly Asp Ser Leu Leu Gln Ile Asp Ser Lys Glu Glu Met Glu Phe Ile
   145                 150                 155                 160
   Asn Leu Ser Ile Trp Lys Leu Lys Gly Gly Tyr Glu Tyr Trp Val Gly
                   165                 170                 175
   Val Phe Gln Asp Gly Pro Ser Gly Ser Trp Phe Trp Glu Asp Gly Ser
               180                 185                 190
   Ser Pro Leu Ser Asp Leu Leu Pro Thr Asp Arg Gln Leu Ser Ala Ser
           195                 200                 205
   Gln Ile Cys Gly Tyr Leu Lys Asp His Thr Leu Ile Ser Asp Asn Cys
       210                 215                 220
   Ser Asn Trp Lys Tyr Phe Ile Cys Glu Lys Lys Ala Phe Gly Ser Cys
   225                 230                 235                 240
   Ile
<210> 9
   <211> 726
   <212> DNA
   <213> Homo sapiens
<400> 9
   atgcacgagg aagaaatata cacctctctt cagtgggata gcccagcacc agacacttac 60
   cagaaatgtc tgtcttccaa caaatgttca ggagcatgct gtcttgtgat ggtgatttca 120
   tgtgttttct gcatgggatt attaacagca tccattttct tgggcgtcaa gttgttgcag 180
   gtgtccacca ttgcgatgca gcagcaagaa aaactcatcc aacaagagag ggcactgcta 240
   aactttacag aatggaagag aagctgtgcc cttcagatga aatattgcca agccttcatg 300
   caaaactcat taagttcagc ccataacagc agtccttgtc caaacaattg gattcagaac 360
   agagaaagtt gttactatgt ctctgaaatt tggagcattt ggcacaccag tcaagagaat 420
   tgtttaaagg aaggttccac gctgctacaa atagagagca aagaagaaat ggattttatc 480
   actggcagct tgaggaagat taaaggaagc tatgattact gggtggggtt gtctcaggat 540
   ggacacagcg gacgctggct ttggcaagat ggctcctctc cttctcctgg cctgttgcca 600
   gcagagagat cccagtcagc taaccaagtc tgtggatacg tgaaaagcaa ttcccttctt 660
   tcgtctaact gcagcacgtg gaagtatttt atctgtgaga agtatgcgtt gagatcctct 720
   gtctga 726
<210> 10
   <211> 795
   <212> DNA
   <213> Mus musculus
<400> 10
   atgcatgcgg aagaaatata tacctctctt cagtgggaca ttcctacctc agaggcctct 60
   cagaagtgcc aatcccctag caaatgttca ggagcatggt gtgttgtgac gatgatttcc 120
   tgtgtggtct gtatgggctt gttagcaacg tccattttct tgggcatcaa gttcttccag 180
   gtatcctctc ttgtcttgga gcagcaggaa agactcatcc aacaggacac agcattggtg 240
   aaccttacac agtggcagag gaaatacaca ctggaatact gccaagcctt actgcagaga 300
   tctctccatt caggcacaga tgcttctact ggaccagttc ttctgacctc tccacagatg 360
   gttccacaga ccctggacag caaggaaaca ggtagtgact gcagcccttg tccacacaac 420
   tggattcaga atggaaaaag ttgttactat gtctttgaac gctgggaaat gtggaacatc 480
   agtaagaaga gctgtttaaa agagggcgct agtctctttc aaatagacag caaagaagaa 540
   atggagttca tcagcagtat agggaaactc aaaggaggaa ataaatattg ggtgggagtg 600
   tttcaagatg gaatcagtgg atcttggttc tgggaagatg gctcttctcc tctctctgac 660
   ttgttgccag cagaaagaca gcgatcagcc ggccagatct gtggatacct caaagattct 720
   actctcatct cagataagtg cgatagctgg aaatatttta tctgtgagaa gaaggcattt 780
   ggatcctgca tctga 795
<210> 11
   <211> 726
   <212> DNA
   <213> Pan troglodytes
<400> 11
   atgcacgagg aagaaatata cacctctctt cagtgggata gcccagcacc agacacttac 60
   cagaaatgtc tgtcttccaa caaatgttca ggagcatgct gtcttgtgat ggtgatttca 120
   tgtgttttct gcatgggatt attaacagca tccattttct tgggcgtcaa gttgttgcag 180
   gtgtccacca ttgcgatgca gcagcaagaa aaactcatcc aacaagagag ggcactgcta 240
   aactttacag aatggaagag aagctgtgcc cttcagatga aatattgcca agccttcatg 300
   caaaactcat taagttcagc ccataacagc agtccttgtc caaacagttg gattcagaac 360
   agagaaagtt gttactatgt ctctgaaatt tggagcattt ggcacaccag tcaagagaat 420
   tgtttaaagg aaggttccac gctgctacaa atagagagca aagaagaaat ggattttatc 480
   actggcagtt tgagaaagat taaaggaagc tatgattact gggtggggtt gtctcaggat 540
   ggacacagcg gacgctggct ttggcaagat ggctcctctc cttctcctgg cctgttgcca 600
   gtagagagat cccagtcagc taaccaagtc tgtggataca tgaaaagcaa ttcccttctt 660
   tcgtctaact gcagcacttg gaagtatttt atctgtgaga agtatgcgtt gagatcctct 720
   gtctga 726
<210> 12
   <211> 726
   <212> DNA
   <213> Macaca mulatta
<400> 12
   atgcatgagg aagaaatata cacctctctt cagtgggata gtccagcacc aaacacttac 60
   cagaaatgtc tgtcttctaa caaatgttca ggagcatggt gtcttgtgat ggcgatttca 120
   tgtattttct gcatggggtt attaacagca tccattttct tgggcgtcaa gttgttgcag 180
   gtgtccacca ttgcaatgca gcagcaagaa aaactcatcc aacaagagag ggcactgcta 240
   aactttacag aatggaagag aagccatgtc cttcagatga aattttgtca aaccttcatg 300
   caaagctctt ttagttcagc ccataactgc agtccttgtc caaacaattg gattcagaac 360
   agagaaagct gttactatgt ctctgaacat tggaaaattt ggcacaccag tcaagagaat 420
   tgtttaaagg aaggttccac gctgctacaa atagagagcg aagaagaaat ggattttatc 480
   actggcagct tgaggaagat tagaggaagc tacgattact gggtggggtt gtctcaggat 540
   ggacacagcg gacgctggct ttggcaagat ggctcctctc cttctcctgg cctgttgcca 600
   gtagagatat cccagtcaac caaccaagtc tgtggataca tcaaaaacag ttcccttctt 660
   tcgtctaact gcagcacttg gaagtatttt atctgcgaga agtatgcatt aagatcctct 720
   gtctga 726
<210> 13
   <211> 726
   <212> DNA
   <213> Canis familiaris
<400> 13
   atgcaggagg aagaaacata cacctctctt cgttgggata gtccaacacc aagcttttac     60
   cagaaacacc tatcttccac caaatattca ggagcatggt gtctggtgac ggtgattaca    120
   tgtattctct gcgtgggctc aatagcaacc tctgttttct tgggcctcaa gttgttccag    180
   gtatctacca ttgcaatgaa acagcgagaa aagctcatcc ttcaggacag agcactgttg    240
   aatttcacac aatgggagag aaaccataac cttcagatga aatattgcca aaccttgatg    300
   caaaactctt tcagttcagc ccacaactgc agcccttgtc ctgacaactg gattcagaat    360
   ggagaaagtt gttaccatgt ctttgaaaac tggaaaattt ggcacaccag taaggaggac    420
   tgtttgaagg agggctctaa tcttctacaa atagacagca aagaagaaat ggactttatc    480
   actggcagcc tgaagaaggt caaaagtggc tttgattact gggtgggact gtctcaagac    540
   ggactcagca aaccttggct ttggcaagat ggttcctctc cctcccctga cctgtcgcca    600
   gtacagacat tgcaatcaac taaccagctc tgtggatatc taaaggacaa gttcctttct    660
   tctgctaact gcagcatttg gaaatatttt atctgtgaga agtatgcatt gagatcctct    720
   aactga                                                               726
<210> 14
   <211> 726
   <212> DNA
   <213> Bos taurus
<400> 14
   atgcaagagg atgaaatata cacctctctt cagtgggata ctccaacatc aaacccttat     60
   cagaaacatc tgtcttctac caaaaattca ggagtatggt gtcttgtgat ggtgatctta    120
   tgtattttct gcattggctc attagcaacc tctattttct tgggcatcaa attgtttcag    180
   atgtccacta ctataatgaa gcagcaagaa aaactcatcc aacaggagag agcactgctc    240
   aacttcacac agtggaagag aaaccccaac ctacagatga catattgcca aaccttaatg    300
   cagaagtctc tcagttcagc ctataactgc agcccttgtc cagacaactg gattcagaat    360
   ggagaaagtt gttatcatgt ctttgaaagc tggacattct ggcacactag tagaaaggat    420
   tgttggaaga agggctctga tcttctgcaa atagagagca aagaagaaat ggactttatc    480
   acgggcagcc tgaagaagat caagagaaac tatgattact gggtaggact gtcacagaat    540
   gggtccaacc aaccttggct ttggcaggat ggctcctctc cttctgctga cctgttgcca    600
   agacagggac cccagtcaac aaatcaggtc tgtggatacc tcagagacaa cgacctttct    660
   tctgctaact gcagcgtttg gaaatatttc atctgtgaga agtacgcact aagatcttct    720
   acctga                                                               726
<210> 15
   <211> 723
   <212> DNA
   <213> Equus caballus
<400> 15
   atgcaggagg aagaaatgta cacctctctc caatgggata acccaacatc aaacccttac      60
   cagaaaaatc tgccttccaa atgttcagga acacggtgtc ttgtgatagt gatttcatgt     120
   attttctgca tgggcttgtt aacaacgtcc attttcttgg gcatcaagtt gttccaggtg     180
   tctgctattg cagtgaagca gcaagaaaaa ctcatccaac aggagagaac actgttgaac     240
   ttcacacagt gtaatagaaa ccatgacttc cagatgaaat gctgtcaaat cctcatgaaa     300
   aactcattaa attcagccca tcactgcagc ccttgtccag acaactggat tcagaatgga     360
   gaaagttgtt actatgtctt tgaaaatcac aaaacttggc ataccagtaa acaggtttgt     420
   ttgaaggagg gctctaatct tctacaaata gacaacaaag aagaaatgga ctttatcaca     480
   ggcagcctga agaggatcaa aagcagctat gattactggg tgggactgtc tcaggacgga     540
   ctcagcggac cttggctttg gcaagatggt tcttctcttt ccccagacct gtggccagta     600
   cagagaccgc aatcacctaa cctggtctgt ggatacctca aaaacaagat ccttttttca    660
   gctaactgca gcagttggaa acattttatc tgtgagaagt atgcattaag atcttgcatc     720
   tga                                                                   723
<210> 16
   <211> 726
   <212> DNA
   <213> Rattus norvegicus
<400> 16
   atgcatgagg aagaaatata cacctctctt cagtgggaca ttccaacctc agaggcctct        60
   cagaagtgtc catcccttag caaatgtcca ggaacatggt gtattgtgac ggtgatttcc       120
   tgtgtggtct gtgtgggctt actagcagca tccattttct tgggcatcaa gttctcccag       180
   gtgtcctctc ttgtaatgga gcagcgggaa aggctcatcc gacaggacac agcattgctg       240
   aacctcacag agtggcagag gaaccataca ctgcagttaa aaagctgcca agcctcacta       300
   caaagatctc tccgttcagg cagtaactgc aacccttgtc caccgaactg gattcagaat       360
   ggaaaaagtt gttactatgc ctttgaccgc tgggaaacgt ggaacaacag taagaagagt       420
   tgtttaaaag agggcgatag tctccttcaa atagacagca aagaagaaat ggagtttatc       480
   aacctcagta tatggaagct caaaggagga tatgaatact gggtgggagt gtttcaagat       540
   ggacccagtg gatcttggtt ttgggaagat ggctcttctc ctctctctga cttgttgcca       600
   acagacagac agctatcagc cagccagatc tgtggatacc tcaaagacca tactctcatc       660
   tcggataact gcagtaactg gaaatatttt atctgtgaga agaaggcatt tggatcctgc       720
   atctga                                                                  726
<210> 17
   <211> 22
   <212> DNA
   <213> Artificial
<220>
   <223> Oligonucleotide primer
<400> 17
   tgtgactgct cccacaactg ga 22
<210> 18
   <211> 22
   <212> DNA
   <213> Artificial
<220>
   <223> Oligonucleotide primer
<400> 18
   tttgcaccaa tcacagcaca ga 22
<210> 19
   <211> 22
   <212> DNA
   <213> Artificial
<220>
   <223> Oligonucleotide primer
<400> 19
   catttgcagt ggcaaagtgg ag 22
<210> 20
   <211> 22
   <212> DNA
   <213> Artificial
<220>
   <223> Oligonucleotide primers
<400> 20
   gtctcgctcc tggaagatgg tg 22
<210> 21
   <211> 26
   <212> DNA
   <213> Artificial
<220>
   <223> Oligonucleotide primer
<400> 21
   gccatttctt gtaccaacct actcct 26
<210> 22
   <211> 23
   <212> DNA
   <213> Artificial
<220>
   <223> Oligonucleotide primer
<400> 22
   cggtgtggta tggatcgtca ctt 23
<210> 23
   <211> 22
   <212> DNA
   <213> Artificial
<220>
   <223> Oligonucleotide primer
<400> 23
   agcctcctgt gtggactgct tt 22
<210> 24
   <211> 22
   <212> DNA
   <213> Artificial
<220>
   <223> Oligonucleotide primer
<400> 24
   ttcatggccc acattttggt tt 22
<210> 25
   <211> 33
   <212> DNA
   <213> Artificial
<220>
   <223> Oligonucleotide primer
<400> 25
   tagtagacgc gtgagcagca ggaaagactc atc 33
<210> 26
   <211> 33
   <212> DNA
   <213> Artificial
<220>
   <223> Oligonucleotide primer
<400> 26
   tagtagacgc gttcagatgc aggatccaaa tgc 33
<210> 27
   <211> 33
   <212> DNA
   <213> Artificial
<220>
   <223> Oligonucleotide primer
<400> 27
   tagtagacgc gtcagcagca agaaaaactc atc 33
<210> 28
   <211> 33
   <212> DNA
   <213> Artificial
<220>
   <223> Oligonucleotide primer
<400> 28
   tagtagacgc gttcagacag aggatctcaa cgc 33
<210> 29
   <211> 22
   <212> PRT
   <213> Artificial
<220>
   <223> Antigenic fragment of murine 5B6
<400> 29
   Asp Gly Ser Ser Pro Leu Ser Asp Leu Leu Pro Ala Glu Arg Gln Arg
   1               5                   10                  15
   Ser Ala Gly Gln Ile Cys
               20
<210> 30
   <211> 27
   <212> PRT
   <213> Artificial
<220>
   <223> Antigenic fragment of human 5B6
<400> 30
   Arg Trp Leu Trp Gln Asp Gly Ser Ser Pro Ser Pro Gly Leu Leu Pro
   1               5                   10                  15
   Ala Glu Arg Ser Gln Ser Ala Asn Gln Val Cys
               20                  25
<210> 31
   <211> 18
   <212> PRT
   <213> Artificial
<220>
   <223> Biotinylation consensus sequence
<400> 31
   Asn Ser Gly Leu His His Ile Leu Asp Ala Gln Lys Met Val Trp Asn
   1               5                   10                  15
   His Arg
<210> 32
   <211> 136
   <212> PRT
   <213> Mus musculus
<400> 32
   Pro Cys Pro Lys Gly Ser Glu Trp Tyr Lys Asp Ser Cys Tyr Ser Gln
   1               5                   10                  15
   Leu Asn Gln Tyr Gly Thr Trp Gln Glu Ser Val Met Ala Cys Ser Ala
               20                  25                  30
   Arg Asn Ala Ser Leu Leu Lys Val Lys Asn Lys Asp Val Leu Glu Phe
           35                  40                  45
   Ile Lys Tyr Lys Lys Leu Arg Tyr Phe Trp Leu Ala Leu Leu Pro Arg
       50                  55                  60
   Lys Asp Arg Thr Gln Tyr Pro Leu Ser Glu Lys Met Phe Leu Ser Glu
   65                  70                  75                  80
   Glu Ser Glu Arg Ser Thr Asp Asp Ile Asp Lys Lys Tyr Cys Gly Tyr
                   85                  90                  95
   Ile Asp Arg Val Asn Val Tyr Tyr Thr Tyr Cys Thr Asp Glu Asn Asn
               100                 105                 110
   Ile Ile Cys Glu Glu Thr Ala Ser Lys Val Gln Leu Glu Ser Val Leu
           115                 120                 125
   Asn Gly Leu Pro Glu Asp Ser Arg
       130                 135
<210> 33
   <211> 127
   <212> PRT
   <213> Mus musculus
<400> 33
   Ser Cys Leu Pro Asn Trp Ile Met His Gly Lys Ser Cys Tyr Leu Phe
   1               5                   10                  15
   Ser Phe Ser Gly Asn Ser Trp Tyr Gly Ser Lys Arg His Cys Ser Gln
               20                  25                  30
   Leu Gly Ala His Leu Leu Lys Ile Asp Asn Ser Lys Glu Phe Glu Phe
           35                  40                  45
   Ile Glu Ser Gln Thr Ser Ser His Arg Ile Asn Ala Phe Trp Ile Gly
       50                  55                  60
   Leu Ser Arg Asn Gln Ser Glu Gly Pro Trp Phe Trp Glu Asp Gly Ser
   65                  70                  75                  80
   Ala Phe Phe Pro Asn Ser Phe Gln Val Arg Asn Thr Val Pro Gln Glu
                   85                  90                  95
   Ser Leu Leu His Asn Cys Val Trp Ile His Gly Ser Glu Val Tyr Asn
               100                 105                 110
   Gln Ile Cys Asn Thr Ser Ser Tyr Ser Ile Cys Glu Lys Glu Leu
           115                 120                 125
<210> 34
   <211> 130
   <212> PRT
   <213> Mus musculus
<400> 34
   Pro Cys Pro Gln Asp Trp Leu Trp His Lys Glu Asn Cys Tyr Leu Phe
   1               5                   10                  15
   His Gly Pro Phe Ser Trp Glu Lys Asn Arg Gln Thr Cys Gln Ser Leu
               20                  25                  30
   Gly Gly Gln Leu Leu Gln Ile Asn Gly Ala Asp Asp Leu Thr Phe Ile
           35                  40                  45
   Leu Gln Ala Ile Ser His Thr Thr Ser Pro Phe Trp Ile Gly Leu His
       50                  55                  60
   Arg Lys Lys Pro Gly Gln Pro Trp Leu Trp Glu Asn Gly Thr Arg Leu
   65                  70                  75                  80
   Asn Phe Gln Phe Phe Lys Thr Arg Gly Val Ser Leu Gln Leu Tyr Ser
                   85                  90                  95
   Ser Gly Asn Cys Ala Tyr Leu Gln Asp Gly Ala Val Phe Ala Glu Asn
               100                 105                 110
   Cys Ile Leu Ile Ala Phe Ser Ile Cys Gln Lys Lys Thr Asn His Leu
           115                 120                 125
   Gln Ile
       130
<210> 35
   <211> 119
   <212> PRT
   <213> Mus musculus
<400> 35
   Pro Cys Pro Asn Asn Trp Ile Cys His Arg Asn Asn Cys Tyr Gln Phe
   1               5                   10                  15
   Phe Asn Glu Glu Lys Thr Trp Asn Gln Ser Gln Ala Ser Cys Leu Ser
               20                  25                  30
   Gln Asn Ser Ser Leu Leu Lys Ile Tyr Ser Lys Glu Glu Gln Asp Phe
           35                  40                  45
   Leu Lys Leu Val Lys Ser Tyr His Trp Met Gly Leu Val Gln Ile Pro
       50                  55                  60
   Ala Asn Gly Ser Trp Gln Trp Glu Asp Gly Ser Ser Leu Ser Tyr Asn
   65                  70                  75                  80
   Gln Leu Thr Leu Val Glu Ile Pro Lys Gly Ser Cys Ala Val Tyr Gly
                   85                  90                  95
   Ser Ser Phe Lys Ala Tyr Thr Glu Asp Cys Ala Asn Leu Asn Thr Tyr
               100                 105                 110
   Ile Cys Met Lys Arg Ala Val
           115
<210> 36
   <211> 119
   <212> PRT
   <213> Homo sapiens
<400> 36
   Pro Cys Pro Lys Asn Trp Ile Cys Tyr Lys Asn Asn Cys Tyr Gln Phe
   1               5                   10                  15
   Phe Asp Glu Ser Lys Asn Trp Tyr Glu Ser Gln Ala Ser Cys Met Ser
               20                  25                  30
   Gln Asn Ala Ser Leu Leu Lys Val Tyr Ser Lys Glu Asp Gln Asp Leu
           35                  40                  45
   Leu Lys Leu Val Lys Ser Tyr His Trp Met Gly Leu Val His Ile Pro
       50                  55                  60
   Thr Asn Gly Ser Trp Gln Trp Glu Asp Gly Ser Ile Leu Ser Pro Asn
   65                  70                  75                  80
   Leu Leu Thr Ile Ile Glu Met Gln Lys Gly Asp Cys Ala Leu Tyr Ala
                   85                  90                  95
   Ser Ser Phe Lys Gly Tyr Ile Glu Asn Cys Ser Thr Pro Asn Thr Tyr
               100                 105                 110
   Ile Cys Met Gln Arg Thr Val
           115
<210> 37
   <211> 123
   <212> PRT
   <213> Rattus rattus
<400> 37
   His Ala Phe Ser Met Gly Lys Lys Ser Gly Lys Lys Phe Phe Val Thr
   1               5                   10                  15
   Asn His Glu Arg Met Pro Phe Ser Lys Val Lys Ala Leu Cys Ser Glu
               20                  25                  30
   Leu Arg Gly Thr Val Ala Ile Pro Arg Asn Ala Glu Glu Asn Lys Ala
           35                  40                  45
   Ile Gln Glu Val Ala Lys Thr Ser Ala Phe Leu Gly Ile Thr Asp Glu
       50                  55                  60
   Val Thr Glu Gly Gln Phe Met Tyr Val Thr Gly Gly Arg Leu Thr Tyr
   65                  70                  75                  80
   Ser Asn Trp Lys Lys Asp Glu Pro Asn Asp His Gly Ser Gly Glu Asp
                   85                  90                  95
   Cys Val Thr Ile Val Asp Asn Gly Leu Trp Asn Asp Ile Ser Cys Gln
               100                 105                 110
   Ala Ser His Thr Ala Val Cys Glu Phe Pro Ala
           115                 120
<210> 38
   <211> 33
   <212> DNA
   <213> Artificial Sequence
<220>
   <223> Oligonucleotide primer
<400> 38
   tagtagacgc gtggtagtga ctgcagccct tgt 33
<210> 39
   <211> 36
   <212> DNA
   <213> Artificial Sequence
<220>
   <223> Oligonucleotide primer
<400> 39
   tagtagacgc gtaacagcag tccttgtcca aacaat 36
<210> 40
   <211> 198
   <212> PRT
   <213> Artificial Sequence
<220>
   <223> Soluble mouse 5B6 including stalk
<400> 40
   Glu Gln Gln Glu Arg Leu Ile Gln Gln Asp Thr Ala Leu Val Asn Leu
   1               5                   10                  15
   Thr Gln Trp Gln Arg Lys Tyr Thr Leu Glu Tyr Cys Gln Ala Leu Leu
               20                  25                  30
   Gln Arg Ser Leu His Ser Gly Thr Asp Ala Ser Thr Gly Pro Val Leu
           35                  40                  45
   Leu Thr Ser Pro Gln Met Val Pro Gln Thr Leu Asp Ser Lys Glu Thr
       50                  55                  60
   Gly Ser Asp Cys Ser Pro Cys Pro His Asn Trp Ile Gln Asn Gly Lys
   65                  70                  75                  80
   Ser Cys Tyr Tyr Val Phe Glu Arg Trp Glu Met Trp Asn Ile Ser Lys
                   85                  90                  95
   Lys Ser Cys Leu Lys Glu Gly Ala Ser Leu Phe Gln Ile Asp Ser Lys
               100                 105                 110
   Glu Glu Met Glu Phe Ile Ser Ser Ile Gly Lys Leu Lys Gly Gly Asn
           115                 120                 125
   Lys Tyr Trp Val Gly Val Phe Gln Asp Gly Ile Ser Gly Ser Trp Phe
       130                 135                 140
   Trp Glu Asp Gly Ser Ser Pro Leu Ser Asp Leu Leu Pro Ala Glu Arg
   145                 150                 155                 160
   Gln Arg Ser Ala Gly Gln Ile Cys Gly Tyr Leu Lys Asp Ser Thr Leu
                   165                 170                 175
   Ile Ser Asp Lys Cys Asp Ser Trp Lys Tyr Phe Ile Cys Glu Lys Lys
               180                 185                 190
   Ala Phe Gly Ser Cys Ile
                             195
<210> 41
   <211> 175
   <212> PRT
   <213> Artificial Sequence
<220>
   <223> Soluble human 5B6 including stalk
<400> 41
   Gln Gln Gln Glu Lys Leu Ile Gln Gln Glu Arg Ala Leu Leu Asn Phe
   1 5 10 15
   Thr Glu Trp Lys Arg Ser Cys Ala Leu Gln Met Lys Tyr Cys Gln Ala
   20 25 30
   Phe Met Gln Asn Ser Leu Ser Ser Ala His Asn Ser Ser Pro Cys Pro
   35 40 45
   Asn Asn Trp Ile Gln Asn Arg Glu Ser Cys Tyr Tyr Val Ser Glu Ile
   50 55 60
   Trp Ser Ile Trp His Thr Ser Gln Glu Asn Cys Leu Lys Glu Gly Ser
   65 70 75 80
   Thr Leu Leu Gln Ile Glu Ser Lys Glu Glu Met Asp Phe Ile Thr Gly
   85 90 95
   Ser Leu Arg Lys Ile Lys Gly Ser Tyr Asp Tyr Trp Val Gly Leu Ser
   100 105 110
   Gln Asp Gly His Ser Gly Arg Trp Leu Trp Gln Asp Gly Ser Ser Pro
   115 120 125
   Ser Pro Gly Leu Leu Pro Ala Glu Arg Ser Gln Ser Ala Asn Gln Val
   130 135 140
   Cys Gly Tyr Val Lys Ser Asn Ser Leu Leu Ser Ser Asn Cys Ser Thr
   145 150 155 160
   Trp Lys Tyr Phe Ile Cys Glu Lys Tyr Ala Leu Arg Ser Ser Val
   165 170 175
<210> 42
   <211> 134
   <212> PRT
   <213> Artificial Sequence
<220>
   <223> Soluble mouse 5B6 without stalk
<400> 42
   Gly Ser Asp Cys Ser Pro Cys Pro His Asn Trp Ile Gln Asn Gly Lys
   1 5 10 15
   Ser Cys Tyr Tyr Val Phe Glu Arg Trp Glu Met Trp Asn Ile Ser Lys
   20 25 30
   Lys Ser Cys Leu Lys Glu Gly Ala Ser Leu Phe Gln Ile Asp Ser Lys
   35 40 45
   Glu Glu Met Glu Phe Ile Ser Ser Ile Gly Lys Leu Lys Gly Gly Asn
   50 55 60
   Lys Tyr Trp Val Gly Val Phe Gln Asp Gly Ile Ser Gly Ser Trp Phe
   65 70 75 80
   Trp Glu Asp Gly Ser Ser Pro Leu Ser Asp Leu Leu Pro Ala Glu Arg
   85 90 95
   Gln Arg Ser Ala Gly Gln Ile Cys Gly Tyr Leu Lys Asp Ser Thr Leu
   100 105 110
   Ile Ser Asp Lys Cys Asp Ser Trp Lys Tyr Phe Ile Cys Glu Lys Lys
   115 120 125
   Ala Phe Gly Ser Cys Ile
   130
<210> 43
   <211> 133
   <212> PRT
   <213> Artificial Sequence
<220>
   <223> Soluble human 5B6 without stalk
<400> 43
   Asn Ser Ser Pro Cys Pro Asn Asn Trp Ile Gln Asn Arg Glu Ser Cys
   1 5 10 15
   Tyr Tyr Val Ser Glu Ile Trp Ser Ile Trp His Thr Ser Gln Glu Asn
   20 25 30
   Cys Leu Lys Glu Gly Ser Thr Leu Leu Gln Ile Glu Ser Lys Glu Glu
   35 40 45
   Met Asp Phe Ile Thr Gly Ser Leu Arg Lys Ile Lys Gly Ser Tyr Asp
   50 55 60
   Tyr Trp Val Gly Leu Ser Gln Asp Gly His Ser Gly Arg Trp Leu Trp
   65 70 75 80
   Gln Asp Gly Ser Ser Pro Ser Pro Gly Leu Leu Pro Ala Glu Arg Ser
   85 90 95
   Gln Ser Ala Asn Gln Val Cys Gly Tyr Val Lys Ser Asn Ser Leu Leu
   100 105 110
   Ser Ser Asn Cys Ser Thr Trp Lys Tyr Phe Ile Cys Glu Lys Tyr Ala
   115 120 125
   Leu Arg Ser Ser Val
   130
<210> 44
   <211> 263
   <212> PRT
   <213> Artificial Sequence
<220>
   <223> Soluble flag tagged mouse 5B6 including stalk
<400> 44
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Asn Ser Gly Leu His His Ile Leu Asp Ala Gln Lys Met Val Trp
           35                  40                  45
   Asn His Arg Gly Ala Arg Gln Asp Tyr Lys Asp Asp Asp Asp Lys Thr
       50                  55                  60
   Arg Glu Gln Gln Glu Arg Leu Ile Gln Gln Asp Thr Ala Leu Val Asn
   65                  70                  75                  80
   Leu Thr Gln Trp Gln Arg Lys Tyr Thr Leu Glu Tyr Cys Gln Ala Leu
                   85                  90                  95
   Leu Gln Arg Ser Leu His Ser Gly Thr Asp Ala Ser Thr Gly Pro Val
               100                 105                 110
   Leu Leu Thr Ser Pro Gln Met Val Pro Gln Thr Leu Asp Ser Lys Glu
           115                 120                 125
   Thr Gly Ser Asp Cys Ser Pro Cys Pro His Asn Trp Ile Gln Asn Gly
       130                 135                 140
   Lys Ser Cys Tyr Tyr Val Phe Glu Arg Trp Glu Met Trp Asn Ile Ser
   145                 150                 155                 160
   Lys Lys Ser Cys Leu Lys Glu Gly Ala Ser Leu Phe Gln Ile Asp Ser
                   165                 170                 175
   Lys Glu Glu Met Glu Phe Ile Ser Ser Ile Gly Lys Leu Lys Gly Gly
               180                 185                 190
   Asn Lys Tyr Trp Val Gly Val Phe Gln Asp Gly Ile Ser Gly Ser Trp
           195                 200                 205
   Phe Trp Glu Asp Gly Ser Ser Pro Leu Ser Asp Leu Leu Pro Ala Glu
       210                 215                 220
   Arg Gln Arg Ser Ala Gly Gln Ile Cys Gly Tyr Leu Lys Asp Ser Thr
   225                 230                 235                 240
   Leu Ile Ser Asp Lys Cys Asp Ser Trp Lys Tyr Phe Ile Cys Glu Lys
                   245                 250                 255
   Lys Ala Phe Gly Ser Cys Ile
               260
<210> 45
   <211> 240
   <212> PRT
   <213> Artificial Sequence
<220>
   <223> Soluble flag tagged human 5B6 including stalk
<400> 45
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Asn Ser Gly Leu His His Ile Leu Asp Ala Gln Lys Met Val Trp
           35                  40                  45
   Asn His Arg Gly Ala Arg Gln Asp Tyr Lys Asp Asp Asp Asp Lys Thr
       50                  55                  60
   Arg Gln Gln Gln Glu Lys Leu Ile Gln Gln Glu Arg Ala Leu Leu Asn
   65                  70                  75                  80
   Phe Thr Glu Trp Lys Arg Ser Cys Ala Leu Gln Met Lys Tyr Cys Gln
                   85                  90                  95
   Ala Phe Met Gln Asn Ser Leu Ser Ser Ala His Asn Ser Ser Pro Cys
               100                 105                 110
   Pro Asn Asn Trp Ile Gln Asn Arg Glu Ser Cys Tyr Tyr Val Ser Glu
           115                 120                 125
   Ile Trp Ser Ile Trp His Thr Ser Gln Glu Asn Cys Leu Lys Glu Gly
       130                 135                 140
   Ser Thr Leu Leu Gln Ile Glu Ser Lys Glu Glu Met Asp Phe Ile Thr
   145                 150                 155                 160
   Gly Ser Leu Arg Lys Ile Lys Gly Ser Tyr Asp Tyr Trp Val Gly Leu
                   165                 170                 175
   Ser Gln Asp Gly His Ser Gly Arg Trp Leu Trp Gln Asp Gly Ser Ser
               180                 185                 190
   Pro Ser Pro Gly Leu Leu Pro Ala Glu Arg Ser Gln Ser Ala Asn Gln
           195                 200                 205
   Val Cys Gly Tyr Val Lys Ser Asn Ser Leu Leu Ser Ser Asn Cys Ser
       210                 215                 220
   Thr Trp Lys Tyr Phe Ile Cys Glu Lys Tyr Ala Leu Arg Ser Ser Val
   225                 230                 235                 240
<210> 46
   <211> 199
   <212> PRT
   <213> Artificial Sequence
<220>
   <223> Soluble flag tagged mouse 5B6 without stalk
<400> 46
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Asn Ser Gly Leu His His Ile Leu Asp Ala Gln Lys Met Val Trp
           35                  40                  45
   Asn His Arg Gly Ala Arg Gln Asp Tyr Lys Asp Asp Asp Asp Lys Thr
       50                  55                  60
   Arg Gly Ser Asp Cys Ser Pro Cys Pro His Asn Trp Ile Gln Asn Gly
   65                  70                  75                  80
   Lys Ser Cys Tyr Tyr Val Phe Glu Arg Trp Glu Met Trp Asn Ile Ser
                   85                  90                  95
   Lys Lys Ser Cys Leu Lys Glu Gly Ala Ser Leu Phe Gln Ile Asp Ser
               100                 105                 110
   Lys Glu Glu Met Glu Phe Ile Ser Ser Ile Gly Lys Leu Lys Gly Gly
           115                 120                 125
   Asn Lys Tyr Trp Val Gly Val Phe Gln Asp Gly Ile Ser Gly Ser Trp
       130                 135                 140
   Phe Trp Glu Asp Gly Ser Ser Pro Leu Ser Asp Leu Leu Pro Ala Glu
   145                 150                 155                 160
   Arg Gln Arg Ser Ala Gly Gln Ile Cys Gly Tyr Leu Lys Asp Ser Thr
                   165                 170                 175
   Leu Ile Ser Asp Lys Cys Asp Ser Trp Lys Tyr Phe Ile Cys Glu Lys
               180                 185                 190
   Lys Ala Phe Gly Ser Cys Ile
           195
<210> 47
   <211> 198
   <212> PRT
   <213> Artificial Sequence
<220>
   <223> Soluble flag tagged human 5B6 without stalk
<400> 47
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Asn Ser Gly Leu His His Ile Leu Asp Ala Gln Lys Met Val Trp
           35                  40                  45
   Asn His Arg Gly Ala Arg Gln Asp Tyr Lys Asp Asp Asp Asp Lys Thr
       50                  55                  60
   Arg Asn Ser Ser Pro Cys Pro Asn Asn Trp Ile Gln Asn Arg Glu Ser
   65                  70                  75                  80
   Cys Tyr Tyr Val Ser Glu Ile Trp Ser Ile Trp His Thr Ser Gln Glu
                   85                  90                  95
   Asn Cys Leu Lys Glu Gly Ser Thr Leu Leu Gln Ile Glu Ser Lys Glu
               100                 105                 110
   Glu Met Asp Phe Ile Thr Gly Ser Leu Arg Lys Ile Lys Gly Ser Tyr
           115                 120                 125
   Asp Tyr Trp Val Gly Leu Ser Gln Asp Gly His Ser Gly Arg Trp Leu
       130                 135                 140
   Trp Gln Asp Gly Ser Ser Pro Ser Pro Gly Leu Leu Pro Ala Glu Arg
   145                 150                 155                 160
   Ser Gln Ser Ala Asn Gln Val Cys Gly Tyr Val Lys Ser Asn Ser Leu
                   165                 170                 175
   Leu Ser Ser Asn Cys Ser Thr Trp Lys Tyr Phe Ile Cys Glu Lys Tyr
               180                 185                 190
   Ala Leu Arg Ser Ser Val
           195